# EUROPEAN PATENT SPECIFICATION

(11) **EP 3 754 024 B1**
(45) Date of publication and mention of the grant of the patent: **27.07.2022**
(21) Application number: 19757526.9
(22) Date of filing: 22.02.2019
(51) Int. Cl.: C12N 15/70, C12N 15/77, C12N 15/81, C12P 13/04, C12N 9/10, C12N 9/12

(54) **A MICROORGANISM PRODUCING A MYCOSPORINE-LIKE AMINO ACID AND A METHOD FOR PRODUCING A MYCOSPORINE-LIKE AMINO ACID USING THE SAME**
EIN MIKROORGANISMUS ZUR HERSTELLUNG EINER MYCOSPORIN-ÄHNLICHEN AMINOSÄURE UND EIN VERFAHREN ZUR HERSTELLUNG EINER MYCOSPORIN-ÄHNLICHEN AMINOSÄURE UNTER VERWENDUNG DESSELBEN
UN MICRO-ORGANISME PRODUCTEUR D'ACIDES AMINÉS DE TYPE MYCOSPORINE ET UN PROCÉDÉ DE PRODUCTION D'ACIDES AMINÉS DE TYPE MYCOSPORINE À L'AIDE DE CELUI-CI

(30) Priority: 23.02.2018 KR 20180022185
(43) Date of publication of application: 23.12.2020
(73) Proprietor: CJ Cheiljedang Corporation, Seoul 04560 (KR)
(72) Inventor: KIM, Sol, Seoul 04560 (KR); SEOK, Jong-cheol, Seoul 04560 (KR); LEE, Kyusung, Seoul 04560 (KR); JANG, Jae Woo, Seoul 04560 (KR)
(74) Representative: Maikowski & Ninnemann Patentanwälte Partnerschaft mbB
(86) International application number: PCT/KR2019/002242
(87) International publication number: WO 2019/164351

(56) References cited:
- EP-B1- 0 663 011
- WO-A1-2015/174427
- KR-B1- 101 911 186
- SEONG-HEE PARK ET AL: "Metabolic Engineering of Saccharomyces cerevisiae for Production of Shinorine, a Sunscreen Material, from Xylose", ACS SYNTHETIC BIOLOGY, vol. 8, no. 2, 26 December 2018 (2018-12-26), pages 346-357, XP055633219, Washington, DC,USA ISSN: 2161-5063, DOI: 10.1021/acssynbio.8b00388
- GUANG YANG ET AL: "Photosynthetic Production of Sunscreen Shinorine Using an Engineered Cyanobacterium", ACS SYNTHETIC BIOLOGY, vol. 7, no. 2, 19 January 2018 (2018-01-19), pages 664-671, XP055751055, Washington, DC,USA ISSN: 2161-5063, DOI: 10.1021/acssynbio.7b00397
- K. T. MIYAMOTO ET AL: "Discovery of Gene Cluster for Mycosporine-Like Amino Acid Biosynthesis from Actinomycetales Microorganisms and Production of a Novel Mycosporine-Like Amino Acid by Heterologous Expression", APPLIED AND ENVIRONMENTAL MICROBIOLOGY, vol. 80, no. 16, 6 June 2014 (2014-06-06), pages 5028-5036, XP055236703, US ISSN: 0099-2240, DOI: 10.1128/AEM.00727-14
- R. WADITEE-SIRISATTHA ET AL: "Identification and Upregulation of Biosynthetic Genes Required for Accumulation of Mycosporine-2-Glycine under Salt Stress Conditions in the Halotolerant Cyanobacterium Aphanothece halophytica", APPLIED AND ENVIRONMENTAL MICROBIOLOGY, vol. 80, no. 5, 27 December 2013 (2013-12-27), pages 1763-1769, XP055236695, US ISSN: 0099-2240, DOI: 10.1128/AEM.03729-13
- SINHA ET AL: "Database on mycosporines and mycosporine-like amino acids (MAAs) in fungi, cyanobacteria, macroalgae, phytoplankton and animals", JOURNAL OF PHOTOCHEMISTRY AND PHOTOBIOLOGY B: BIOLOGY, ELSEVIER SCIENCE S.A., BASEL, CH, vol. 89, no. 1, 30 October 2007 (2007-10-30), pages 29-35, XP022321796, ISSN: 1011-1344, DOI: 10.1016/J.JPHOTOBIOL.2007.07.006
- YOTA TSUGE ET AL: "Metabolic engineering of Corynebacterium glutamicum for production of sunscreen shinorine", BIOSCIENCE BIOTECHNOLOGY BIOCHEMISTRY, vol. 82, no. 7, 20 March 2018 (2018-03-20) , pages 1252-1259, XP055751051, JP ISSN: 0916-8451, DOI: 10.1080/09168451.2018.1452602
- GAO, Q.: "Microbial ultraviolet sunscreens", Nature Reviews Microbiology, November 2011 (2011-11), pages 791-802, XP055575448,
- GUERARD-HELAINE, C.: "Transketolase-Aldolase Symbiosis for the Stereoselective Preparation of Aldoses and Ketoses of Biological Interest", Advanced Synthesis & Catalysis, vol. 359, 7 June 2017 (2017-06-07), pages 2061-2065, XP055633215,
- ROSIC, N. N.: "Mycosporine-like amino acids from coral dinoflagellates", Applied Environmental Microbiology, vol. 7 7, no. 24, December 2011 (2011-12), pages 8478-8486, XP055633216,
- PATNAIK, R.: "Pathway engineering for production of aromatics in Escherichia coli: confirmation of stoichiometric analysis by independent modulation of AroG, TktA, and Pps activities", Biotechnology and Bioengineering, vol. 46, 1995, pages 361-370, XP002931419, DOI: doi:10.1002/bit.260460409
- PARK, S.-H.: "Metabolic engineering of Saccharomyces cerevisiae for production of shinorine, a sunscreen material, from xylose", ACS Synthetic Biology, 26 December 2018 (2018-12-26), pages 3 46-357, XP055633219,

<110> CJ CheilJedang Corporation
<120> A microorganism for producing a Mycosporine-like amino acid and a method for preparing a Mycosporine-like amino acid using the same
<130> OPA18547-PCT
<150> KR10-2018-0022185
   <151> 2018-02-23
<160> 124
<170> KoPatentIn 3.0
<210> 1
   <211> 1053
   <212> DNA
   <213> Escherichia coli W3110
<400> 1
   atgaattatc agaacgacga tttacgcatc aaagaaatca aagagttact tcctcctgtc        60
   gcattgctgg aaaaattccc cgctactgaa aatgccgcga atacggttgc ccatgcccga       120
   aaagcgatcc ataagatcct gaaaggtaat gatgatcgcc tgttggttgt gattggccca       180
   tgctcaattc atgatcctgt cgcggcaaaa gagtatgcca ctcgcttgct ggcgctgcgt       240
   gaagagctga aagatgagct ggaaatcgta atgcgcgtct attttgaaaa gccgcgtacc       300
   acggtgggct ggaaagggct gattaacgat ccgcatatgg ataatagctt ccagatcaac       360
   gacggtctgc gtatagcccg taaattgctg cttgatatta acgacagcgg tctgccagcg       420
   gcaggtgagt ttctcgatat gatcacccca caatatctcg ctgacctgat gagctggggc       480
   gcaattggcg cacgtaccac cgaatcgcag gtgcaccgcg aactggcatc agggctttct       540
   tgtccggtcg gcttcaaaaa tggcaccgac ggtacgatta aagtggctat cgatgccatt       600
   aatgccgccg gtgcgccgca ctgcttcctg tccgtaacga aatgggggca ttcggcgatt       660
   gtgaatacca gcggtaacgg cgattgccat atcattctgc gcggcggtaa agagcctaac       720
   tacagcgcga agcacgttgc tgaagtgaaa gaagggctga acaaagcagg cctgccagca       780
   caggtgatga tcgatttcag ccatgctaac tcgtccaaac aattcaaaaa gcagatggat       840
   gtttgtgctg acgtttgcca gcagattgcc ggtggcgaaa aggccattat tggcgtgatg       900
   gtggaaagcc atctggtgga aggcaatcag agcctcgaga gcggggagcc gctggcctac       960
   ggtaagagca tcaccgatgc ctgcatcggc tgggaagata ccgatgctct gttacgtcaa      1020
   ctggcgaatg cagtaaaagc gcgtcgcggg taa                                    1053
<210> 2
   <211> 350
   <212> PRT
   <213> Escherichia coli W3110
<400> 2
   Met Asn Tyr Gln Asn Asp Asp Leu Arg Ile Lys Glu Ile Lys Glu Leu
   1               5                   10                  15
   Leu Pro Pro Val Ala Leu Leu Glu Lys Phe Pro Ala Thr Glu Asn Ala
               20                  25                  30
   Ala Asn Thr Val Ala His Ala Arg Lys Ala Ile His Lys Ile Leu Lys
           35                  40                  45
   Gly Asn Asp Asp Arg Leu Leu Val Val Ile Gly Pro Cys Ser Ile His
       50                  55                  60
   Asp Pro Val Ala Ala Lys Glu Tyr Ala Thr Arg Leu Leu Ala Leu Arg
   65                  70                  75                  80
   Glu Glu Leu Lys Asp Glu Leu Glu Ile Val Met Arg Val Tyr Phe Glu
                   85                  90                  95
   Lys Pro Arg Thr Thr Val Gly Trp Lys Gly Leu Ile Asn Asp Pro His
               100                 105                 110
   Met Asp Asn Ser Phe Gln Ile Asn Asp Gly Leu Arg Ile Ala Arg Lys
           115                 120                 125
   Leu Leu Leu Asp Ile Asn Asp Ser Gly Leu Pro Ala Ala Gly Glu Phe
       130                 135                 140
   Leu Asp Met Ile Thr Pro Gln Tyr Leu Ala Asp Leu Met Ser Trp Gly
   145                 150                 155                 160
   Ala Ile Gly Ala Arg Thr Thr Glu Ser Gln Val His Arg Glu Leu Ala
                   165                 170                 175
   Ser Gly Leu Ser Cys Pro Val Gly Phe Lys Asn Gly Thr Asp Gly Thr
               180                 185                 190
   Ile Lys Val Ala Ile Asp Ala Ile Asn Ala Ala Gly Ala Pro His Cys
           195                 200                 205
   Phe Leu Ser Val Thr Lys Trp Gly His Ser Ala Ile Val Asn Thr Ser
       210                 215                 220
   Gly Asn Gly Asp Cys His Ile Ile Leu Arg Gly Gly Lys Glu Pro Asn
   225                 230                 235                 240
   Tyr Ser Ala Lys His Val Ala Glu Val Lys Glu Gly Leu Asn Lys Ala
                   245                 250                 255
   Gly Leu Pro Ala Gln Val Met Ile Asp Phe Ser His Ala Asn Ser Ser
               260                 265                 270
   Lys Gln Phe Lys Lys Gln Met Asp Val Cys Ala Asp Val Cys Gln Gln
           275                 280                 285
   Ile Ala Gly Gly Glu Lys Ala Ile Ile Gly Val Met Val Glu Ser His
       290                 295                 300
   Leu Val Glu Gly Asn Gln Ser Leu Glu Ser Gly Glu Pro Leu Ala Tyr
   305                 310                 315                 320
   Gly Lys Ser Ile Thr Asp Ala Cys Ile Gly Trp Glu Asp Thr Asp Ala
                   325                 330                 335
   Leu Leu Arg Gln Leu Ala Asn Ala Val Lys Ala Arg Arg Gly
   340 345 350
<210> 3
   <211> 49
   <212> DNA
   <213> Artificial Sequence
<220>
   <223> fhuA arm 1_F
<400> 3
   ccaagcgacg cccaacctgc catcatggcg cgttccaaaa ctgctcagc 49
<210> 4
   <211> 40
   <212> DNA
   <213> Artificial Sequence
<220>
   <223> fhuA arm 1_R
<400> 4
   tatcctcgag actagtgagc tcctttcagc ggttcggtgg 40
<210> 5
   <211> 40
   <212> DNA
   <213> Artificial Sequence
<220>
   <223> fhuA arm 2_F
<400> 5
   ctagtctcga ggatatcgac cacaccctgc tgaccggtgt 40
<210> 6
   <211> 48
   <212> DNA
   <213> Artificial Sequence
<220>
   <223> fhuA arm 2_R
<400> 6
   atagaatacc aattggcatg ctttttagaa acggaaggtt gcggttgc 48
<210> 7
   <211> 34
   <212> DNA
   <213> Artificial Sequence
<220>
   <223> Pn_aroG_F
<400> 7
   aaaggagctc actagtagga tgctcctgtt atgg 34
<210> 8
   <211> 36
   <212> DNA
   <213> Artificial Sequence
<220>
   <223> Pn_aroG_R
<400> 8
   cagggtgtgg tcgatatctt acccgcgacg cgcttt 36
<210> 9
   <211> 34
   <212> DNA
   <213> Artificial Sequence
<220>
   <223> Ptrc_F
<400> 9
   aaaggagctc actagtccgc ttgctgcaac tctc 34
<210> 10
   <211> 40
   <212> DNA
   <213> Artificial Sequence
<220>
   <223> Ptrc_R
<400> 10
   tggacgatac tcatatgttt cctgtgtgaa attgttatcc 40
<210> 11
   <211> 35
   <212> DNA
   <213> Artificial Sequence
<220>
   <223> Pcj1_F
<400> 11
   aaaggagctc actagtaccg cgggcttatt ccatt 35
<210> 12
   <211> 41
   <212> DNA
   <213> Artificial Sequence
<220>
   <223> Pcj1_R
<400> 12
   atctatagtc tgcatatgtt aatctcctag attgggtttc a 41
<210> 13
   <211> 32
   <212> DNA
   <213> Artificial Sequence
<220>
   <223> aroG_F
<400> 13
   taggagatta acatatgaat tatcagaacg ac 32
<210> 14
   <211> 18
   <212> DNA
   <213> Artificial Sequence
<220>
   <223> certification primer
<400> 14
   ggaacgctca gattgcgt 18
<210> 15
   <211> 45
   <212> DNA
   <213> Artificial Sequence
<220>
   <223> Ava_ABCD_F
<400> 15
   acaatttcac acaggaaaga tatcatgagt atcgtccaag caaag 45
<210> 16
   <211> 45
   <212> DNA
   <213> Artificial Sequence
<220>
   <223> Ava_ABCD_R
<400> 16
   ctcatccgcc aaaacagctc tagattatga attattttcc agaca 45
<210> 17
   <211> 6461
   <212> DNA
   <213> *Anabaena* variabilis ATCC29413
<400> 17
   atgagtatcg tccaagcaaa gtttgaagct aaggaaacat cttttcatgt agaaggttac 60
   gaaaagattg agtatgattt ggtgtatgta gatggtattt ttgaaatcca gaattctgca 120
   ctagcagatg tatatcaagg ttttggacga tgcttggcga ttgtagatgc taacgtcagt 180
   cggttgtatg gtaatcaaat tcaggcatat ttccagtatt atggtataga actgaggcta 240
   tttcctatta ccattactga accagataag actattcaaa ctttcgagag agttatagat 300
   gtctttgcag atttcaaatt agtccgcaaa gaaccagtat tagtcgtggg tggcggttta 360
   attacagatg ttgtcggctt tgcttgttct acatatcgtc gcagcagcaa ttacatccgc 420
   attcctacta cattgattgg attaattgat gccagtgtag caattaaggt agcagttaat 480
   catcgcaaac tgaaaaaccg tttgggtgct tatcatgctt ctcgcaaagt atttttagat 540
   ttctccttgt tgcgtactct ccctacagac caagtacgta acgggatggc ggaattggta 600
   aaaatcgctg tagtagcgca tcaagaagtt tttgaattgt tggagaagta cggcgaagaa 660
   ttactacgta ctcattttgg caatatagat gcaactccag agattaaaga aatagcccat 720
   cgtttgactt acaaagctat ccataagatg ttggaattgg aagttcccaa cctgcatgag 780
   ttagacctag atagggtgat tgcttacggt cacacttgga gtcccacctt ggaacttgcg 840
   cctcgtctac ccatgttcca cggacacgcc gttaatgtag atatggcttt ctcggcaacg 900
   atcgccgccc gtagaggata tattacaatt gcagaacgcg atcgtatttt aggattaatg 960
   agtcgcgttg gtctatccct cgaccatccc atgttggata tagatatttt gtggcgtggt 1020
   actgaatcta tcacattaac tcgtgatggt ttgttaagag ctgctatgcc aaaacccatt 1080
   ggtgattgtg tcttcgtcaa tgacctgaca agagaagaat tagcagccgc attagctgac 1140
   cacaaagaac tttgtaccag ttatccccgt ggtggtgaag gtgtggatgt gtatcccgtt 1200
   tatcaaaaag aattaatcgg gagtgttaaa taatgacttt tttgaattca aaatgcaaaa 1260
   tactccacgg atacactgcg cgagcgcggt agcatttctg ttcgcggagc gtcccgtagg 1320
   gaaagagaag gctacgcaaa taatcggaca ctaattgtct ttaattttga attttgaatt 1380
   ttgaattttg aattggagcg aagcgacttg acaaatgtga ttgtccaacc aacagctaga 1440
   cctgttacac cattgggaat tttaaccaag cagttagaag ccatagtcca agaggttaag 1500
   caacatccag atttacctgg ggaattgata gcaaacatcc atcaggcttg gcgtttagcc 1560
   gcaggtatag acccttattt ggaagaatgc accactccag aatctcctga actcgctgca 1620
   ttggcaaaaa ccacagccac cgaagcctgg ggagaacact tccacggagg tacaaccgtc 1680
   cgtcctctag aacaagagat gctttctggt catatcgaag gacaaacctt aaagatgttt 1740
   gttcacatga ccaaagctaa aaaagtctta gaaattggga tgtttaccgg ttattcggcg 1800
   ctggcgatgg cggaagcatt accagaggat ggactgcttg tggcttgtga agttgaccct 1860
   tacgcggcgg aaattggaca gaaagccttt caacaatctc cccacggtgg aaagattcgt 1920
   gtggaattgg atgcagcctt agcaactctt gataagttag cagaagctgg ggagtctttt 1980
   gacttggtat ttatcgacgc agataaaaaa gagtatgtag cctattttca caagttgcta 2040
   ggtagcagtt tgttagcacc agatggcttt atttgtgtag ataacacctt attacaaggg 2100
   gaagtttatc taccagcaga ggaacgtagc gtcaatggtg aagcgatcgc gcaatttaat 2160
   catacagtag ctatagaccc ccgtgtagaa caggttttgt tgccgttgcg agatggttta 2220
   acaattatcc gcagaataca accttaattg tccaatcgac tatggcacaa tcccttcccc 2280
   tttcttccgc acctgctaca ccgtctcttc cttcccagac gaaaatagcc gcaattatcc 2340
   aaaatatctg cactttggct ttgttattac tagcattgcc cattaatgcc accattgttt 2400
   ttatatcctt gttagtcttc cgaccgcaaa aggtcaaagc agcaaacccc caaaccattc 2460
   ttatcagtgg cggtaagatg accaaagctt tacaactagc aaggtcattc cacgcggctg 2520
   gacatagagt tgtcttggtg gaaacccata aatactggtt gactggtcat cgtttttccc 2580
   aagcagtgga taagttttac acagtccccg caccccagga caatccccaa gcttacattc 2640
   aggctttggt agatatcgtc aaacaagaaa acatcgatgt ttatattccc gtcaccagtc 2700
   cagtgggtag ctactacgac tcattagcca aaccagagtt atcccattat tgcgaagtgt 2760
   ttcactttga cgcagatatt acccaaatgt tggatgataa atttgcgttg acacaaaaag 2820
   cgcgatcgct tggtttatca gtacccaaat cctttaaaat tacctcacca gaacaagtca 2880
   tcaacttcga tttttctgga gagacacgta aatacatcct caaaagcatt ccctacgact 2940
   cagtgcggcg gttggactta accaaactcc cctgtgctac tccagaggaa acagcagcat 3000
   tcgtcagaag tttgccaatt actcccgaaa aaccgtggat tatgcaggaa tttatccccg 3060
   gtaaggaatt ctgcacccat agcaccgttc ggaatgggga actcagactg cattgctgtt 3120
   gcgaatcttc agccttccaa gttaattatg agaatgtaaa taacccgcaa attaccgaat 3180
   gggtacagca ttttgtcaag gaactgaaac tgacaggaca gatttccttt gactttatcc 3240
   aagccgaaga cggaacagtt tacgccatcg agtgtaaccc ccgcacacat tcagcaatta 3300
   ccacatttta cgaccacccc caggtagcag aagcgtactt gagtcaagca ccgacgactg 3360
   aaaccataca accactaacg acaagcaagc ctacctattg gacttatcac gaagtttggc 3420
   gtttaactgg tatccgttct ttcacccagt tgcaaagatg gctggggaat atttggcgcg 3480
   ggactgatgc gatttatcag ccagatgacc ccttaccgtt tttgatggta catcattggc 3540
   aaattcccct actgttattg aataatttgc gtcgtcttaa aggttggacg cggatagatt 3600
   tcaatattgg gaagttggtg gaattggggg gagattagtt tttaaacgca gagggacgct 3660
   gaggttagcg cagcgaaaag ttctggagga gggtttccct ccgtaggaaa cttttcaaga 3720
   gagagggacg cggagtgtgt tttctctgcg tctctgcgtg agaaattttt tattattgag 3780
   caaagttaga agatatgcag actatagatt ttaatattcg taagttactt gtagagtgga 3840
   acgcgaccca cagagattat gatctttccc agagtttaca tgaactaatt gtagctcaag 3900
   tagaacgaac acctgaggcg atcgctgtca cctttgacaa gcaacaacta acttatcaag 3960
   aactaaatca taaagcaaac cagctaggac attatttaca aacattagga gtccagccag 4020
   aaaccctggt aggcgtttgt ttagaacgtt ccttagaaat ggttatctgt cttttaggaa 4080
   tcctcaaagc tgggggtgct tatgttccta ttgaccctga atatcctcaa gaacgcatag 4140
   cttatatgct agaagattct caggtgaagg tactactaac tcaagaaaaa ttactcaatc 4200
   aaattcccca ccatcaagca caaactatct gtgtagatag ggaatgggag aaaatttcca 4260
   cacaagctaa taccaatccc aaaagtaata taaaaacgga taatcttgct tatgtaattt 4320
   acacctctgg ttccactggt aaaccaaaag gtgcaatgaa cacccacaaa ggtatctgta 4380
   atcgcttatt gtggatgcag gaagcttatc aaatcgattc cacagatagc attttacaaa 4440
   aaaccccctt tagttttgat gtttccgttt gggagttctt ttggacttta ttaactggcg 4500
   cacgtttggt aatagccaaa ccaggcggac ataaagatag tgcttacctc atcgatttaa 4560
   ttactcaaga acaaatcact acgttgcatt ttgtcccctc aatgctgcaa gtgtttttac 4620
   aaaatcgcca tgtaagcaaa tgcagctctc taaaaagagt tatttgtagc ggtgaagctt 4680
   tatctataga tttacaaaat agatttttcc agcatttgca atgtgaatta cataacctct 4740
   atggcccgac agaagcagca attgatgtca cattttggca atgtagaaaa gatagtaatt 4800
   taaagagtgt acctattggt cgtcccattg ctaatactca aatttatatt cttgatgccg 4860
   atttacaacc agtaaatatt ggtgtcactg gtgaaattta tattggtggt gtaggggttg 4920
   ctcgtggtta tttgaataaa gaagaattga ccaaagaaaa atttattatt aatccctttc 4980
   ccaattctga gtttaagcga ctttataaaa caggtgattt agctcgttat ttacccgatg 5040
   gaaatattga atatcttggt agaacagatt atcaagtaaa aattcggggt tatagaattg 5100
   aaattggcga gattgaaaat gttttatctt cacacccaca agtcagagaa gctgtagtca 5160
   tagcgcggga tgataacgct caagaaaaac aaatcatcgc ttatattacc tataactcca 5220
   tcaaacctca gcttgataat ctgcgtgatt tcctaaaagc aaggctacct gattttatga 5280
   ttccagccgc ttttgtgatg ctggagcatc ttcctttaac tcccagtggt aaagtagacc 5340
   gtaaggcatt acctaagcct gatttattta attatagtga acataattcc tatgtagcgc 5400
   ctcggaatga agttgaagaa aaattagtac aaatctggtc gaatattctg catttaccta 5460
   aagtaggtgt gacagaaaac tttttcgcta ttggtggtaa ttccctcaaa gctctacatt 5520
   taatttctca aattgaagag ttatttgcta aagagatatc cttagcaaca cttttaacaa 5580
   atccagtaat tgcagattta gccaaggtta ttcaagcaaa caaccaaatc cataattcac      5640
   ccctagttcc aattcaacca caaggtaagc agcagccttt cttttgtata catcctgctg      5700
   gtggtcatgt tttatgctat tttaaactcg cacaatatat aggaactgac caaccatttt      5760
   atggcttaca agctcaagga ttttatggag atgaagcacc cttgacgcga gttgaagata      5820
   tggctagtct ctacgtcaaa actattagag aatttcaacc ccaagggcct tatcgtgtcg      5880
   gggggtggtc atttggtgga gtcgtagctt atgaagtagc acagcagtta catagacaag      5940
   gacaagaagt atctttacta gcaatattag attcttacgt accgattctg ctggataaac      6000
   aaaaacccat tgatgacgtt tatttagttg gtgttctctc cagagttttt ggcggtatgt      6060
   ttggtcaaga taatctagtc acacctgaag aaatagaaaa tttaactgta gaagaaaaaa      6120
   ttaattacat cattgataaa gcacggagcg ctagaatatt cccgcctggt gtagaacgtc      6180
   aaaataatcg ccgtattctt gatgttttgg tgggaacttt aaaagcaact tattcctata      6240
   taagacaacc atatccagga aaagtcactg tatttcgagc cagggaaaaa catattatgg      6300
   ctcctgaccc gaccttagtt tgggtagaat tattttctgt aatggcggct caagaaatta      6360
   agattattga tgtccctgga aaccattatt cgtttgttct agaaccccat gtacaggttt      6420
   tagcacagcg tttacaagat tgtctggaaa ataattcata a                          6461
<210> 18
   <211> 2379
   <212> DNA
   <213> Escherichia coli W3110
<400> 18
   atgtccaaca atggctcgtc accgctggtg ctttggtata accaactcgg catgaatgat      60
   gtagacaggg ttgggggcaa aaatgcctcc ctgggtgaaa tgattactaa tctttccgga     120
   atgggtgttt ccgttccgaa tggtttcgcc acaaccgccg acgcgtttaa ccagtttctg     180
   gaccaaagcg gcgtaaacca gcgcatttat gaactgctgg ataaaacgga tattgacgat     240
   gttactcagc ttgcgaaagc gggcgcgcaa atccgccagt ggattatcga cactcccttc     300
   cagcctgagc tggaaaacgc catccgcgaa gcctatgcac agctttccgc cgatgacgaa     360
   aacgcctctt ttgcggtgcg ctcctccgcc accgcagaag atatgccgga cgcttctttt     420
   gccggtcagc aggaaacctt cctcaacgtt cagggttttg acgccgttct cgtggcagtg     480
   aaacatgtat ttgcttctct gtttaacgat cgcgccatct cttatcgtgt gcaccagggt     540
   tacgatcacc gtggtgtggc gctctccgcc ggtgttcaac ggatggtgcg ctctgacctc     600
   gcatcatctg gcgtgatgtt ctccattgat accgaatccg gctttgacca ggtggtgttt     660
   atcacttccg catggggcct tggtgagatg gtcgtgcagg gtgcggttaa cccggatgag     720
   ttttacgtgc ataaaccgac actggcggcg aatcgcccgg ctatcgtgcg ccgcaccatg     780
   gggtcgaaaa aaatccgcat ggtttacgcg ccgacccagg agcacggcaa gcaggttaaa        840
   atcgaagacg taccgcagga acagcgtgac atcttctcgc tgaccaacga agaagtgcag        900
   gaactggcaa aacaggccgt acaaattgag aaacactacg gtcgcccgat ggatattgag        960
   tgggcgaaag atggccacac cggtaaactg ttcattgtgc aggcgcgtcc ggaaaccgtg       1020
   cgctcacgcg gtcaggtcat ggagcgttat acgctgcatt cacagggtaa gattatcgcc       1080
   gaaggccgtg ctatcggtca tcgcatcggt gcgggtccgg tgaaagtcat ccatgacatc       1140
   agcgaaatga accgcatcga acctggcgac gtgctggtta ctgacatgac cgacccggac       1200
   tgggaaccga tcatgaagaa agcatctgcc atcgtcacca accgtggcgg tcgtacctgt       1260
   cacgcggcga tcatcgctcg tgaactgggc attccggcgg tagtgggctg tggagatgca       1320
   acagaacgga tgaaagacgg tgagaacgtc actgtttctt gtgccgaagg tgataccggt       1380
   tacgtctatg cggagttgct ggaatttagc gtgaaaagct ccagcgtaga aacgatgccg       1440
   gatctgccgt tgaaagtgat gatgaacgtc ggtaacccgg accgtgcttt cgacttcgcc       1500
   tgcctaccga acgaaggcgt gggccttgcg cgtctggaat ttatcatcaa ccgtatgatt       1560
   ggcgtccacc cacgcgcact gcttgagttt gacgatcagg aaccgcagtt gcaaaacgaa       1620
   atccgcgaga tgatgaaagg ttttgattct ccgcgtgaat tttacgttgg tcgtctgact       1680
   gaagggatcg cgacgctggg tgccgcgttt tatccgaagc gcgtcattgt ccgtctctct       1740
   gattttaaat cgaacgaata tgccaacctg gtcggtggtg agcgttacga gccagatgaa       1800
   gagaacccga tgctcggctt ccgtggcgcg ggccgctatg tttccgacag cttccgcgac       1860
   tgtttcgcgc tggagtgtga agcagtgaaa cgtgtgcgca acgacatggg actgaccaac       1920
   gttgagatca tgatcccgtt cgtgcgtacc gtagatcagg cgaaagcggt ggttgaagaa       1980
   ctggcgcgtc aggggctgaa acgtggcgag aacgggctga aaatcatcat gatgtgtgaa       2040
   atcccgtcca acgccttgct ggccgagcag ttcctcgaat atttcgacgg cttctcaatt       2100
   ggctcaaacg atatgacgca gctggcgctc ggtctggacc gtgactccgg cgtggtgtct       2160
   gaattgttcg atgagcgcaa cgatgcggtg aaagcactgc tgtcgatggc tatccgtgcc       2220
   gcgaagaaac agggcaaata tgtcgggatt tgcggtcagg gtccgtccga ccacgaagac       2280
   tttgccgcat ggttgatgga agaggggatc gatagcctgt ctctgaaccc ggacaccgtg       2340
   gtgcaaacct ggttaagcct ggctgaactg aagaaataa                              2379
<210> 19
   <211> 792
   <212> PRT
   <213> Escherichia coli W3110
<400> 19
   Met Ser Asn Asn Gly Ser Ser Pro Leu Val Leu Trp Tyr Asn Gln Leu
    1               5                  10                  15
   Gly Met Asn Asp Val Asp Arg Val Gly Gly Lys Asn Ala Ser Leu Gly
   20 25 30
   Glu Met Ile Thr Asn Leu Ser Gly Met Gly Val Ser Val Pro Asn Gly
   35 40 45
   Phe Ala Thr Thr Ala Asp Ala Phe Asn Gln Phe Leu Asp Gln Ser Gly
   50 55 60
   Val Asn Gln Arg Ile Tyr Glu Leu Leu Asp Lys Thr Asp Ile Asp Asp
   65 70 75 80
   Val Thr Gln Leu Ala Lys Ala Gly Ala Gln Ile Arg Gln Trp Ile Ile
   85 90 95
   Asp Thr Pro Phe Gln Pro Glu Leu Glu Asn Ala Ile Arg Glu Ala Tyr
   100 105 110
   Ala Gln Leu Ser Ala Asp Asp Glu Asn Ala Ser Phe Ala Val Arg Ser
   115 120 125
   Ser Ala Thr Ala Glu Asp Met Pro Asp Ala Ser Phe Ala Gly Gln Gln
   130 135 140
   Glu Thr Phe Leu Asn Val Gln Gly Phe Asp Ala Val Leu Val Ala Val
   145 150 155 160
   Lys His Val Phe Ala Ser Leu Phe Asn Asp Arg Ala Ile Ser Tyr Arg
   165 170 175
   Val His Gln Gly Tyr Asp His Arg Gly Val Ala Leu Ser Ala Gly Val
   180 185 190
   Gln Arg Met Val Arg Ser Asp Leu Ala Ser Ser Gly Val Met Phe Ser
   195 200 205
   Ile Asp Thr Glu Ser Gly Phe Asp Gln Val Val Phe Ile Thr Ser Ala
   210 215 220
   Trp Gly Leu Gly Glu Met Val Val Gln Gly Ala Val Asn Pro Asp Glu
   225 230 235 240
   Phe Tyr Val His Lys Pro Thr Leu Ala Ala Asn Arg Pro Ala Ile Val
   245 250 255
   Arg Arg Thr Met Gly Ser Lys Lys Ile Arg Met Val Tyr Ala Pro Thr
   260 265 270
   Gln Glu His Gly Lys Gln Val Lys Ile Glu Asp Val Pro Gln Glu Gln
   275 280 285
   Arg Asp Ile Phe Ser Leu Thr Asn Glu Glu Val Gln Glu Leu Ala Lys
   290 295 300
   Gln Ala Val Gln Ile Glu Lys His Tyr Gly Arg Pro Met Asp Ile Glu
   305 310 315 320
   Trp Ala Lys Asp Gly His Thr Gly Lys Leu Phe Ile Val Gln Ala Arg
   325 330 335
   Pro Glu Thr Val Arg Ser Arg Gly Gln Val Met Glu Arg Tyr Thr Leu
   340 345 350
   His Ser Gln Gly Lys Ile Ile Ala Glu Gly Arg Ala Ile Gly His Arg
           355                 360                 365
   Ile Gly Ala Gly Pro Val Lys Val Ile His Asp Ile Ser Glu Met Asn
       370                 375                 380
   Arg Ile Glu Pro Gly Asp Val Leu Val Thr Asp Met Thr Asp Pro Asp
   385                 390                 395                 400
   Trp Glu Pro Ile Met Lys Lys Ala Ser Ala Ile Val Thr Asn Arg Gly
                   405                 410                 415
   Gly Arg Thr Cys His Ala Ala Ile Ile Ala Arg Glu Leu Gly Ile Pro
               420                 425                 430
   Ala Val Val Gly Cys Gly Asp Ala Thr Glu Arg Met Lys Asp Gly Glu
           435                 440                 445
   Asn Val Thr Val Ser Cys Ala Glu Gly Asp Thr Gly Tyr Val Tyr Ala
       450                 455                 460
   Glu Leu Leu Glu Phe Ser Val Lys Ser Ser Ser Val Glu Thr Met Pro
   465                 470                 475                 480
   Asp Leu Pro Leu Lys Val Met Met Asn Val Gly Asn Pro Asp Arg Ala
                   485                 490                 495
   Phe Asp Phe Ala Cys Leu Pro Asn Glu Gly Val Gly Leu Ala Arg Leu
               500                 505                 510
   Glu Phe Ile Ile Asn Arg Met Ile Gly Val His Pro Arg Ala Leu Leu
           515                 520                 525
   Glu Phe Asp Asp Gln Glu Pro Gln Leu Gln Asn Glu Ile Arg Glu Met
       530                 535                 540
   Met Lys Gly Phe Asp Ser Pro Arg Glu Phe Tyr Val Gly Arg Leu Thr
   545                 550                 555                 560
   Glu Gly Ile Ala Thr Leu Gly Ala Ala Phe Tyr Pro Lys Arg Val Ile
                   565                 570                 575
   Val Arg Leu Ser Asp Phe Lys Ser Asn Glu Tyr Ala Asn Leu Val Gly
               580                 585                 590
   Gly Glu Arg Tyr Glu Pro Asp Glu Glu Asn Pro Met Leu Gly Phe Arg
           595                 600                 605
   Gly Ala Gly Arg Tyr Val Ser Asp Ser Phe Arg Asp Cys Phe Ala Leu
       610                 615                 620
   Glu Cys Glu Ala Val Lys Arg Val Arg Asn Asp Met Gly Leu Thr Asn
   625                 630                 635                 640
   Val Glu Ile Met Ile Pro Phe Val Arg Thr Val Asp Gln Ala Lys Ala
                   645                 650                 655
   Val Val Glu Glu Leu Ala Arg Gln Gly Leu Lys Arg Gly Glu Asn Gly
               660                 665                 670
   Leu Lys Ile Ile Met Met Cys Glu Ile Pro Ser Asn Ala Leu Leu Ala
           675                 680                 685
   Glu Gln Phe Leu Glu Tyr Phe Asp Gly Phe Ser Ile Gly Ser Asn Asp
       690                 695                 700
   Met Thr Gln Leu Ala Leu Gly Leu Asp Arg Asp Ser Gly Val Val Ser
   705                 710                 715                 720
   Glu Leu Phe Asp Glu Arg Asn Asp Ala Val Lys Ala Leu Leu Ser Met
                   725                 730                 735
   Ala Ile Arg Ala Ala Lys Lys Gln Gly Lys Tyr Val Gly Ile Cys Gly
               740                 745                 750
   Gln Gly Pro Ser Asp His Glu Asp Phe Ala Ala Trp Leu Met Glu Glu
           755                 760                 765
   Gly Ile Asp Ser Leu Ser Leu Asn Pro Asp Thr Val Val Gln Thr Trp
       770                 775                 780
   Leu Ser Leu Ala Glu Leu Lys Lys
   785                 790
<210> 20
   <211> 34
   <212> DNA
   <213> Artificial Sequence
<220>
   <223> Pn-pps_F
<400> 20
   aaaggagctc actagttttg ttcttcccgt gatg 34
<210> 21
   <211> 36
   <212> DNA
   <213> Artificial Sequence
<220>
   <223> Pn-pps_R
<400> 21
   cagggtgtgg tcgatatctt atttcttcag ttcagc 36
<210> 22
   <211> 32
   <212> DNA
   <213> Artificial Sequence
<220>
   <223> pps_F
<400> 22
   taggagatta acatatgtcc aacaatggct cg 32
<210> 23
   <211> 1992
   <212> DNA
   <213> Escherichia coli W3110
<400> 23
   atgtcctcac gtaaagagct tgccaatgct attcgtgcgc tgagcatgga cgcagtacag      60
   aaagccaaat ccggtcaccc gggtgcccct atgggtatgg ctgacattgc cgaagtcctg     120
   tggcgtgatt tcctgaaaca caacccgcag aatccgtcct gggctgaccg tgaccgcttc     180
   gtgctgtcca acggccacgg ctccatgctg atctacagcc tgctgcacct caccggttac     240
   gatctgccga tggaagaact gaaaaacttc cgtcagctgc actctaaaac tccgggtcac     300
   ccggaagtgg gttacaccgc tggtgtggaa accaccaccg gtccgctggg tcagggtatt     360
   gccaacgcag tcggtatggc gattgcagaa aaaacgctgg cggcgcagtt taaccgtccg     420
   ggccacgaca ttgtcgacca ctacacctac gccttcatgg gcgacggctg catgatggaa     480
   ggcatctccc acgaagtttg ctctctggcg ggtacgctga agctgggtaa actgattgca     540
   ttctacgatg acaacggtat ttctatcgat ggtcacgttg aaggctggtt caccgacgac     600
   accgcaatgc gtttcgaagc ttacggctgg cacgttattc gcgacatcga cggtcatgac     660
   gcggcatcta tcaaacgcgc agtagaagaa gcgcgcgcag tgactgacaa accttccctg     720
   ctgatgtgca aaaccatcat cggtttcggt tccccgaaca aagccggtac ccacgactcc     780
   cacggtgcgc cgctgggcga cgctgaaatt gccctgaccc gcgaacaact gggctggaaa     840
   tatgcgccgt tcgaaatccc gtctgaaatc tatgctcagt gggatgcgaa agaagcaggc     900
   caggcgaaag aatccgcatg gaacgagaaa ttcgctgctt acgcgaaagc ttatccgcag     960
   gaagccgctg aatttacccg ccgtatgaaa ggcgaaatgc cgtctgactt cgacgctaaa    1020
   gcgaaagagt tcatcgctaa actgcaggct aatccggcga aaatcgccag ccgtaaagcg    1080
   tctcagaatg ctatcgaagc gttcggtccg ctgttgccgg aattcctcgg cggttctgct    1140
   gacctggcgc cgtctaacct gaccctgtgg tctggttcta aagcaatcaa cgaagatgct    1200
   gcgggtaact acatccacta cggtgttcgc gagttcggta tgaccgcgat tgctaacggt    1260
   atctccctgc acggtggctt cctgccgtac acctccacct tcctgatgtt cgtggaatac    1320
   gcacgtaacg ccgtacgtat ggctgcgctg atgaaacagc gtcaggtgat ggtttacacc    1380
   cacgactcca tcggtctggg cgaagacggc ccgactcacc agccggttga gcaggtcgct    1440
   tctctgcgcg taaccccgaa catgtctaca tggcgtccgt gtgaccaggt tgaatccgcg    1500
   gtcgcgtgga aatacggtgt tgagcgtcag gacggcccga ccgcactgat cctctcccgt    1560
   cagaacctgg cgcagcagga acgaactgaa gagcaactgg caaacatcgc gcgcggtggt    1620
   tatgtgctga aagactgcgc cggtcagccg gaactgattt tcatcgctac cggttcagaa    1680
   gttgaactgg ctgttgctgc ctacgaaaaa ctgactgccg aaggcgtgaa agcgcgcgtg    1740
   gtgtccatgc cgtctaccga cgcatttgac aagcaggatg ctgcttaccg tgaatccgta 1800
   ctgccgaaag cggttactgc acgcgttgct gtagaagcgg gtattgctga ctactggtac 1860
   aagtatgttg gcctgaacgg tgctatcgtc ggtatgacca ccttcggtga atctgctccg 1920
   gcagagctgc tgtttgaaga gttcggcttc actgttgata acgttgttgc gaaagcaaaa 1980
   gaactgctgt aa 1992
<210> 24
   <211> 663
   <212> PRT
   <213> Escherichia coli W3110
<400> 24
   Met Ser Ser Arg Lys Glu Leu Ala Asn Ala Ile Arg Ala Leu Ser Met
   1               5                   10                  15
   Asp Ala Val Gln Lys Ala Lys Ser Gly His Pro Gly Ala Pro Met Gly
               20                  25                  30
   Met Ala Asp Ile Ala Glu Val Leu Trp Arg Asp Phe Leu Lys His Asn
           35                  40                  45
   Pro Gln Asn Pro Ser Trp Ala Asp Arg Asp Arg Phe Val Leu Ser Asn
       50                  55                  60
   Gly His Gly Ser Met Leu Ile Tyr Ser Leu Leu His Leu Thr Gly Tyr
   65                  70                  75                  80
   Asp Leu Pro Met Glu Glu Leu Lys Asn Phe Arg Gln Leu His Ser Lys
                   85                  90                  95
   Thr Pro Gly His Pro Glu Val Gly Tyr Thr Ala Gly Val Glu Thr Thr
               100                 105                 110
   Thr Gly Pro Leu Gly Gln Gly Ile Ala Asn Ala Val Gly Met Ala Ile
           115                 120                 125
   Ala Glu Lys Thr Leu Ala Ala Gln Phe Asn Arg Pro Gly His Asp Ile
       130                 135                 140
   Val Asp His Tyr Thr Tyr Ala Phe Met Gly Asp Gly Cys Met Met Glu
   145                 150                 155                 160
   Gly Ile Ser His Glu Val Cys Ser Leu Ala Gly Thr Leu Lys Leu Gly
                   165                 170                 175
   Lys Leu Ile Ala Phe Tyr Asp Asp Asn Gly Ile Ser Ile Asp Gly His
               180                 185                 190
   Val Glu Gly Trp Phe Thr Asp Asp Thr Ala Met Arg Phe Glu Ala Tyr
           195                 200                 205
   Gly Trp His Val Ile Arg Asp Ile Asp Gly His Asp Ala Ala Ser Ile
       210                 215                 220
   Lys Arg Ala Val Glu Glu Ala Arg Ala Val Thr Asp Lys Pro Ser Leu
   225                 230                 235                 240
   Leu Met Cys Lys Thr Ile Ile Gly Phe Gly Ser Pro Asn Lys Ala Gly
                   245                 250                 255
   Thr His Asp Ser His Gly Ala Pro Leu Gly Asp Ala Glu Ile Ala Leu
               260                 265                 270
   Thr Arg Glu Gln Leu Gly Trp Lys Tyr Ala Pro Phe Glu Ile Pro Ser
           275                 280                 285
   Glu Ile Tyr Ala Gln Trp Asp Ala Lys Glu Ala Gly Gln Ala Lys Glu
       290                 295                 300
   Ser Ala Trp Asn Glu Lys Phe Ala Ala Tyr Ala Lys Ala Tyr Pro Gln
   305                 310                 315                 320
   Glu Ala Ala Glu Phe Thr Arg Arg Met Lys Gly Glu Met Pro Ser Asp
                   325                 330                 335
   Phe Asp Ala Lys Ala Lys Glu Phe Ile Ala Lys Leu Gln Ala Asn Pro
               340                 345                 350
   Ala Lys Ile Ala Ser Arg Lys Ala Ser Gln Asn Ala Ile Glu Ala Phe
           355                 360                 365
   Gly Pro Leu Leu Pro Glu Phe Leu Gly Gly Ser Ala Asp Leu Ala Pro
       370                 375                 380
   Ser Asn Leu Thr Leu Trp Ser Gly Ser Lys Ala Ile Asn Glu Asp Ala
   385                 390                 395                 400
   Ala Gly Asn Tyr Ile His Tyr Gly Val Arg Glu Phe Gly Met Thr Ala
                   405                 410                 415
   Ile Ala Asn Gly Ile Ser Leu His Gly Gly Phe Leu Pro Tyr Thr Ser
               420                 425                 430
   Thr Phe Leu Met Phe Val Glu Tyr Ala Arg Asn Ala Val Arg Met Ala
           435                 440                 445
   Ala Leu Met Lys Gln Arg Gln Val Met Val Tyr Thr His Asp Ser Ile
       450                 455                 460
   Gly Leu Gly Glu Asp Gly Pro Thr His Gln Pro Val Glu Gln Val Ala
   465                 470                 475                 480
   Ser Leu Arg Val Thr Pro Asn Met Ser Thr Trp Arg Pro Cys Asp Gln
                   485                 490                 495
   Val Glu Ser Ala Val Ala Trp Lys Tyr Gly Val Glu Arg Gln Asp Gly
               500                 505                 510
   Pro Thr Ala Leu Ile Leu Ser Arg Gln Asn Leu Ala Gln Gln Glu Arg
           515                 520                 525
   Thr Glu Glu Gln Leu Ala Asn Ile Ala Arg Gly Gly Tyr Val Leu Lys
       530                 535                 540
   Asp Cys Ala Gly Gln Pro Glu Leu Ile Phe Ile Ala Thr Gly Ser Glu
   545                 550                 555                 560
   Val Glu Leu Ala Val Ala Ala Tyr Glu Lys Leu Thr Ala Glu Gly Val
                   565                 570                 575
   Lys Ala Arg Val Val Ser Met Pro Ser Thr Asp Ala Phe Asp Lys Gln
                   580                 585                 590
   Asp Ala Ala Tyr Arg Glu Ser Val Leu Pro Lys Ala Val Thr Ala Arg
               595                 600                 605
   Val Ala Val Glu Ala Gly Ile Ala Asp Tyr Trp Tyr Lys Tyr Val Gly
       610                 615                 620
   Leu Asn Gly Ala Ile Val Gly Met Thr Thr Phe Gly Glu Ser Ala Pro
   625                 630                 635                 640
   Ala Glu Leu Leu Phe Glu Glu Phe Gly Phe Thr Val Asp Asn Val Val
                   645                 650                 655
   Ala Lys Ala Lys Glu Leu Leu
                   660
<210> 25
   <211> 34
   <212> DNA
   <213> Artificial Sequence
<220>
   <223> Pn-tktA_F
<400> 25
   aaaggagctc actagtgttt tctcttccgc acaa 34
<210> 26
   <211> 36
   <212> DNA
   <213> Artificial Sequence
<220>
   <223> Pn-tktA_R
<400> 26
   cagggtgtgg tcgatatctt acagcagttc ttttgc 36
<210> 27
   <211> 32
   <212> DNA
   <213> Artificial Sequence
<220>
   <223> tktA_F
<400> 27
   taggagatta acatatgtcc tcacgtaaag ag 32
<210> 28
   <211> 26
   <212> DNA
   <213> Artificial Sequence
<220>
   <223> Pcj1-aroG_R
<400> 28
   cccgcggttt acccgcgacg cgcttt 26
<210> 29
   <211> 26
   <212> DNA
   <213> Artificial Sequence
<220>
   <223> Pcj1-pps_F
<400> 29
   gcgggtaaac cgcgggctta ttccat 26
<210> 30
   <211> 26
   <212> DNA
   <213> Artificial Sequence
<220>
   <223> Pcj1-pps_R
<400> 30
   cccgcggttt atttcttcag ttcagc 26
<210> 31
   <211> 26
   <212> DNA
   <213> Artificial Sequence
<220>
   <223> Pcj1-tktA_F
<400> 31
   agaaataaac cgcgggctta ttccat 26
<210> 32
   <211> 71
   <212> DNA
   <213> Artificial Sequence
<220>
   <223> aroD deletion cassette_F
<400> 32
   tcatggggtt cggtgcctga caggctgacc gcgtgcagaa agggtaaaaa gctggagctg 60
   cttcgaagtt c 71
<210> 33
   <211> 79
   <212> DNA
   <213> Artificial Sequence
<220>
   <223> aroD deletion cassette_R
<400> 33
   atatattttt tagttcggcg gggagggtgt tcccgccgaa atattattgc gccatggtcc 60
   atatgaatat cctccttag 79
<210> 34
   <211> 23
   <212> DNA
   <213> Artificial Sequence
<220>
   <223> aroD deletion_F
<400> 34
   caaagatttc cctctggaat atg 23
<210> 35
   <211> 21
   <212> DNA
   <213> Artificial Sequence
<220>
   <223> aroD deletion_R
<400> 35
   cagatgtgat tttccctacg c 21
<210> 36
   <211> 1389
   <212> DNA
   <213> Corynebacterium glutamicum ATCC13032
<400> 36
   gtgagttgga cagttgatat ccctaaagaa gttctccctg atttgccacc attgccagaa 60
   ggcatgcagc agcagttcga ggacaccatt tcccgtgacg ctaagcagca acctacgtgg 120
   gatcgtgcac aggcagaaaa cgtgcgcaag atccttgagt cggttcctcc aatcgttgtt 180
   gcccctgagg tacttgagct gaagcagaag cttgctgatg ttgccaacgg taaggccttc 240
   ctcttgcagg gtggtgactg tgcggaaact ttcgagtcaa acactgagcc gcacattcgc 300
   gccaacgtaa agactctgct gcagatggca gttgttttga cctacggtgc atccactcct 360
   gtgatcaaga tggctcgtat tgctggtcag tacgcaaagc ctcgctcttc tgatctggat 420
   ggaaatggtc tgccaaacta ccgtggcgat atcgtcaacg gtgtggaggc aaccccagag 480
   gctcgtcgcc acgatcctgc ccgcatgatc cgtgcttacg ctaacgcttc tgctgcgatg 540
   aacttggtgc gcgcgctcac cagctctggc accgctgatc tttaccgtct cagcgagtgg         600
   aaccgcgagt tcgttgcgaa ctccccagct ggtgcacgct acgaggctct tgctcgtgag         660
   atcgactccg gtctgcgctt catggaagca tgtggcgtgt ccgatgagtc cctgcgtgct         720
   gcagatatct actgctccca cgaggctttg ctggtggatt acgagcgttc catgctgcgt         780
   cttgcaaccg atgaggaagg caacgaggaa ctttacgatc tttcagctca ccagctgtgg         840
   atcggcgagc gcacccgtgg catggatgat ttccatgtga acttcgcatc catgatctct         900
   aacccaatcg gcatcaagat tggtcctggt atcacccctg aagaggctgt tgcatacgct         960
   gacaagctcg atccgaactt cgagcctggc cgtttgacca tcgttgctcg catgggccac        1020
   gacaaggttc gctccgtact tcctggtgtt atccaggctg ttgaggcatc cggacacaag        1080
   gttatttggc agtccgatcc gatgcacggc aacactttca ccgcatccaa tggctacaag        1140
   acccgtcact tcgacaaggt tatcgatgag gtccagggct tcttcgaggt ccaccgcgca        1200
   ttgggcaccc acccaggcgg aatccacatt gagttcactg gtgaagatgt caccgagtgc        1260
   ctcggtggcg ctgaagacat caccgatgtt gatctgccag gccgctacga gtccgcatgc        1320
   gatcctcgcc tgaacactca gcagtctttg gagttggctt tcctcgttgc agaaatgctg        1380
   cgtaactaa                                                                1389
<210> 37
   <211> 462
   <212> PRT
   <213> Corynebacterium glutamicum ATCC13032
<400> 37
   Met Ser Trp Thr Val Asp Ile Pro Lys Glu Val Leu Pro Asp Leu Pro
    1               5                  10                  15
   Pro Leu Pro Glu Gly Met Gln Gln Gln Phe Glu Asp Thr Ile Ser Arg
               20                  25                  30
   Asp Ala Lys Gln Gln Pro Thr Trp Asp Arg Ala Gln Ala Glu Asn Val
           35                  40                  45
   Arg Lys Ile Leu Glu Ser Val Pro Pro Ile Val Val Ala Pro Glu Val
       50                  55                  60
   Leu Glu Leu Lys Gln Lys Leu Ala Asp Val Ala Asn Gly Lys Ala Phe
   65                  70                  75                  80
   Leu Leu Gln Gly Gly Asp Cys Ala Glu Thr Phe Glu Ser Asn Thr Glu
                   85                  90                  95
   Pro His Ile Arg Ala Asn Val Lys Thr Leu Leu Gln Met Ala Val Val
               100                 105                 110
   Leu Thr Tyr Gly Ala Ser Thr Pro Val Ile Lys Met Ala Arg Ile Ala
           115                 120                 125
   Gly Gln Tyr Ala Lys Pro Arg Ser Ser Asp Leu Asp Gly Asn Gly Leu
       130                 135                 140
   Pro Asn Tyr Arg Gly Asp Ile Val Asn Gly Val Glu Ala Thr Pro Glu
   145                 150                 155                 160
   Ala Arg Arg His Asp Pro Ala Arg Met Ile Arg Ala Tyr Ala Asn Ala
                   165                 170                 175
   Ser Ala Ala Met Asn Leu Val Arg Ala Leu Thr Ser Ser Gly Thr Ala
               180                 185                 190
   Asp Leu Tyr Arg Leu Ser Glu Trp Asn Arg Glu Phe Val Ala Asn Ser
           195                 200                 205
   Pro Ala Gly Ala Arg Tyr Glu Ala Leu Ala Arg Glu Ile Asp Ser Gly
       210                 215                 220
   Leu Arg Phe Met Glu Ala Cys Gly Val Ser Asp Glu Ser Leu Arg Ala
   225                 230                 235                 240
   Ala Asp Ile Tyr Cys Ser His Glu Ala Leu Leu Val Asp Tyr Glu Arg
                   245                 250                 255
   Ser Met Leu Arg Leu Ala Thr Asp Glu Glu Gly Asn Glu Glu Leu Tyr
               260                 265                 270
   Asp Leu Ser Ala His Gln Leu Trp Ile Gly Glu Arg Thr Arg Gly Met
           275                 280                 285
   Asp Asp Phe His Val Asn Phe Ala Ser Met Ile Ser Asn Pro Ile Gly
       290                 295                 300
   Ile Lys Ile Gly Pro Gly Ile Thr Pro Glu Glu Ala Val Ala Tyr Ala
   305                 310                 315                 320
   Asp Lys Leu Asp Pro Asn Phe Glu Pro Gly Arg Leu Thr Ile Val Ala
                   325                 330                 335
   Arg Met Gly His Asp Lys Val Arg Ser Val Leu Pro Gly Val Ile Gln
               340                 345                 350
   Ala Val Glu Ala Ser Gly His Lys Val Ile Trp Gln Ser Asp Pro Met
           355                 360                 365
   His Gly Asn Thr Phe Thr Ala Ser Asn Gly Tyr Lys Thr Arg His Phe
       370                 375                 380
   Asp Lys Val Ile Asp Glu Val Gln Gly Phe Phe Glu Val His Arg Ala
   385                 390                 395                 400
   Leu Gly Thr His Pro Gly Gly Ile His Ile Glu Phe Thr Gly Glu Asp
                   405                 410                 415
   Val Thr Glu Cys Leu Gly Gly Ala Glu Asp Ile Thr Asp Val Asp Leu
               420                 425                 430
   Pro Gly Arg Tyr Glu Ser Ala Cys Asp Pro Arg Leu Asn Thr Gln Gln
           435                 440                 445
   Ser Leu Glu Leu Ala Phe Leu Val Ala Glu Met Leu Arg Asn
       450                 455                 460
<210> 38
   <211> 36
   <212> DNA
   <213> Artificial Sequence
<220>
   <223> Pn-cgl aroG_F
<400> 38
   ggtatcgata agcttgattc gatctgctcc cattcc 36
<210> 39
   <211> 36
   <212> DNA
   <213> Artificial Sequence
<220>
   <223> Pn-cgl aroG_R
<400> 39
   cggtggcggc cgctctagtt agttacgcag catttc 36
<210> 40
   <211> 39
   <212> DNA
   <213> Artificial Sequence
<220>
   <223> Pcj7-cgl aroG_F
<400> 40
   cgaaaggaaa cactcgatgt gagttggaca gttgatatc 39
<210> 41
   <211> 37
   <212> DNA
   <213> Artificial Sequence
<220>
   <223> Pcj7-cgl aroG_R
<400> 41
   ccgccaaaac agctctagtt tagttacgca gcatttc 37
<210> 42
   <211> 60
   <212> DNA
   <213> Artificial Sequence
<220>
   <223> Ava_ABCD_F
<400> 42
   ccaaacacca acaaaaggct ctacccatat gatgagtatc gtccaagcaa agtttgaagc 60 60
<210> 43
   <211> 60
   <212> DNA
   <213> Artificial Sequence
<220>
   <223> Ava_ABCD_R
<400> 43
   agagggtgac gctagtcaga actagtttat gaattatttt ccagacaatc ttgtaaacgc 60 60
<210> 44
   <211> 54
   <212> DNA
   <213> Artificial Sequence
<220>
   <223> O2 promoter_F
<400> 44
   ccaaacacca acaaaaggct ctacccatat gcaataatcg tgaattttgg cagc 54
<210> 45
   <211> 53
   <212> DNA
   <213> Artificial Sequence
<220>
   <223> O2 promoter_R
<400> 45
   caaactttgc ttggacgata ctcattgttt tgatctcctc caataatcta tgc 53
<210> 46
   <211> 37
   <212> DNA
   <213> Artificial Sequence
<220>
   <223> Pn-cgl tkt_F
<400> 46
   ggtatcgata agcttgataa gattgatcac acctttg 37
<210> 47
   <211> 36
   <212> DNA
   <213> Artificial Sequence
<220>
   <223> Pn-cgl tkt_R
<400> 47
   cggtggcggc cgctctagtt aaccgttaat ggagtc 36
<210> 48
   <211> 36
   <212> DNA
   <213> Artificial Sequence
<220>
   <223> Pcj7-cgl tkt_F
<400> 48
   cgaaaggaaa cactcgattt gaccaccttg acgctg 36
<210> 49
   <211> 37
   <212> DNA
   <213> Artificial Sequence
<220>
   <223> Pcj7-cgl tkt_R
<400> 49
   ccgccaaaac agctctagtt taaccgttaa tggagtc 37
<210> 50
   <211> 36
   <212> DNA
   <213> Artificial Sequence
<220>
   <223> Ptrc-cgl pps_F
<400> 50
   tcacacagga aagatatcat gaccaacagt ttgaac 36
<210> 51
   <211> 39
   <212> DNA
   <213> Artificial Sequence
<220>
   <223> Ptrc-cgl pps_R
<400> 51
   ccgccaaaac agctctagtt tacttcgtgc cggtcattg 39
<210> 52
   <211> 36
   <212> DNA
   <213> Artificial Sequence
<220>
   <223> Pcj7-cgl pps_F
<400> 52
   cgaaaggaaa cactcgatat gaccaacagt ttgaac 36
<210> 53
   <211> 37
   <212> DNA
   <213> Artificial Sequence
<220>
   <223> Pcj7-aroG_F
<400> 53
   ttagaaatgg aacctaaaag aaacatccca gcgctac 37
<210> 54
   <211> 36
   <212> DNA
   <213> Artificial Sequence
<220>
   <223> Pcj7-aroG_R
<400> 54
   tagcgctggg atgtttcttt agttacgcag catttc 36
<210> 55
   <211> 36
   <212> DNA
   <213> Artificial Sequence
<220>
   <223> Pcj7-tkt_F
<400> 55
   gaaatgctgc gtaactaaag aaacatccca gcgcta 36
<210> 56
   <211> 37
   <212> DNA
   <213> Artificial Sequence
<220>
   <223> Pcj7-tkt_R
<400> 56
   gtagcgctgg gatgtttctt taaccgttaa tggagtc 37
<210> 57
   <211> 37
   <212> DNA
   <213> Artificial Sequence
<220>
   <223> Pcj7-pps_F
<400> 57
   gactccatta acggttaaag aaacatccca gcgctac 37
<210> 58
   <211> 39
   <212> DNA
   <213> Artificial Sequence
<220>
   <223> Pcj7-pps_R
<400> 58
   ggagaatact gtcgttcatt tacttcgtgc cggtcattg 39
<210> 59
   <211> 26
   <212> DNA
   <213> Artificial Sequence
<220>
   <223> pGPD_F
<400> 59
   ggatccacag tttattcctg gcatcc 26
<210> 60
   <211> 40
   <212> DNA
   <213> Artificial Sequence
<220>
   <223> pGPD_R
<400> 60
   tttgcttgga cgatactcat ttgtttgttt atgtgtgttt 40
<210> 61
   <211> 40
   <212> DNA
   <213> Artificial Sequence
<220>
   <223> Ava_A_F
<400> 61
   aaacacacat aaacaaacaa atgagtatcg tccaagcaaa 40
<210> 62
   <211> 40
   <212> DNA
   <213> Artificial Sequence
<220>
   <223> Ava_A_R
<400> 62
   ggatgccagg aataaactgt ttatttaaca ctcccgatta 40
<210> 63
   <211> 40
   <212> DNA
   <213> Artificial Sequence
<220>
   <223> pGPD_F
<400> 63
   taatcgggag tgttaaataa acagtttatt cctggcatcc 40
<210> 64
   <211> 40
   <212> DNA
   <213> Artificial Sequence
<220>
   <223> pGPD_R
<400> 64
   tggacaatca catttgtcaa ttgtttgttt atgtgtgttt 40
<210> 65
   <211> 40
   <212> DNA
   <213> Artificial Sequence
<220>
   <223> Ava_B_F
<400> 65
   aaacacacat aaacaaacaa ttgacaaatg tgattgtcca 40
<210> 66
   <211> 26
   <212> DNA
   <213> Artificial Sequence
<220>
   <223> Ava_B_R
<400> 66
   gtcgacttaa ggttgtattc tgcgga 26
<210> 67
   <211> 26
   <212> DNA
   <213> Artificial Sequence
<220>
   <223> pGPD_F
<400> 67
   ggatccacag tttattcctg gcatcc 26
<210> 68
   <211> 40
   <212> DNA
   <213> Artificial Sequence
<220>
   <223> pGPD_R
<400> 68
   aaagagattg attgtgccat ttgtttgttt atgtgtgttt 40
<210> 69
   <211> 40
   <212> DNA
   <213> Artificial Sequence
<220>
   <223> Ava_C_F
<400> 69
   aaacacacat aaacaaacaa atggcacaat caatctcttt 40
<210> 70
   <211> 40
   <212> DNA
   <213> Artificial Sequence
<220>
   <223> Ava_C_R
<400> 70
   ggatgccagg aataaactgt ttagtcgccc cctaattcca 40
<210> 71
   <211> 40
   <212> DNA
   <213> Artificial Sequence
<220>
   <223> pGPD_F
<400> 71
   tggaattagg gggcgactaa acagtttatt cctggcatcc 40
<210> 72
   <211> 40
   <212> DNA
   <213> Artificial Sequence
<220>
   <223> pGPD_R
<400> 72
   aggatattaa gtactggcat ttgtttgttt atgtgtgttt 40
<210> 73
   <211> 40
   <212> DNA
   <213> Artificial Sequence
<220>
   <223> Ava_D_F
<400> 73
   aaacacacat aaacaaacaa atgccagtac ttaatatcct 40
<210> 74
   <211> 26
   <212> DNA
   <213> Artificial Sequence
<220>
   <223> Ava_D_R
<400> 74
   gtcgactcaa ttttgtaaca cctttt 26
<210> 75
   <211> 26
   <212> DNA
   <213> Artificial Sequence
<220>
   <223> TKL1_F
<400> 75
   ggatccatga ctcaattcac tgacat 26
<210> 76
   <211> 26
   <212> DNA
   <213> Artificial Sequence
<220>
   <223> TKL1_R
<400> 76
   gtcgacttag aaagcttttt tcaaag 26
<210> 77
   <211> 26
   <212> DNA
   <213> Artificial Sequence
<220>
   <223> ARO4_F
<400> 77
   ggatccatga gtgaatctcc aatgtt 26
<210> 78
   <211> 26
   <212> DNA
   <213> Artificial Sequence
<220>
   <223> ARO4_R
<400> 78
   gtcgacctat ttcttgttaa cttctc 26
<210> 79
   <211> 26
   <212> DNA
   <213> Artificial Sequence
<220>
   <223> pps_F
<400> 79
   ggatccatgt ccaacaatgg ctcgtc 26
<210> 80
   <211> 26
   <212> DNA
   <213> Artificial Sequence
<220>
   <223> pps_R
<400> 80
   gtcgacttat ttcttcagtt cagcca 26
<210> 81
   <211> 26
   <212> DNA
   <213> Artificial Sequence
<220>
   <223> pGPD_F
<400> 81
   ggatccacag tttattcctg gcatcc 26
<210> 82
   <211> 40
   <212> DNA
   <213> Artificial Sequence
<220>
   <223> pGPD_R
<400> 82
   atgtcagtga attgagtcat ttgtttgttt atgtgtgttt 40
<210> 83
   <211> 40
   <212> DNA
   <213> Artificial Sequence
<220>
   <223> TKL1_F
<400> 83
   aaacacacat aaacaaacaa atgactcaat tcactgacat 40
<210> 84
   <211> 40
   <212> DNA
   <213> Artificial Sequence
<220>
   <223> TKL1_R
<400> 84
   ggatgccagg aataaactgt ttagaaagct tttttcaaag 40
<210> 85
   <211> 40
   <212> DNA
   <213> Artificial Sequence
<220>
   <223> pGPD_F
<400> 85
   ctttgaaaaa agctttctaa acagtttatt cctggcatcc 40
<210> 86
   <211> 40
   <212> DNA
   <213> Artificial Sequence
<220>
   <223> pGPD_R
<400> 86
   aacattggag attcactcat ttgtttgttt atgtgtgttt 40
<210> 87
   <211> 40
   <212> DNA
   <213> Artificial Sequence
<220>
   <223> ARO4_F
<400> 87
   aaacacacat aaacaaacaa atgagtgaat ctccaatgtt 40
<210> 88
   <211> 26
   <212> DNA
   <213> Artificial Sequence
<220>
   <223> ARO4_R
<400> 88
   gtcgacctat ttcttgttaa cttctc 26
<210> 89
   <211> 438
   <212> DNA
   <213> Corynebacterium glutamicum ATCC13032
<400> 89
   atgcctggaa aaattctcct cctcaacggc ccaaacctga acatgctggg caaacgcgag 60
   cctgacattt acggacacga caccttggaa gacgtcgtcg cgctggcaac cgctgaggct 120
   gcgaagcacg gccttgaggt tgaggcgctg cagagcaatc acgaaggtga gctaatcgat 180
   gcgctgcaca acgctcgcgg cacccacatc ggttgcgtga ttaacccggg cggcctgact 240
   cacacttcgg tggcgctttt ggatgctgtg aaggcgtctg agcttcctac cgttgaggtg 300
   cacatttcca atccgcatgc ccgtgaagag ttccgccacc attcttacat ttccctcgcc 360
   gcggtctccg ttatcgctgg cgctggcatc cagggttacc gtttcgcggt cgatatcctg 420
   gcaaatctca aaaagtag 438
<210> 90
   <211> 145
   <212> PRT
   <213> Corynebacterium glutamicum ATCC13032
<400> 90
   Met Pro Gly Lys Ile Leu Leu Leu Asn Gly Pro Asn Leu Asn Met Leu
   1               5                   10                  15
   Gly Lys Arg Glu Pro Asp Ile Tyr Gly His Asp Thr Leu Glu Asp Val
               20                  25                  30
   Val Ala Leu Ala Thr Ala Glu Ala Ala Lys His Gly Leu Glu Val Glu
           35                  40                  45
   Ala Leu Gln Ser Asn His Glu Gly Glu Leu Ile Asp Ala Leu His Asn
       50                  55                  60
   Ala Arg Gly Thr His Ile Gly Cys Val Ile Asn Pro Gly Gly Leu Thr
   65                  70                  75                  80
   His Thr Ser Val Ala Leu Leu Asp Ala Val Lys Ala Ser Glu Leu Pro
                   85                  90                  95
   Thr Val Glu Val His Ile Ser Asn Pro His Ala Arg Glu Glu Phe Arg
               100                 105                 110
   His His Ser Tyr Ile Ser Leu Ala Ala Val Ser Val Ile Ala Gly Ala
           115                 120                 125
   Gly Ile Gln Gly Tyr Arg Phe Ala Val Asp Ile Leu Ala Asn Leu Lys
       130                 135                 140
   Lys
   145
<210> 91
   <211> 32
   <212> DNA
   <213> Artificial Sequence
<220>
   <223> aroD_F
<400> 91
   gatcggatcc cactggacgt ttgggtgaga cc 32
<210> 92
   <211> 37
   <212> DNA
   <213> Artificial Sequence
<220>
   <223> aroD_R
<400> 92
   gatcggatcc actagtttta ggttccattt ctaattg 37
<210> 93
   <211> 29
   <212> DNA
   <213> Artificial Sequence
<220>
   <223> aroD_F
<400> 93
   gatcactagt atgaacgaca gtattctcc 29
<210> 94
   <211> 30
   <212> DNA
   <213> Artificial Sequence
<220>
   <223> aroD_D
<400> 94
   gatcaagctt gttacatcct gacgttgtgg 30
<210> 95
   <211> 2103
   <212> DNA
   <213> Corynebacterium glutamicum ATCC13032
<400> 95
   ttgaccacct tgacgctgtc acctgaactt caggcgctca ctgtacgcaa ttacccctct       60
   gattggtccg atgtggacac caaggctgta gacactgttc gtgtcctcgc tgcagacgct      120
   gtagaaaact gtggctccgg ccacccaggc accgcaatga gcctggctcc ccttgcatac      180
   accttgtacc agcgggttat gaacgtagat ccacaggaca ccaactgggc aggccgtgac      240
   cgcttcgttc tttcttgtgg ccactcctct ttgacccagt acatccagct ttacttgggt      300
   ggattcggcc ttgagatgga tgacctgaag gctctgcgca cctgggattc cttgacccca      360
   ggacaccctg agtaccgcca caccaagggc gttgagatca ccactggccc tcttggccag      420
   ggtcttgcat ctgcagttgg tatggccatg gctgctcgtc gtgagcgtgg cctattcgac      480
   ccaaccgctg ctgagggcga atccccattc gaccaccaca tctacgtcat tgcttctgat      540
   ggtgacctgc aggaaggtgt cacctctgag gcatcctcca tcgctggcac ccagcagctg      600
   ggcaacctca tcgtgttctg ggatgacaac cgcatctcca tcgaagacaa cactgagatc      660
   gctttcaacg aggacgttgt tgctcgttac aaggcttacg gctggcagac cattgaggtt      720
   gaggctggcg aggacgttgc agcaatcgaa gctgcagtgg ctgaggctaa gaaggacacc      780
   aagcgaccta ccttcatccg cgttcgcacc atcatcggct tcccagctcc aactatgatg      840
   aacaccggtg ctgtgcacgg tgctgctctt ggcgcagctg aggttgcagc aaccaagact      900
   gagcttggat tcgatcctga ggctcacttc gcgatcgacg atgaggttat cgctcacacc      960
   cgctccctcg cagagcgcgc tgcacagaag aaggctgcat ggcaggtcaa gttcgatgag     1020
   tgggcagctg ccaaccctga gaacaaggct ctgttcgatc gcctgaactc ccgtgagctt      1080
   ccagcgggct acgctgacga gctcccaaca tgggatgcag atgagaaggg cgtcgcaact      1140
   cgtaaggctt ccgaggctgc acttcaggca ctgggcaaga cccttcctga gctgtggggc      1200
   ggttccgctg acctcgcagg ttccaacaac accgtgatca agggctcccc ttccttcggc      1260
   cctgagtcca tctccaccga gacctggtct gctgagcctt acggccgtaa cctgcacttc      1320
   ggtatccgtg agcacgctat gggatccatc ctcaacggca tttccctcca cggtggcacc      1380
   cgcccatacg gcggaacctt cctcatcttc tccgactaca tgcgtcctgc agttcgtctt      1440
   gcagctctca tggagaccga cgcttactac gtctggaccc acgactccat cggtctgggc      1500
   gaagatggcc caacccacca gcctgttgaa accttggctg cactgcgcgc catcccaggt      1560
   ctgtccgtcc tgcgtcctgc agatgcgaac gagaccgccc aggcttgggc tgcagcactt      1620
   gagtacaagg aaggccctaa gggtcttgca ctgacccgcc agaacgttcc tgttctggaa      1680
   ggcaccaagg agaaggctgc tgaaggcgtt cgccgcggtg gctacgtcct ggttgagggt      1740
   tccaaggaaa ccccagatgt gatcctcatg ggctccggct ccgaggttca gcttgcagtt      1800
   aacgctgcga aggctctgga agctgagggc gttgcagctc gcgttgtttc cgttccttgc      1860
   atggattggt tccaggagca ggacgcagag tacatcgagt ccgttctgcc tgcagctgtg      1920
   accgctcgtg tgtctgttga agctggcatc gcaatgcctt ggtaccgctt cttgggcacc      1980
   cagggccgtg ctgtctccct tgagcacttc ggtgcttctg cggattacca gaccctgttt      2040
   gagaagttcg gcatcaccac cgatgcagtc gtggcagcgg ccaaggactc cattaacggt      2100
   taa                                                                    2103
<210> 96
   <211> 700
   <212> PRT
   <213> Corynebacterium glutamicum ATCC13032
<400> 96
   Met Thr Thr Leu Thr Leu Ser Pro Glu Leu Gln Ala Leu Thr Val Arg
   1               5                   10                  15
   Asn Tyr Pro Ser Asp Trp Ser Asp Val Asp Thr Lys Ala Val Asp Thr
               20                  25                  30
   Val Arg Val Leu Ala Ala Asp Ala Val Glu Asn Cys Gly Ser Gly His
           35                  40                  45
   Pro Gly Thr Ala Met Ser Leu Ala Pro Leu Ala Tyr Thr Leu Tyr Gln
       50                  55                  60
   Arg Val Met Asn Val Asp Pro Gln Asp Thr Asn Trp Ala Gly Arg Asp
   65                  70                  75                  80
   Arg Phe Val Leu Ser Cys Gly His Ser Ser Leu Thr Gln Tyr Ile Gln
                   85                  90                  95
   Leu Tyr Leu Gly Gly Phe Gly Leu Glu Met Asp Asp Leu Lys Ala Leu
               100                 105                 110
   Arg Thr Trp Asp Ser Leu Thr Pro Gly His Pro Glu Tyr Arg His Thr
           115                 120                 125
   Lys Gly Val Glu Ile Thr Thr Gly Pro Leu Gly Gln Gly Leu Ala Ser
       130                 135                 140
   Ala Val Gly Met Ala Met Ala Ala Arg Arg Glu Arg Gly Leu Phe Asp
   145                 150                 155                 160
   Pro Thr Ala Ala Glu Gly Glu Ser Pro Phe Asp His His Ile Tyr Val
                   165                 170                 175
   Ile Ala Ser Asp Gly Asp Leu Gln Glu Gly Val Thr Ser Glu Ala Ser
               180                 185                 190
   Ser Ile Ala Gly Thr Gln Gln Leu Gly Asn Leu Ile Val Phe Trp Asp
           195                 200                 205
   Asp Asn Arg Ile Ser Ile Glu Asp Asn Thr Glu Ile Ala Phe Asn Glu
       210                 215                 220
   Asp Val Val Ala Arg Tyr Lys Ala Tyr Gly Trp Gln Thr Ile Glu Val
   225                 230                 235                 240
   Glu Ala Gly Glu Asp Val Ala Ala Ile Glu Ala Ala Val Ala Glu Ala
                   245                 250                 255
   Lys Lys Asp Thr Lys Arg Pro Thr Phe Ile Arg Val Arg Thr Ile Ile
               260                 265                 270
   Gly Phe Pro Ala Pro Thr Met Met Asn Thr Gly Ala Val His Gly Ala
           275                 280                 285
   Ala Leu Gly Ala Ala Glu Val Ala Ala Thr Lys Thr Glu Leu Gly Phe
       290                 295                 300
   Asp Pro Glu Ala His Phe Ala Ile Asp Asp Glu Val Ile Ala His Thr
   305                 310                 315                 320
   Arg Ser Leu Ala Glu Arg Ala Ala Gln Lys Lys Ala Ala Trp Gln Val
                   325                 330                 335
   Lys Phe Asp Glu Trp Ala Ala Ala Asn Pro Glu Asn Lys Ala Leu Phe
               340                 345                 350
   Asp Arg Leu Asn Ser Arg Glu Leu Pro Ala Gly Tyr Ala Asp Glu Leu
           355                 360                 365
   Pro Thr Trp Asp Ala Asp Glu Lys Gly Val Ala Thr Arg Lys Ala Ser
       370                 375                 380
   Glu Ala Ala Leu Gln Ala Leu Gly Lys Thr Leu Pro Glu Leu Trp Gly
   385                 390                 395                 400
   Gly Ser Ala Asp Leu Ala Gly Ser Asn Asn Thr Val Ile Lys Gly Ser
                   405                 410                 415
   Pro Ser Phe Gly Pro Glu Ser Ile Ser Thr Glu Thr Trp Ser Ala Glu
               420                 425                 430
   Pro Tyr Gly Arg Asn Leu His Phe Gly Ile Arg Glu His Ala Met Gly
           435                 440                 445
   Ser Ile Leu Asn Gly Ile Ser Leu His Gly Gly Thr Arg Pro Tyr Gly
       450                 455                 460
   Gly Thr Phe Leu Ile Phe Ser Asp Tyr Met Arg Pro Ala Val Arg Leu
   465                 470                 475                 480
   Ala Ala Leu Met Glu Thr Asp Ala Tyr Tyr Val Trp Thr His Asp Ser
                   485                 490                 495
   Ile Gly Leu Gly Glu Asp Gly Pro Thr His Gln Pro Val Glu Thr Leu
               500                 505                 510
   Ala Ala Leu Arg Ala Ile Pro Gly Leu Ser Val Leu Arg Pro Ala Asp
           515                 520                 525
   Ala Asn Glu Thr Ala Gln Ala Trp Ala Ala Ala Leu Glu Tyr Lys Glu
       530                 535                 540
   Gly Pro Lys Gly Leu Ala Leu Thr Arg Gln Asn Val Pro Val Leu Glu
   545                 550                 555                 560
   Gly Thr Lys Glu Lys Ala Ala Glu Gly Val Arg Arg Gly Gly Tyr Val
                   565                 570                 575
   Leu Val Glu Gly Ser Lys Glu Thr Pro Asp Val Ile Leu Met Gly Ser
               580                 585                 590
   Gly Ser Glu Val Gln Leu Ala Val Asn Ala Ala Lys Ala Leu Glu Ala
           595                 600                 605
   Glu Gly Val Ala Ala Arg Val Val Ser Val Pro Cys Met Asp Trp Phe
       610                 615                 620
   Gln Glu Gln Asp Ala Glu Tyr Ile Glu Ser Val Leu Pro Ala Ala Val
   625                 630                 635                 640
   Thr Ala Arg Val Ser Val Glu Ala Gly Ile Ala Met Pro Trp Tyr Arg
                   645                 650                 655
   Phe Leu Gly Thr Gln Gly Arg Ala Val Ser Leu Glu His Phe Gly Ala
               660                 665                 670
   Ser Ala Asp Tyr Gln Thr Leu Phe Glu Lys Phe Gly Ile Thr Thr Asp
           675                 680                 685
   Ala Val Val Ala Ala Ala Lys Asp Ser Ile Asn Gly
       690                 695                 700
<210> 97
   <211> 1095
   <212> DNA
   <213> Corynebacterium glutamicum ATCC13032
<400> 97
   atgaccaaca gtttgaacat cccgtttgtc cagcgcttcg atgaaggcct ggatcctgtt        60
   ctagaagtac tcggtggcaa gggcgcttca ctagtcacca tgacagatgc tggaatgccc       120
   gttccacctg gatttgtggt cactactgcc agctttgatg aattcatccg tgaagcaggg       180
   gttgctgaac acatcgataa attcctaaac gatctcgatg cagaagatgt taaggaagtg       240
   gatcgagttt ctgcgatcat ccgcgatgag ctgtgcagtc ttgacgttcc agagaatgct       300
   cgtttcgcag tgcaccaggc ttatcgcgat ctcatggaac gatgcggtgg cgacgtcccg       360
   gttgctgtcc ggtcatcggc cactgccgaa gatctgcccg atgcttcctt cgcagggcaa       420
   caggacacct atctgtggca agtcggtttg agcgctgtca ctgaacacat ccgtaaatgc       480
   tgggcttcgc tgttcacttc ccgtgccatt atctaccgtc tgaaaaacaa catccccaat       540
   gagggcctct ccatggcggt agttgttcaa aaaatggtca actctcgtgt cgcaggcgtg       600
   gcaatcacta tgaatccttc caacggcgac cgctcgaaga tcaccatcga ttcctcatgg       660
   ggtgttggtg aaatggtggt ctcaggtgaa gtgacaccag acaatatctt gctggacaag       720
   atcacgctgc aggttgtctc cgaacacatt ggaagcaaac acgctgaact catccccgat       780
   gccaccagtg gaagcctcgt ggaaaagccc gttgatgaag aacgcgcaaa ccgccgcagt       840
   ctgactgatg aggaaatgct cgctgtggca caaatggcta agcgtgcaga aaaacactac       900
   aagtgcccac aagatatcga atgggcgctg gacgctgatc tgccagatgg agaaaacctt       960
   ctgttattgc aatcccgccc ggaaactatc cactccaacg gtgtgaagaa ggaaacccca      1020
   actccgcagg ctgccaaaac cataggcacc ttcgatttca gctcaatcac cgtcgcaatg      1080
   accggcacga agtaa                                                       1095
<210> 98
   <211> 364
   <212> PRT
   <213> Corynebacterium glutamicum ATCC13032
<400> 98
   Met Thr Asn Ser Leu Asn Ile Pro Phe Val Gln Arg Phe Asp Glu Gly
   1 5 10 15
   Leu Asp Pro Val Leu Glu Val Leu Gly Gly Lys Gly Ala Ser Leu Val
   20 25 30
   Thr Met Thr Asp Ala Gly Met Pro Val Pro Pro Gly Phe Val Val Thr
   35 40 45
   Thr Ala Ser Phe Asp Glu Phe Ile Arg Glu Ala Gly Val Ala Glu His
   50 55 60
   Ile Asp Lys Phe Leu Asn Asp Leu Asp Ala Glu Asp Val Lys Glu Val
   65 70 75 80
   Asp Arg Val Ser Ala Ile Ile Arg Asp Glu Leu Cys Ser Leu Asp Val
   85 90 95
   Pro Glu Asn Ala Arg Phe Ala Val His Gln Ala Tyr Arg Asp Leu Met
   100 105 110
   Glu Arg Cys Gly Gly Asp Val Pro Val Ala Val Arg Ser Ser Ala Thr
               115                 120                 125
   Ala Glu Asp Leu Pro Asp Ala Ser Phe Ala Gly Gln Gln Asp Thr Tyr
       130                 135                 140
   Leu Trp Gln Val Gly Leu Ser Ala Val Thr Glu His Ile Arg Lys Cys
   145                 150                 155                 160
   Trp Ala Ser Leu Phe Thr Ser Arg Ala Ile Ile Tyr Arg Leu Lys Asn
                   165                 170                 175
   Asn Ile Pro Asn Glu Gly Leu Ser Met Ala Val Val Val Gln Lys Met
               180                 185                 190
   Val Asn Ser Arg Val Ala Gly Val Ala Ile Thr Met Asn Pro Ser Asn
           195                 200                 205
   Gly Asp Arg Ser Lys Ile Thr Ile Asp Ser Ser Trp Gly Val Gly Glu
       210                 215                 220
   Met Val Val Ser Gly Glu Val Thr Pro Asp Asn Ile Leu Leu Asp Lys
   225                 230                 235                 240
   Ile Thr Leu Gln Val Val Ser Glu His Ile Gly Ser Lys His Ala Glu
                   245                 250                 255
   Leu Ile Pro Asp Ala Thr Ser Gly Ser Leu Val Glu Lys Pro Val Asp
               260                 265                 270
   Glu Glu Arg Ala Asn Arg Arg Ser Leu Thr Asp Glu Glu Met Leu Ala
           275                 280                 285
   Val Ala Gln Met Ala Lys Arg Ala Glu Lys His Tyr Lys Cys Pro Gln
       290                 295                 300
   Asp Ile Glu Trp Ala Leu Asp Ala Asp Leu Pro Asp Gly Glu Asn Leu
   305                 310                 315                 320
   Leu Leu Leu Gln Ser Arg Pro Glu Thr Ile His Ser Asn Gly Val Lys
                   325                 330                 335
   Lys Glu Thr Pro Thr Pro Gln Ala Ala Lys Thr Ile Gly Thr Phe Asp
               340                 345                 350
   Phe Ser Ser Ile Thr Val Ala Met Thr Gly Thr Lys
           355                 360
<210> 99
   <211> 3926
   <212> DNA
   <213> Saccharomyces cerevisiae
<400> 99
   ttggtcatgc ttatgaagct atactaaccc cacaagcatt acatggtgaa tgtgtgtcca        60
   ttggtatggt taaagaggcg gaattatccc gttatttcgg tattctctcc cctacccaag       120
   ttgcacgtct atccaagatt ttggttgcct acgggttgcc tgtttcgcct gatgagaaat       180
   ggtttaaaga gctaacctta cataagaaaa caccattgga tatcttattg aagaaaatga       240
   gtattgacaa gaaaaacgag ggttccaaaa agaaggtggt cattttagaa agtattggta       300
   agtgctatgg tgactccgct caatttgtta gcgatgaaga cctgagattt attctaacag        360
   atgaaaccct cgtttacccc ttcaaggaca tccctgctga tcaacagaaa gttgttatcc        420
   cccctggttc taagtccatc tccaatcgtg ctttaattct tgctgccctc ggtgaaggtc        480
   aatgtaaaat caagaactta ttacattctg atgatactaa acatatgtta accgctgttc        540
   atgaattgaa aggtgctacg atatcatggg aagataatgg tgagacggta gtggtggaag        600
   gacatggtgg ttccacattg tcagcttgtg ctgacccctt atatctaggt aatgcaggta        660
   ctgcatctag atttttgact tccttggctg ccttggtcaa ttctacttca agccaaaagt        720
   atatcgtttt aactggtaac gcaagaatgc aacaaagacc aattgctcct ttggtcgatt        780
   ctttgcgtgc taatggtact aaaattgagt acttgaataa tgaaggttcc ctgccaatca        840
   aagtttatac tgattcggta ttcaaaggtg gtagaattga attagctgct acagtttctt        900
   ctcagtacgt atcctctatc ttgatgtgtg ccccatacgc tgaagaacct gtaactttgg        960
   ctcttgttgg tggtaagcca atctctaaat tgtacgtcga tatgacaata aaaatgatgg       1020
   aaaaattcgg tatcaatgtt gaaacttcta ctacagaacc ttacacttat tatattccaa       1080
   agggacatta tattaaccca tcagaatacg tcattgaaag tgatgcctca agtgctacat       1140
   acccattggc cttcgccgca atgactggta ctaccgtaac ggttccaaac attggttttg       1200
   agtcgttaca aggtgatgcc agatttgcaa gagatgtctt gaaacctatg ggttgtaaaa       1260
   taactcaaac ggcaacttca actactgttt cgggtcctcc tgtaggtact ttaaagccat       1320
   taaaacatgt tgatatggag ccaatgactg atgcgttctt aactgcatgt gttgttgccg       1380
   ctatttcgca cgacagtgat ccaaattctg caaatacaac caccattgaa ggtattgcaa       1440
   accagcgtgt caaagagtgt aacagaattt tggccatggc tacagagctc gccaaatttg       1500
   gcgtcaaaac tacagaatta ccagatggta ttcaagtcca tggtttaaac tcgataaaag       1560
   atttgaaggt tccttccgac tcttctggac ctgtcggtgt atgcacatat gatgatcatc       1620
   gtgtggccat gagtttctcg cttcttgcag gaatggtaaa ttctcaaaat gaacgtgacg       1680
   aagttgctaa tcctgtaaga atacttgaaa gacattgtac tggtaaaacc tggcctggct       1740
   ggtgggatgt gttacattcc gaactaggtg ccaaattaga tggtgcagaa cctttagagt       1800
   gcacatccaa aaagaactca aagaaaagcg ttgtcattat tggcatgaga gcagctggca       1860
   aaactactat aagtaaatgg tgcgcatccg ctctgggtta caaattagtt gacctagacg       1920
   agctgtttga gcaacagcat aacaatcaaa gtgttaaaca atttgttgtg gagaacggtt       1980
   gggagaagtt ccgtgaggaa gaaacaagaa ttttcaagga agttattcaa aattacggcg       2040
   atgatggata tgttttctca acaggtggcg gtattgttga aagcgctgag tctagaaaag       2100
   ccttaaaaga ttttgcctca tcaggtggat acgttttaca cttacatagg gatattgagg       2160
   agacaattgt ctttttacaa agtgatcctt caagacctgc ctatgtggaa gaaattcgtg 2220
   aagtttggaa cagaagggag gggtggtata aagaatgctc aaatttctct ttctttgctc 2280
   ctcattgctc cgcagaagct gagttccaag ctctaagaag atcgtttagt aagtacattg 2340
   caaccattac aggtgtcaga gaaatagaaa ttccaagcgg aagatctgcc tttgtgtgtt 2400
   taacctttga tgacttaact gaacaaactg agaatttgac tccaatctgt tatggttgtg 2460
   aggctgtaga ggtcagagta gaccatttgg ctaattactc tgctgatttc gtgagtaaac 2520
   agttatctat attgcgtaaa gccactgaca gtattcctat catttttact gtgcgaacca 2580
   tgaagcaagg tggcaacttt cctgatgaag agttcaaaac cttgagagag ctatacgata 2640
   ttgccttgaa gaatggtgtt gaattccttg acttagaact aactttacct actgatatcc 2700
   aatatgaggt tattaacaaa aggggcaaca ccaagatcat tggttcccat catgacttcc 2760
   aaggattata ctcctgggac gacgctgaat gggaaaacag attcaatcaa gcgttaactc 2820
   ttgatgtgga tgttgtaaaa tttgtgggta cggctgttaa tttcgaagat aatttgagac 2880
   tggaacactt tagggataca cacaagaata agcctttaat tgcagttaat atgacttcta 2940
   aaggtagcat ttctcgtgtt ttgaataatg ttttaacacc tgtgacatca gatttattgc 3000
   ctaactccgc tgcccctggc caattgacag tagcacaaat taacaagatg tatacatcta 3060
   tgggaggtat cgagcctaag gaactgtttg ttgttggaaa gccaattggc cactctagat 3120
   cgccaatttt acataacact ggctatgaaa ttttaggttt acctcacaag ttcgataaat 3180
   ttgaaactga atccgcacaa ttggtgaaag aaaaactttt ggacggaaac aagaactttg 3240
   gcggtgctgc agtcacaatt cctctgaaat tagatataat gcagtacatg gatgaattga 3300
   ctgatgctgc taaagttatt ggtgctgtaa acacagttat accattgggt aacaagaagt 3360
   ttaagggtga taataccgac tggttaggta tccgtaatgc cttaattaac aatggcgttc 3420
   ccgaatatgt tggtcatacc gctggtttgg ttatcggtgc aggtggcact tctagagccg 3480
   ccctttacgc cttgcacagt ttaggttgca aaaagatctt cataatcaac aggacaactt 3540
   cgaaattgaa gccattaata gagtcacttc catctgaatt caacattatt ggaatagagt 3600
   ccactaaatc tatagaagag attaaggaac acgttggcgt tgctgtcagc tgtgtaccag 3660
   ccgacaaacc attagatgac gaacttttaa gtaagctgga gagattcctt gtgaaaggtg 3720
   cccatgctgc ttttgtacca accttattgg aagccgcata caaaccaagc gttactcccg 3780
   ttatgacaat ttcacaagac aaatatcaat ggcacgttgt ccctggatca caaatgttag 3840
   tacaccaagg tgtagctcag tttgaaaagt ggacaggatt caagggccct ttcaaggcca 3900
   tttttgatgc cgttacgaaa gagtag 3926
<210> 100
   <211> 2247
   <212> DNA
   <213> Anabaena variabilis ATCC29413
<400> 100
   atgagtatcg tccaagcaaa gtttgaagct aaggaaacat cttttcatgt agaaggttac 60
   gaaaagattg agtatgattt ggtgtatgta gatggtattt ttgaaatcca gaattctgca 120
   ctagcagatg tatatcaagg ttttggacga tgcttggcga ttgtagatgc taacgtcagt 180
   cggttgtatg gtaatcaaat tcaggcatat ttccagtatt atggtataga actgaggcta 240
   tttcctatta ccattactga accagataag actattcaaa ctttcgagag agttatagat 300
   gtctttgcag atttcaaatt agtccgcaaa gaaccagtat tagtcgtggg tggcggttta 360
   attacagatg ttgtcggctt tgcttgttct acatatcgtc gcagcagcaa ttacatccgc 420
   attcctacta cattgattgg attaattgat gccagtgtag caattaaggt agcagttaat 480
   catcgcaaac tgaaaaaccg tttgggtgct tatcatgctt ctcgcaaagt atttttagat 540
   ttctccttgt tgcgtactct ccctacagac caagtacgta acgggatggc ggaattggta 600
   aaaatcgctg tagtagcgca tcaagaagtt tttgaattgt tggagaagta cggcgaagaa 660
   ttactacgta ctcattttgg caatatagat gcaactccag agattaaaga aatagcccat 720
   cgtttgactt acaaagctat ccataagatg ttggaattgg aagttcccaa cctgcatgag 780
   ttagacctag atagggtgat tgcttacggt cacacttgga gtcccacctt ggaacttgcg 840
   cctcgtctac ccatgttcca cggacacgcc gttaatgtag atatggcttt ctcggcaacg 900
   atcgccgccc gtagaggata tattacaatt gcagaacgcg atcgtatttt aggattaatg 960
   agtcgcgttg gtctatccct cgaccatccc atgttggata tagatatttt gtggcgtggt 1020
   actgaatcta tcacattaac tcgtgatggt ttgttaagag ctgctatgcc aaaacccatt 1080
   ggtgattgtg tcttcgtcaa tgacctgaca agagaagaat tagcagccgc attagctgac 1140
   cacaaagaac tttgtaccag ttatccccgt ggtggtgaag gtgtggatgt gtatcccgtt 1200
   tatcaaaaag aattaatcgg gagtgttaaa taatgacttt tttgaattca aaatgcaaaa 1260
   tactccacgg atacactgcg cgagcgcggt agcatttctg ttcgcggagc gtcccgtagg 1320
   gaaagagaag gctacgcaaa taatcggaca ctaattgtct ttaattttga attttgaatt 1380
   ttgaattttg aattggagcg aagcgacttg acaaatgtga ttgtccaacc aacagctaga 1440
   cctgttacac cattgggaat tttaaccaag cagttagaag ccatagtcca agaggttaag 1500
   caacatccag atttacctgg ggaattgata gcaaacatcc atcaggcttg gcgtttagcc 1560
   gcaggtatag acccttattt ggaagaatgc accactccag aatctcctga actcgctgca 1620
   ttggcaaaaa ccacagccac cgaagcctgg ggagaacact tccacggagg tacaaccgtc 1680
   cgtcctctag aacaagagat gctttctggt catatcgaag gacaaacctt aaagatgttt 1740
   gttcacatga ccaaagctaa aaaagtctta gaaattggga tgtttaccgg ttattcggcg 1800
   ctggcgatgg cggaagcatt accagaggat ggactgcttg tggcttgtga agttgaccct 1860
   tacgcggcgg aaattggaca gaaagccttt caacaatctc cccacggtgg aaagattcgt 1920
   gtggaattgg atgcagcctt agcaactctt gataagttag cagaagctgg ggagtctttt 1980
   gacttggtat ttatcgacgc agataaaaaa gagtatgtag cctattttca caagttgcta 2040
   ggtagcagtt tgttagcacc agatggcttt atttgtgtag ataacacctt attacaaggg 2100
   gaagtttatc taccagcaga ggaacgtagc gtcaatggtg aagcgatcgc gcaatttaat 2160
   catacagtag ctatagaccc ccgtgtagaa caggttttgt tgccgttgcg agatggttta 2220
   acaattatcc gcagaataca accttaa 2247
<210> 101
   <211> 3532
   <212> DNA
   <213> Anabaena variabilis ATCC29413
<400> 101
   tccagaattc tgcactagca gatgtatatc aaggttttgg acgatgcttg gcgattgtag          60
   atgctaacgt cagtcggttg tatggtaatc aaattcaggc atatttccag tattatggta         120
   tagaactgag gctatttcct attaccatta ctgaaccaga taagactatt caaactttcg         180
   agagagttat agatgtcttt gcagatttca aattagtccg caaagaacca gtattagtcg         240
   tgggtggcgg tttaattaca gatgttgtcg gctttgcttg ttctacatat cgtcgcagca         300
   gcaattacat ccgcattcct actacattga ttggattaat tgatgccagt gtagcaatta         360
   aggtagcagt taatcatcgc aaactgaaaa accgtttggg tgcttatcat gcttctcgca         420
   aagtattttt agatttctcc ttgttgcgta ctctccctac agaccaagta cgtaacggga         480
   tggcggaatt ggtaaaaatc gctgtagtag cgcatcaaga agtttttgaa ttgttggaga         540
   agtacggcga agaattacta cgtactcatt ttggcaatat agatgcaact ccagagatta         600
   aagaaatagc ccatcgtttg acttacaaag ctatccataa gatgttggaa ttggaagttc         660
   ccaacctgca tgagttagac ctagataggg tgattgctta cggtcacact tggagtccca         720
   ccttggaact tgcgcctcgt ctacccatgt tccacggaca cgccgttaat gtagatatgg         780
   ctttctcggc aacgatcgcc gcccgtagag gatatattac aattgcagaa cgcgatcgta         840
   ttttaggatt aatgagtcgc gttggtctat ccctcgacca tcccatgttg gatatagata         900
   ttttgtggcg tggtactgaa tctatcacat taactcgtga tggtttgtta agagctgcta         960
   tgccaaaacc cattggtgat tgtgtcttcg tcaatgacct gacaagagaa gaattagcag        1020
   ccgcattagc tgaccacaaa gaactttgta ccagttatcc ccgtggtggt gaaggtgtgg        1080
   atgtgtatcc cgtttatcaa aaagaattaa tcgggagtgt taaataatga cttttttgaa        1140
   ttcaaaatgc aaaatactcc acggatacac tgcgcgagcg cggtagcatt tctgttcgcg        1200
   gagcgtcccg tagggaaaga gaaggctacg caaataatcg gacactaatt gtctttaatt 1260
   ttgaattttg aattttgaat tttgaattgg agcgaagcga cttgacaaat gtgattgtcc 1320
   aaccaacagc tagacctgtt acaccattgg gaattttaac caagcagtta gaagccatag 1380
   tccaagaggt taagcaacat ccagatttac ctggggaatt gatagcaaac atccatcagg 1440
   cttggcgttt agccgcaggt atagaccctt atttggaaga atgcaccact ccagaatctc 1500
   ctgaactcgc tgcattggca aaaaccacag ccaccgaagc ctggggagaa cacttccacg 1560
   gaggtacaac cgtccgtcct ctagaacaag agatgctttc tggtcatatc gaaggacaaa 1620
   ccttaaagat gtttgttcac atgaccaaag ctaaaaaagt cttagaaatt gggatgttta 1680
   ccggttattc ggcgctggcg atggcggaag cattaccaga ggatggactg cttgtggctt 1740
   gtgaagttga cccttacgcg gcggaaattg gacagaaagc ctttcaacaa tctccccacg 1800
   gtggaaagat tcgtgtggaa ttggatgcag ccttagcaac tcttgataag ttagcagaag 1860
   ctggggagtc ttttgacttg gtatttatcg acgcagataa aaaagagtat gtagcctatt 1920
   ttcacaagtt gctaggtagc agtttgttag caccagatgg ctttatttgt gtagataaca 1980
   ccttattaca aggggaagtt tatctaccag cagaggaacg tagcgtcaat ggtgaagcga 2040
   tcgcgcaatt taatcataca gtagctatag acccccgtgt agaacaggtt ttgttgccgt 2100
   tgcgagatgg tttaacaatt atccgcagaa tacaacctta attgtccaat cgactatggc 2160
   acaatccctt ccccttcttt ccgcacctgc tacaccgtct cttccttccc agacgaaaat 2220
   agccgcaatt atccaaaata tctgcacttt ggctttgtta ttactagcat tgcccattaa 2280
   tgccaccatt gtttttatat ccttgttagt cttccgaccg caaaaggtca aagcagcaaa 2340
   cccccaaacc attcttatca gtggcggtaa gatgaccaaa gctttacaac tagcaaggtc 2400
   attccacgcg gctggacata gagttgtctt ggtggaaacc cataaatact ggttgactgg 2460
   tcatcgtttt tcccaagcag tggataagtt ttacacagtc cccgcacccc aggacaatcc 2520
   ccaagcttac attcaggctt tggtagatat cgtcaaacaa gaaaacatcg atgtttatat 2580
   tcccgtcacc agtccagtgg gtagctacta cgactcatta gccaaaccag agttatccca 2640
   ttattgcgaa gtgtttcact ttgacgcaga tattacccaa atgttggatg ataaatttgc 2700
   gttgacacaa aaagcgcgat cgcttggttt atcagtaccc aaatccttta aaattacctc 2760
   accagaacaa gtcatcaact tcgatttttc tggagagaca cgtaaataca tcctcaaaag 2820
   cattccctac gactcagtgc ggcggttgga cttaaccaaa ctcccctgtg ctactccaga 2880
   ggaaacagca gcattcgtca gaagtttgcc aattactccc gaaaaaccgt ggattatgca 2940
   ggaatttatc cccggtaagg aattctgcac ccatagcacc gttcggaatg gggaactcag 3000
   actgcattgc tgttgcgaat cttcagcctt ccaagttaat tatgagaatg taaataaccc 3060
   gcaaattacc gaatgggtac agcattttgt caaggaactg aaactgacag gacagatttc 3120
   ctttgacttt atccaagccg aagacggaac agtttacgcc atcgagtgta acccccgcac 3180
   acattcagca attaccacat tttacgacca cccccaggta gcagaagcgt acttgagtca 3240
   agcaccgacg actgaaacca tacaaccact aacgacaagc aagcctacct attggactta 3300
   tcacgaagtt tggcgtttaa ctggtatccg ttctttcacc cagttgcaaa gatggctggg 3360
   gaatatttgg cgcgggactg atgcgattta tcagccagat gaccccttac cgtttttgat 3420
   ggtacatcat tggcaaattc ccctactgtt attgaataat ttgcgtcgtc ttaaaggttg 3480
   gacgcggata gatttcaata ttgggaagtt ggtggaattg gggggagatt ag 3532
<210> 102
   <211> 1233
   <212> DNA
   <213> Anabaena variabilis ATCC29413
<400> 102
   atgagtatcg tccaagcaaa gtttgaagct aaggaaacat cttttcatgt agaaggttac      60
   gaaaagattg agtatgattt ggtgtatgta gatggtattt ttgaaatcca gaattctgca     120
   ctagcagatg tatatcaagg ttttggacga tgcttggcga ttgtagatgc taacgtcagt     180
   cggttgtatg gtaatcaaat tcaggcatat ttccagtatt atggtataga actgaggcta     240
   tttcctatta ccattactga accagataag actattcaaa ctttcgagag agttatagat     300
   gtctttgcag atttcaaatt agtccgcaaa gaaccagtat tagtcgtggg tggcggttta     360
   attacagatg ttgtcggctt tgcttgttct acatatcgtc gcagcagcaa ttacatccgc     420
   attcctacta cattgattgg attaattgat gccagtgtag caattaaggt agcagttaat     480
   catcgcaaac tgaaaaaccg tttgggtgct tatcatgctt ctcgcaaagt atttttagat     540
   ttctccttgt tgcgtactct ccctacagac caagtacgta acgggatggc ggaattggta     600
   aaaatcgctg tagtagcgca tcaagaagtt tttgaattgt tggagaagta cggcgaagaa     660
   ttactacgta ctcattttgg caatatagat gcaactccag agattaaaga aatagcccat     720
   cgtttgactt acaaagctat ccataagatg ttggaattgg aagttcccaa cctgcatgag     780
   ttagacctag atagggtgat tgcttacggt cacacttgga gtcccacctt ggaacttgcg     840
   cctcgtctac ccatgttcca cggacacgcc gttaatgtag atatggcttt ctcggcaacg     900
   atcgccgccc gtagaggata tattacaatt gcagaacgcg atcgtatttt aggattaatg     960
   agtcgcgttg gtctatccct cgaccatccc atgttggata tagatatttt gtggcgtggt    1020
   actgaatcta tcacattaac tcgtgatggt ttgttaagag ctgctatgcc aaaacccatt    1080
   ggtgattgtg tcttcgtcaa tgacctgaca agagaagaat tagcagccgc attagctgac    1140
   cacaaagaac tttgtaccag ttatccccgt ggtggtgaag gtgtggatgt gtatcccgtt    1200
   tatcaaaaag aattaatcgg gagtgttaaa taa                                 1233
<210> 103
   <211> 840
   <212> DNA
   <213> Anabaena variabilis ATCC29413
<400> 103
<210> 104
   <211> 1377
   <212> DNA
   <213> Anabaena variabilis ATCC29413
<400> 104
   atggcacaat cccttcccct ttcttccgca cctgctacac cgtctcttcc ttcccagacg 60
   aaaatagccg caattatcca aaatatctgc actttggctt tgttattact agcattgccc 120
   attaatgcca ccattgtttt tatatccttg ttagtcttcc gaccgcaaaa ggtcaaagca 180
   gcaaaccccc aaaccattct tatcagtggc ggtaagatga ccaaagcttt acaactagca 240
   aggtcattcc acgcggctgg acatagagtt gtcttggtgg aaacccataa atactggttg 300
   actggtcatc gtttttccca agcagtggat aagttttaca cagtccccgc accccaggac 360
   aatccccaag cttacattca ggctttggta gatatcgtca aacaagaaaa catcgatgtt 420
   tatattcccg tcaccagtcc agtgggtagc tactacgact cattagccaa accagagtta 480
   tcccattatt gcgaagtgtt tcactttgac gcagatatta cccaaatgtt ggatgataaa 540
   tttgcgttga cacaaaaagc gcgatcgctt ggtttatcag tacccaaatc ctttaaaatt 600
   acctcaccag aacaagtcat caacttcgat ttttctggag agacacgtaa atacatcctc 660
   aaaagcattc cctacgactc agtgcggcgg ttggacttaa ccaaactccc ctgtgctact 720
   ccagaggaaa cagcagcatt cgtcagaagt ttgccaatta ctcccgaaaa accgtggatt 780
   atgcaggaat ttatccccgg taaggaattc tgcacccata gcaccgttcg gaatggggaa 840
   ctcagactgc attgctgttg cgaatcttca gccttccaag ttaattatga gaatgtaaat 900
   aacccgcaaa ttaccgaatg ggtacagcat tttgtcaagg aactgaaact gacaggacag 960
   atttcctttg actttatcca agccgaagac ggaacagttt acgccatcga gtgtaacccc 1020
   cgcacacatt cagcaattac cacattttac gaccaccccc aggtagcaga agcgtacttg 1080
   agtcaagcac cgacgactga aaccatacaa ccactaacga caagcaagcc tacctattgg 1140
   acttatcacg aagtttggcg tttaactggt atccgttctt tcacccagtt gcaaagatgg 1200
   ctggggaata tttggcgcgg gactgatgcg atttatcagc cagatgaccc cttaccgttt 1260
   ttgatggtac atcattggca aattccccta ctgttattga ataatttgcg tcgtcttaaa 1320
   ggttggacgc ggatagattt caatattggg aagttggtgg aattgggggg agattag 1377
<210> 105
   <211> 2358
   <212> DNA
   <213> Anabaena variabilis ATCC29413
<400> 105
   gttcctattg accctgaata tcctcaagaa cgcatagctt atatgctaga agattctcag      60
   gtgaaggtac tactaactca agaaaaatta ctcaatcaaa ttccccacca tcaagcacaa     120
   actatctgtg tagataggga atgggagaaa atttccacac aagctaatac caatcccaaa     180
   agtaatataa aaacggataa tcttgcttat gtaatttaca cctctggttc cactggtaaa     240
   ccaaaaggtg caatgaacac ccacaaaggt atctgtaatc gcttattgtg gatgcaggaa     300
   gcttatcaaa tcgattccac agatagcatt ttacaaaaaa ccccctttag ttttgatgtt     360
   tccgtttggg agttcttttg gactttatta actggcgcac gtttggtaat agccaaacca     420
   ggcggacata aagatagtgc ttacctcatc gatttaatta ctcaagaaca aatcactacg     480
   ttgcattttg tcccctcaat gctgcaagtg tttttacaaa atcgccatgt aagcaaatgc     540
   agctctctaa aaagagttat ttgtagcggt gaagctttat ctatagattt acaaaataga     600
   tttttccagc atttgcaatg tgaattacat aacctctatg gcccgacaga agcagcaatt     660
   gatgtcacat tttggcaatg tagaaaagat agtaatttaa agagtgtacc tattggtcgt     720
   cccattgcta atactcaaat ttatattctt gatgccgatt tacaaccagt aaatattggt     780
   gtcactggtg aaatttatat tggtggtgta ggggttgctc gtggttattt gaataaagaa     840
   gaattgacca aagaaaaatt tattattaat ccctttccca attctgagtt taagcgactt 900
   tataaaacag gtgatttagc tcgttattta cccgatggaa atattgaata tcttggtaga 960
   acagattatc aagtaaaaat tcggggttat agaattgaaa ttggcgagat tgaaaatgtt 1020
   ttatcttcac acccacaagt cagagaagct gtagtcatag cgcgggatga taacgctcaa 1080
   gaaaaacaaa tcatcgctta tattacctat aactccatca aacctcagct tgataatctg 1140
   cgtgatttcc taaaagcaag gctacctgat tttatgattc cagccgcttt tgtgatgctg 1200
   gagcatcttc ctttaactcc cagtggtaaa gtagaccgta aggcattacc taagcctgat 1260
   ttatttaatt atagtgaaca taattcctat gtagcgcctc ggaatgaagt tgaagaaaaa 1320
   ttagtacaaa tctggtcgaa tattctgcat ttacctaaag taggtgtgac agaaaacttt 1380
   ttcgctattg gtggtaattc cctcaaagct ctacatttaa tttctcaaat tgaagagtta 1440
   tttgctaaag agatatcctt agcaacactt ttaacaaatc cagtaattgc agatttagcc 1500
   aaggttattc aagcaaacaa ccaaatccat aattcacccc tagttccaat tcaaccacaa 1560
   ggtaagcagc agcctttctt ttgtatacat cctgctggtg gtcatgtttt atgctatttt 1620
   aaactcgcac aatatatagg aactgaccaa ccattttatg gcttacaagc tcaaggattt 1680
   tatggagatg aagcaccctt gacgcgagtt gaagatatgg ctagtctcta cgtcaaaact 1740
   attagagaat ttcaacccca agggccttat cgtgtcgggg ggtggtcatt tggtggagtc 1800
   gtagcttatg aagtagcaca gcagttacat agacaaggac aagaagtatc tttactagca 1860
   atattagatt cttacgtacc gattctgctg gataaacaaa aacccattga tgacgtttat 1920
   ttagttggtg ttctctccag agtttttggc ggtatgtttg gtcaagataa tctagtcaca 1980
   cctgaagaaa tagaaaattt aactgtagaa gaaaaaatta attacatcat tgataaagca 2040
   cggagcgcta gaatattccc gcctggtgta gaacgtcaaa ataatcgccg tattcttgat 2100
   gttttggtgg gaactttaaa agcaacttat tcctatataa gacaaccata tccaggaaaa 2160
   gtcactgtat ttcgagccag ggaaaaacat attatggctc ctgacccgac cttagtttgg 2220
   gtagaattat tttctgtaat ggcggctcaa gaaattaaga ttattgatgt ccctggaaac 2280
   cattattcgt ttgttctaga accccatgta caggttttag cacagcgttt acaagattgt 2340
   ctggaaaata attcataa 2358
<210> 106
   <211> 1233
   <212> DNA
   <213> Nostoc punctiforme ATCC29133
<400> 106
   atgagtaatg ttcaagcatc gtttgaagca acggaagctg aattccgcgt ggaaggttac      60
   gaaaaaattg agtttagtct tgtctatgta aatggtgcat ttgatatcag taacagagaa     120
   attgcagaca gctatgagaa gtttggccgt tgtctgactg tgattgatgc taatgtcaac     180
   agattatatg gcaagcaaat caagtcatat tttagacact atggtattga tctgaccgta     240
   gttcccattg tgattactga gcctactaaa acccttgcaa cctttgagaa aattgttgat     300
   gctttttctg actttggttt aatccgcaag gaaccagtat tagtagtggg tggtggttta     360
   accactgatg tagctgggtt tgcgtgtgct gcttaccgtc gtaagagtaa ctatattcgg     420
   gttccgacaa cgctgattgg tctgattgat gcaggtgtag cgattaaggt tgcagtcaac     480
   catcgcaagt taaaaaatcg cttgggtgca tatcatgctc ctttgaaagt catcctcgat     540
   ttctccttct tgcaaacatt accaacggct caagttcgta atgggatggc agagttggtc     600
   aaaattgctg ttgtggcgaa ctctgaagtc tttgaattgt tgtatgaata tggcgaagag     660
   ttgctttcca ctcactttgg atatgtgaat ggtacaaagg aactgaaagc gatcgcacat     720
   aaactcaatt acgaggcaat aaaaactatg ctggagttgg aaactccaaa cttgcatgag     780
   ttagacctcg atcgcgtcat tgcctacggt cacacttgga gtccgacctt agaattagct     840
   ccgatgatac cgttgtttca cggtcatgcc gtcaatatag acatggcttt gtctgcaact     900
   attgcggcaa gacgtggcta cattacatca ggagaacgcg atcgcatttt gagcttgatg     960
   agtcgtatag gtttatcaat cgatcatcct ctactagatg gcgatttgct ctggtatgct    1020
   acccaatcta ttagcttgac gcgagacggg aaacaacgcg cagctatgcc taaacccatt    1080
   ggtgagtgct tctttgtcaa cgatttcacc cgtgaagaac tagatgcagc tttagctgaa    1140
   cacaaacgtc tgtgtgctac ataccctcgt ggtggagatg gcattgacgc ttacatagaa    1200
   actcaagaag aatccaaact attgggagtg tga                                 1233
<210> 107
   <211> 834
   <212> DNA
   <213> Nostoc punctiforme ATCC29133
<400> 107
   atgaccagta ttttaggacg agataccgca agaccaataa cgccacatag cattctggta      60
   gcacagctac aaaaaaccct cagaatggca gaggaaagta atattccttc agagatactg     120
   acttctctgc gccaagggtt gcaattagca gcaggtttag atccctatct ggatgattgc     180
   actacccctg aatcgaccgc attgacagca ctagcgcaga agacaagcat tgaagactgg     240
   agtaaacgct tcagtgatgg tgaaacagtg cgtcaattag agcaagaaat gctctcagga     300
   catcttgaag gacaaacact aaagatgttt gtgcatatca ctaaggctaa aagcatccta     360
   gaagtgggaa tgttcacagg atattcagct ttggcaatgg cagaggcgtt accagatgat     420
   gggcgactga ttgcttgtga agtagactcc tatgtggccg agtttgctca aacttgcttt     480
   caagagtctc cccacggccg caagattgtt gtagaagtgg cacctgcact agagacgctg     540
   cacaagctgg tggctaaaaa agaatccttt gatctgatct tcattgatgc ggataaaaag     600
   gagtatatag aatacttcca aattatcttg gatagccatt tactagctcc cgacggatta     660
   atctgtgtag ataatacttt gttgcaagga caagtttacc tgccatcaga acagcgtaca     720
   gccaatggtg aagcgatcgc tcaattcaac cgcattgtcg ccgcagatcc tcgtgtagag     780
   caagttctgt tacccatacg agatggtata accctgatta gacgcttggt ataa           834
<210> 108
   <211> 1086
   <212> DNA
   <213> Nostoc punctiforme ATCC29133
<400> 108
   tactggttat cagggcatag attctcaaat tctgtgagtc gtttttatac agttcctgca      60
   ccacaagacg acccagaagg ctatacccaa gcgctattgg aaattgtcaa acgagagaag     120
   attgacgttt atgtacccgt atgcagccct gtagctagtt attacgactc tttggcaaag     180
   tctgcactat cagaatattg tgaggttttt cactttgatg ctgatataac caagatgctg     240
   gatgataaat ttgcctttac cgatcgggcg cgatcgcttg gtttatcagc cccgaaatct     300
   tttaaaatta ccgatcctga acaagttatc aacttcgatt ttagtaaaga gacgcgcaaa     360
   tatattctta agagtatttc ttacgactca gttcgccgct taaatttaac caaacttcct     420
   tgtgataccc cagaagagac agcagcgttt gtcaagagtt tacccatcag cccagaaaaa     480
   ccttggatta tgcaagaatt tattcctggg aaagaattat gcacccatag cacagtccga     540
   gacggcgaat taaggttgca ttgctgttca aattcttcag cgtttcagat taactatgaa     600
   aatgtcgaaa atccccaaat tcaagaatgg gtacaacatt tcgtcaaaag tttacggctg     660
   actggacaaa tatctcttga ctttatccaa gctgaagatg gtacagctta tgccattgaa     720
   tgtaatcctc gcacccattc ggcgatcaca atgttctaca atcacccagg tgttgcagaa     780
   gcctatcttg gtaaaactcc tctagctgca cctttggaac ctcttgcaga tagcaagccc     840
   acttactgga tatatcacga aatctggcga ttgactggga ttcgctctgg acaacaatta     900
   caaacttggt ttgggagatt agtcagaggt acagatgcca tttatcgcct ggacgatccg     960
   ataccatttt taactttgca ccattggcag attactttac ttttgctaca aaatttgcaa    1020
   cgactcaaag gttgggtaaa gatcgatttt aatatcggta aactcgtgga attagggggc    1080
   gactaa                                                              1086
<210> 109
   <211> 1047
   <212> DNA
   <213> Nostoc punctiforme ATCC29133
<400> 109
   atgccagtac ttaatatcct tcatttagtt gggtctgcac acgataagtt ttactgtgat      60
   ttatcacgtc tttatgccca agactgttta gctgcaacag cagatccatc gctttataac     120
   tttcaaattg catatatcac acccgatcgg cagtggcgat ttcctgactc tctcagtcga     180
   gaagatattg ctcttaccaa accgattcct gtgtttgatg ccatacaatt tctaacaggc     240
   caaaacattg acatgatgtt accacaaatg ttttgtattc ctggaatgac tcagtaccgt     300
   gccctattcg atctgctcaa gatcccttat ataggaaata ccccagatat tatggcgatc     360
   gcggcccaca aagccagagc caaagcaatt gtcgaagcag caggggtaaa agtgcctcgt     420
   ggagaattgc ttcgccaagg agatattcca acaattacac ctccagcagt cgtcaaacct     480
   gtaagttctg acaactcttt aggagtagtc ttagttaaag atgtgactga atatgatgct     540
   gccttaaaga aagcatttga atatgcttcg gaggtcatcg tagaagcatt catcgaactt     600
   ggtcgagaag tcagatgcgg catcattgta aaagacggtg agctaatagg tttacccctt     660
   gaagagtatc tggtagaccc acacgataaa cctatccgta actatgctga taaactccaa     720
   caaactgacg atggcgactt gcatttgact gctaaagata atatcaaggc ttggatttta     780
   gaccctaacg acccaatcac ccaaaaggtt cagcaagtgg ctaaaaggtg tcatcaggct     840
   ttgggttgtc gccactacag tttatttgac ttccgaatcg atccaaaggg acaaccttgg     900
   ttcttagaag ctggattata ttgttctttt gcccccaaaa gtgtgatttc ttctatggcg     960
   aaagcagccg gaatccctct aaatgattta ttaataaccg ctattaatga aacattgggt    1020
   agtaataaaa aggtgttaca aaattga                                        1047
<210> 110
   <211> 1626
   <212> DNA
   <213> Saccharomyces cerevisiae CEN.PK-1D
<400> 110
   aacaagccgg gctttacctt gtctgacaac tacacctatg ttttcttggg tgacggttgt 60
   ttgcaagaag gtatttcttc agaagcttcc tccttggctg gtcatttgaa attgggtaac 120
   ttgattgcca tctacgatga caacaagatc actatcgatg gtgctaccag tatctcattc 180
   gatgaagatg ttgctaagag atacgaagcc tacggttggg aagttttgta cgtagaaaat 240
   ggtaacgaag atctagccgg tattgccaag gctattgctc aagctaagtt atccaaggac 300
   aaaccaactt tgatcaaaat gaccacaacc attggttacg gttccttgca tgccggctct 360
   cactctgtgc acggtgcccc attgaaagca gatgatgtta aacaactaaa gagcaaattc 420
   ggtttcaacc cagacaagtc ctttgttgtt ccacaagaag tttacgacca ctaccaaaag 480
   acaattttaa agccaggtgt cgaagccaac aacaagtgga acaagttgtt cagcgaatac 540
   caaaagaaat tcccagaatt aggtgctgaa ttggctagaa gattgagcgg ccaactaccc 600
   gcaaattggg aatctaagtt gccaacttac accgccaagg actctgccgt ggccactaga 660
   aaattatcag aaactgttct tgaggatgtt tacaatcaat tgccagagtt gattggtggt 720
   tctgccgatt taacaccttc taacttgacc agatggaagg aagcccttga cttccaacct 780
   ccttcttccg gttcaggtaa ctactctggt agatacatta ggtacggtat tagagaacac 840
   gctatgggtg ccataatgaa cggtatttca gctttcggtg ccaactacaa accatacggt 900
   ggtactttct tgaacttcgt ttcttatgct gctggtgccg ttagattgtc cgctttgtct 960
   ggccacccag ttatttgggt tgctacacat gactctatcg gtgtcggtga agatggtcca 1020
   acacatcaac ctattgaaac tttagcacac ttcagatccc taccaaacat tcaagtttgg 1080
   agaccagctg atggtaacga agtttctgcc gcctacaaga actctttaga atccaagcat 1140
   actccaagta tcattgcttt gtccagacaa aacttgccac aattggaagg tagctctatt 1200
   gaaagcgctt ctaagggtgg ttacgtacta caagatgttg ctaacccaga tattatttta 1260
   gtggctactg gttccgaagt gtctttgagt gttgaagctg ctaagacttt ggccgcaaag 1320
   aacatcaagg ctcgtgttgt ttctctacca gatttcttca cttttgacaa acaaccccta 1380
   gaatacagac tatcagtctt accagacaac gttccaatca tgtctgttga agttttggct 1440
   accacatgtt ggggcaaata cgctcatcaa tccttcggta ttgacagatt tggtgcctcc 1500
   ggtaaggcac cagaagtctt caagttcttc ggtttcaccc cagaaggtgt tgctgaaaga 1560
   gctcaaaaga ccattgcatt ctataagggt gacaagctaa tttctccttt gaaaaaagct 1620
   ttctaa 1626
<210> 111
   <211> 1113
   <212> DNA
   <213> Saccharomyces cerevisiae CEN.PK-1D
<400> 111
   atgagtgaat ctccaatgtt cgctgccaac ggcatgccaa aggtaaatca aggtgctgaa      60
   gaagatgtca gaattttagg ttacgaccca ttagcttctc cagctctcct tcaagtgcaa     120
   atcccagcca caccaacttc tttggaaact gccaagagag gtagaagaga agctatagat     180
   attattaccg gtaaagacga cagagttctt gtcattgtcg gtccttgttc catccatgat     240
   ctagaagccg ctcaagaata cgctttgaga ttaaagaaat tgtcagatga attaaaaggt     300
   gatttatcca tcattatgag agcatacttg gagaagccaa gaacaaccgt cggctggaaa     360
   ggtctaatta atgaccctga tgttaacaac actttcaaca tcaacaaggg tttgcaatcc     420
   gctagacaat tgtttgtcaa cttgacaaat atcggtttgc caattggttc tgaaatgctt     480
   gataccattt ctcctcaata cttggctgat ttggtctcct tcggtgccat tggtgccaga     540
   accaccgaat ctcaactgca cagagaattg gcctccggtt tgtctttccc agttggtttc     600
   aagaacggta ccgatggtac cttaaatgtt gctgtggatg cttgtcaagc cgctgctcat     660
   tctcaccatt tcatgggtgt tactaagcat ggtgttgctg ctatcaccac tactaagggt     720
   aacgaacact gcttcgttat tctaagaggt ggtaaaaagg gtaccaacta cgacgctaag     780
   tccgttgcag aagctaaggc tcaattgcct gccggttcca acggtctaat gattgactac     840
   tctcacggta actccaataa ggatttcaga aaccaaccaa aggtcaatga cgttgtttgt     900
   gagcaaatcg ctaacggtga aaacgccatt accggtgtca tgattgaatc aaacatcaac     960
   gaaggtaacc aaggcatccc agccgaaggt aaagccggct tgaaatatgg tgtttccatc    1020
   actgatgctt gtataggttg ggaaactact gaagacgtct tgaggaaatt ggctgctgct    1080
   gtcagacaaa gaagagaagt taacaagaaa tag                                 1113
<210> 112
   <211> 1588
   <212> PRT
   <213> Saccharomyces cerevisiae
<400> 112
   Met Val Gln Leu Ala Lys Val Pro Ile Leu Gly Asn Asp Ile Ile His
   1               5                   10                  15
   Val Gly Tyr Asn Ile His Asp His Leu Val Glu Thr Ile Ile Lys His
               20                  25                  30
   Cys Pro Ser Ser Thr Tyr Val Ile Cys Asn Asp Thr Asn Leu Ser Lys
           35                  40                  45
   Val Pro Tyr Tyr Gln Gln Leu Val Leu Glu Phe Lys Ala Ser Leu Pro
       50                  55                  60
   Glu Gly Ser Arg Leu Leu Thr Tyr Val Val Lys Pro Gly Glu Thr Ser
   65                  70                  75                  80
   Lys Ser Arg Glu Thr Lys Ala Gln Leu Glu Asp Tyr Leu Leu Val Glu
                   85                  90                  95
   Gly Cys Thr Arg Asp Thr Val Met Val Ala Ile Gly Gly Gly Val Ile
               100                 105                 110
   Gly Asp Met Ile Gly Phe Val Ala Ser Thr Phe Met Arg Gly Val Arg
           115                 120                 125
   Val Val Gln Val Pro Thr Ser Leu Leu Ala Met Val Asp Ser Ser Ile
       130                 135                 140
   Gly Gly Lys Thr Ala Ile Asp Thr Pro Leu Gly Lys Asn Phe Ile Gly
   145                 150                 155                 160
   Ala Phe Trp Gln Pro Lys Phe Val Leu Val Asp Ile Lys Trp Leu Glu
                   165                 170                 175
   Thr Leu Ala Lys Arg Glu Phe Ile Asn Gly Met Ala Glu Val Ile Lys
               180                 185                 190
   Thr Ala Cys Ile Trp Asn Ala Asp Glu Phe Thr Arg Leu Glu Ser Asn
           195                 200                 205
   Ala Ser Leu Phe Leu Asn Val Val Asn Gly Ala Lys Asn Val Lys Val
   210 215 220
   Thr Asn Gln Leu Thr Asn Glu Ile Asp Glu Ile Ser Asn Thr Asp Ile
   225 230 235 240
   Glu Ala Met Leu Asp His Thr Tyr Lys Leu Val Leu Glu Ser Ile Lys
   245 250 255
   Val Lys Ala Glu Val Val Ser Ser Asp Glu Arg Glu Ser Ser Leu Arg
   260 265 270
   Asn Leu Leu Asn Phe Gly His Ser Ile Gly His Ala Tyr Glu Ala Ile
   275 280 285
   Leu Thr Pro Gln Ala Leu His Gly Glu Cys Val Ser Ile Gly Met Val
   290 295 300
   Lys Glu Ala Glu Leu Ser Arg Tyr Phe Gly Ile Leu Ser Pro Thr Gln
   305 310 315 320
   Val Ala Arg Leu Ser Lys Ile Leu Val Ala Tyr Gly Leu Pro Val Ser
   325 330 335
   Pro Asp Glu Lys Trp Phe Lys Glu Leu Thr Leu His Lys Lys Thr Pro
   340 345 350
   Leu Asp Ile Leu Leu Lys Lys Met Ser Ile Asp Lys Lys Asn Glu Gly
   355 360 365
   Ser Lys Lys Lys Val Val Ile Leu Glu Ser Ile Gly Lys Cys Tyr Gly
   370 375 380
   Asp Ser Ala Gln Phe Val Ser Asp Glu Asp Leu Arg Phe Ile Leu Thr
   385 390 395 400
   Asp Glu Thr Leu Val Tyr Pro Phe Lys Asp Ile Pro Ala Asp Gln Gln
   405 410 415
   Lys Val Val Ile Pro Pro Gly Ser Lys Ser Ile Ser Asn Arg Ala Leu
   420 425 430
   Ile Leu Ala Ala Leu Gly Glu Gly Gln Cys Lys Ile Lys Asn Leu Leu
   435 440 445
   His Ser Asp Asp Thr Lys His Met Leu Thr Ala Val His Glu Leu Lys
   450 455 460
   Gly Ala Thr Ile Ser Trp Glu Asp Asn Gly Glu Thr Val Val Val Glu
   465 470 475 480
   Gly His Gly Gly Ser Thr Leu Ser Ala Cys Ala Asp Pro Leu Tyr Leu
   485 490 495
   Gly Asn Ala Gly Thr Ala Ser Arg Phe Leu Thr Ser Leu Ala Ala Leu
   500 505 510
   Val Asn Ser Thr Ser Ser Gln Lys Tyr Ile Val Leu Thr Gly Asn Ala
   515 520 525
   Arg Met Gln Gln Arg Pro Ile Ala Pro Leu Val Asp Ser Leu Arg Ala
   530 535 540
   Asn Gly Thr Lys Ile Glu Tyr Leu Asn Asn Glu Gly Ser Leu Pro Ile
   545                 550                 555                 560
   Lys Val Tyr Thr Asp Ser Val Phe Lys Gly Gly Arg Ile Glu Leu Ala
                   565                 570                 575
   Ala Thr Val Ser Ser Gln Tyr Val Ser Ser Ile Leu Met Cys Ala Pro
               580                 585                 590
   Tyr Ala Glu Glu Pro Val Thr Leu Ala Leu Val Gly Gly Lys Pro Ile
           595                 600                 605
   Ser Lys Leu Tyr Val Asp Met Thr Ile Lys Met Met Glu Lys Phe Gly
       610                 615                 620
   Ile Asn Val Glu Thr Ser Thr Thr Glu Pro Tyr Thr Tyr Tyr Ile Pro
   625                 630                 635                 640
   Lys Gly His Tyr Ile Asn Pro Ser Glu Tyr Val Ile Glu Ser Asp Ala
                   645                 650                 655
   Ser Ser Ala Thr Tyr Pro Leu Ala Phe Ala Ala Met Thr Gly Thr Thr
               660                 665                 670
   Val Thr Val Pro Asn Ile Gly Phe Glu Ser Leu Gln Gly Asp Ala Arg
           675                 680                 685
   Phe Ala Arg Asp Val Leu Lys Pro Met Gly Cys Lys Ile Thr Gln Thr
       690                 695                 700
   Ala Thr Ser Thr Thr Val Ser Gly Pro Pro Val Gly Thr Leu Lys Pro
   705                 710                 715                 720
   Leu Lys His Val Asp Met Glu Pro Met Thr Asp Ala Phe Leu Thr Ala
                   725                 730                 735
   Cys Val Val Ala Ala Ile Ser His Asp Ser Asp Pro Asn Ser Ala Asn
               740                 745                 750
   Thr Thr Thr Ile Glu Gly Ile Ala Asn Gln Arg Val Lys Glu Cys Asn
           755                 760                 765
   Arg Ile Leu Ala Met Ala Thr Glu Leu Ala Lys Phe Gly Val Lys Thr
       770                 775                 780
   Thr Glu Leu Pro Asp Gly Ile Gln Val His Gly Leu Asn Ser Ile Lys
   785                 790                 795                 800
   Asp Leu Lys Val Pro Ser Asp Ser Ser Gly Pro Val Gly Val Cys Thr
                   805                 810                 815
   Tyr Asp Asp His Arg Val Ala Met Ser Phe Ser Leu Leu Ala Gly Met
               820                 825                 830
   Val Asn Ser Gln Asn Glu Arg Asp Glu Val Ala Asn Pro Val Arg Ile
           835                 840                 845
   Leu Glu Arg His Cys Thr Gly Lys Thr Trp Pro Gly Trp Trp Asp Val
       850                 855                 860
   Leu His Ser Glu Leu Gly Ala Lys Leu Asp Gly Ala Glu Pro Leu Glu
   865                 870                 875                 880
   Cys Thr Ser Lys Lys Asn Ser Lys Lys Ser Val Val Ile Ile Gly Met
                   885                 890                 895
   Arg Ala Ala Gly Lys Thr Thr Ile Ser Lys Trp Cys Ala Ser Ala Leu
               900                 905                 910
   Gly Tyr Lys Leu Val Asp Leu Asp Glu Leu Phe Glu Gln Gln His Asn
           915                 920                 925
   Asn Gln Ser Val Lys Gln Phe Val Val Glu Asn Gly Trp Glu Lys Phe
       930                 935                 940
   Arg Glu Glu Glu Thr Arg Ile Phe Lys Glu Val Ile Gln Asn Tyr Gly
   945                 950                 955                 960
   Asp Asp Gly Tyr Val Phe Ser Thr Gly Gly Gly Ile Val Glu Ser Ala
                   965                 970                 975
   Glu Ser Arg Lys Ala Leu Lys Asp Phe Ala Ser Ser Gly Gly Tyr Val
               980                 985                 990
   Leu His Leu His Arg Asp Ile Glu Glu Thr Ile Val Phe Leu Gln Ser
           995                 1000                1005
   Asp Pro Ser Arg Pro Ala Tyr Val Glu Glu Ile Arg Glu Val Trp Asn
      1010                1015                1020
   Arg Arg Glu Gly Trp Tyr Lys Glu Cys Ser Asn Phe Ser Phe Phe Ala
   1025                1030                1035                1040
   Pro His Cys Ser Ala Glu Ala Glu Phe Gln Ala Leu Arg Arg Ser Phe
                  1045                1050                1055
   Ser Lys Tyr Ile Ala Thr Ile Thr Gly Val Arg Glu Ile Glu Ile Pro
              1060                1065                1070
   Ser Gly Arg Ser Ala Phe Val Cys Leu Thr Phe Asp Asp Leu Thr Glu
          1075                1080                1085
   Gln Thr Glu Asn Leu Thr Pro Ile Cys Tyr Gly Cys Glu Ala Val Glu
      1090                1095                1100
   Val Arg Val Asp His Leu Ala Asn Tyr Ser Ala Asp Phe Val Ser Lys
   1105                1110                1115                1120
   Gln Leu Ser Ile Leu Arg Lys Ala Thr Asp Ser Ile Pro Ile Ile Phe
                  1125                1130                1135
   Thr Val Arg Thr Met Lys Gln Gly Gly Asn Phe Pro Asp Glu Glu Phe
              1140                1145                1150
   Lys Thr Leu Arg Glu Leu Tyr Asp Ile Ala Leu Lys Asn Gly Val Glu
          1155                1160                1165
   Phe Leu Asp Leu Glu Leu Thr Leu Pro Thr Asp Ile Gln Tyr Glu Val
      1170                1175                1180
   Ile Asn Lys Arg Gly Asn Thr Lys Ile Ile Gly Ser His His Asp Phe
   1185                1190                1195                1200
   Gln Gly Leu Tyr Ser Trp Asp Asp Ala Glu Trp Glu Asn Arg Phe Asn
                  1205                1210                1215
   Gln Ala Leu Thr Leu Asp Val Asp Val Val Lys Phe Val Gly Thr Ala
   1220 1225 1230
   Val Asn Phe Glu Asp Asn Leu Arg Leu Glu His Phe Arg Asp Thr His
   1235 1240 1245
   Lys Asn Lys Pro Leu Ile Ala Val Asn Met Thr Ser Lys Gly Ser Ile
   1250 1255 1260
   Ser Arg Val Leu Asn Asn Val Leu Thr Pro Val Thr Ser Asp Leu Leu
   1265 1270 1275 1280
   Pro Asn Ser Ala Ala Pro Gly Gln Leu Thr Val Ala Gln Ile Asn Lys
   1285 1290 1295
   Met Tyr Thr Ser Met Gly Gly Ile Glu Pro Lys Glu Leu Phe Val Val
   1300 1305 1310
   Gly Lys Pro Ile Gly His Ser Arg Ser Pro Ile Leu His Asn Thr Gly
   1315 1320 1325
   Tyr Glu Ile Leu Gly Leu Pro His Lys Phe Asp Lys Phe Glu Thr Glu
   1330 1335 1340
   Ser Ala Gln Leu Val Lys Glu Lys Leu Leu Asp Gly Asn Lys Asn Phe
   1345 1350 1355 1360
   Gly Gly Ala Ala Val Thr Ile Pro Leu Lys Leu Asp Ile Met Gln Tyr
   1365 1370 1375
   Met Asp Glu Leu Thr Asp Ala Ala Lys Val Ile Gly Ala Val Asn Thr
   1380 1385 1390
   Val Ile Pro Leu Gly Asn Lys Lys Phe Lys Gly Asp Asn Thr Asp Trp
   1395 1400 1405
   Leu Gly Ile Arg Asn Ala Leu Ile Asn Asn Gly Val Pro Glu Tyr Val
   1410 1415 1420
   Gly His Thr Ala Gly Leu Val Ile Gly Ala Gly Gly Thr Ser Arg Ala
   1425 1430 1435 1440
   Ala Leu Tyr Ala Leu His Ser Leu Gly Cys Lys Lys Ile Phe Ile Ile
   1445 1450 1455
   Asn Arg Thr Thr Ser Lys Leu Lys Pro Leu Ile Glu Ser Leu Pro Ser
   1460 1465 1470
   Glu Phe Asn Ile Ile Gly Ile Glu Ser Thr Lys Ser Ile Glu Glu Ile
   1475 1480 1485
   Lys Glu His Val Gly Val Ala Val Ser Cys Val Pro Ala Asp Lys Pro
   1490 1495 1500
   Leu Asp Asp Glu Leu Leu Ser Lys Leu Glu Arg Phe Leu Val Lys Gly
   1505 1510 1515 1520
   Ala His Ala Ala Phe Val Pro Thr Leu Leu Glu Ala Ala Tyr Lys Pro
   1525 1530 1535
   Ser Val Thr Pro Val Met Thr Ile Ser Gln Asp Lys Tyr Gln Trp His
   1540 1545 1550
   Val Val Pro Gly Ser Gln Met Leu Val His Gln Gly Val Ala Gln Phe
   1555 1560 1565
   Glu Lys Trp Thr Gly Phe Lys Gly Pro Phe Lys Ala Ile Phe Asp Ala
   1570 1575 1580
   Val Thr Lys Glu
   1585
<210> 113
   <211> 820
   <212> PRT
   <213> Anabaena variabilis ATCC29413
<400> 113
   Met Ser Ile Val Gln Ala Lys Phe Glu Ala Lys Glu Thr Ser Phe His
   1               5                   10                  15
   Val Glu Gly Tyr Glu Lys Ile Glu Tyr Asp Leu Val Tyr Val Asp Gly
               20                  25                  30
   Ile Phe Glu Ile Gln Asn Ser Ala Leu Ala Asp Val Tyr Gln Gly Phe
           35                  40                  45
   Gly Arg Cys Leu Ala Ile Val Asp Ala Asn Val Ser Arg Leu Tyr Gly
       50                  55                  60
   Asn Gln Ile Gln Ala Tyr Phe Gln Tyr Tyr Gly Ile Glu Leu Arg Leu
   65                  70                  75                  80
   Phe Pro Ile Thr Ile Thr Glu Pro Asp Lys Thr Ile Gln Thr Phe Glu
                   85                  90                  95
   Arg Val Ile Asp Val Phe Ala Asp Phe Lys Leu Val Arg Lys Glu Pro
               100                 105                 110
   Val Leu Val Val Gly Gly Gly Leu Ile Thr Asp Val Val Gly Phe Ala
           115                 120                 125
   Cys Ser Thr Tyr Arg Arg Ser Ser Asn Tyr Ile Arg Ile Pro Thr Thr
       130                 135                 140
   Leu Ile Gly Leu Ile Asp Ala Ser Val Ala Ile Lys Val Ala Val Asn
   145                 150                 155                 160
   His Arg Lys Leu Lys Asn Arg Leu Gly Ala Tyr His Ala Ser Arg Lys
                   165                 170                 175
   Val Phe Leu Asp Phe Ser Leu Leu Arg Thr Leu Pro Thr Asp Gln Val
               180                 185                 190
   Arg Asn Gly Met Ala Glu Leu Val Lys Ile Ala Val Val Ala His Gln
           195                 200                 205
   Glu Val Phe Glu Leu Leu Glu Lys Tyr Gly Glu Glu Leu Leu Arg Thr
       210                 215                 220
   His Phe Gly Asn Ile Asp Ala Thr Pro Glu Ile Lys Glu Ile Ala His
   225                 230                 235                 240
   Arg Leu Thr Tyr Lys Ala Ile His Lys Met Leu Glu Leu Glu Val Pro
                   245                 250                 255
   Asn Leu His Glu Leu Asp Leu Asp Arg Val Ile Ala Tyr Gly His Thr
               260                 265                 270
   Trp Ser Pro Thr Leu Glu Leu Ala Pro Arg Leu Pro Met Phe His Gly
           275                 280                 285
   His Ala Val Asn Val Asp Met Ala Phe Ser Ala Thr Ile Ala Ala Arg
       290                 295                 300
   Arg Gly Tyr Ile Thr Ile Ala Glu Arg Asp Arg Ile Leu Gly Leu Met
   305                 310                 315                 320
   Ser Arg Val Gly Leu Ser Leu Asp His Pro Met Leu Asp Ile Asp Ile
                   325                 330                 335
   Leu Trp Arg Gly Thr Glu Ser Ile Thr Leu Thr Arg Asp Gly Leu Leu
               340                 345                 350
   Arg Ala Ala Met Pro Lys Pro Ile Gly Asp Cys Val Phe Val Asn Asp
           355                 360                 365
   Leu Thr Arg Glu Glu Leu Ala Ala Ala Leu Ala Asp His Lys Glu Leu
       370                 375                 380
   Cys Thr Ser Tyr Pro Arg Gly Gly Glu Gly Val Asp Val Tyr Pro Val
   385                 390                 395                 400
   Tyr Gln Lys Glu Leu Ile Gly Ser Val Lys Met Ser Ile Val Gln Ala
                   405                 410                 415
   Lys Phe Glu Ala Lys Glu Thr Ser Phe His Val Glu Gly Tyr Glu Lys
               420                 425                 430
   Ile Glu Tyr Asp Leu Val Tyr Val Asp Gly Ile Phe Glu Ile Gln Asn
           435                 440                 445
   Ser Ala Leu Ala Asp Val Tyr Gln Gly Phe Gly Arg Cys Leu Ala Ile
       450                 455                 460
   Val Asp Ala Asn Val Ser Arg Leu Tyr Gly Asn Gln Ile Gln Ala Tyr
   465                 470                 475                 480
   Phe Gln Tyr Tyr Gly Ile Glu Leu Arg Leu Phe Pro Ile Thr Ile Thr
                   485                 490                 495
   Glu Pro Asp Lys Thr Ile Gln Thr Phe Glu Arg Val Ile Asp Val Phe
               500                 505                 510
   Ala Asp Phe Lys Leu Val Arg Lys Glu Pro Val Leu Val Val Gly Gly
           515                 520                 525
   Gly Leu Ile Thr Asp Val Val Gly Phe Ala Cys Ser Thr Tyr Arg Arg
       530                 535                 540
   Ser Ser Asn Tyr Ile Arg Ile Pro Thr Thr Leu Ile Gly Leu Ile Asp
   545                 550                 555                 560
   Ala Ser Val Ala Ile Lys Val Ala Val Asn His Arg Lys Leu Lys Asn
                   565                 570                 575
   Arg Leu Gly Ala Tyr His Ala Ser Arg Lys Val Phe Leu Asp Phe Ser
   580 585 590
   Leu Leu Arg Thr Leu Pro Thr Asp Gln Val Arg Asn Gly Met Ala Glu
   595 600 605
   Leu Val Lys Ile Ala Val Val Ala His Gln Glu Val Phe Glu Leu Leu
   610 615 620
   Glu Lys Tyr Gly Glu Glu Leu Leu Arg Thr His Phe Gly Asn Ile Asp
   625 630 635 640
   Ala Thr Pro Glu Ile Lys Glu Ile Ala His Arg Leu Thr Tyr Lys Ala
   645 650 655
   Ile His Lys Met Leu Glu Leu Glu Val Pro Asn Leu His Glu Leu Asp
   660 665 670
   Leu Asp Arg Val Ile Ala Tyr Gly His Thr Trp Ser Pro Thr Leu Glu
   675 680 685
   Leu Ala Pro Arg Leu Pro Met Phe His Gly His Ala Val Asn Val Asp
   690 695 700
   Met Ala Phe Ser Ala Thr Ile Ala Ala Arg Arg Gly Tyr Ile Thr Ile
   705 710 715 720
   Ala Glu Arg Asp Arg Ile Leu Gly Leu Met Ser Arg Val Gly Leu Ser
   725 730 735
   Leu Asp His Pro Met Leu Asp Ile Asp Ile Leu Trp Arg Gly Thr Glu
   740 745 750
   Ser Ile Thr Leu Thr Arg Asp Gly Leu Leu Arg Ala Ala Met Pro Lys
   755 760 765
   Pro Ile Gly Asp Cys Val Phe Val Asn Asp Leu Thr Arg Glu Glu Leu
   770 775 780
   Ala Ala Ala Leu Ala Asp His Lys Glu Leu Cys Thr Ser Tyr Pro Arg
   785 790 795 800
   Gly Gly Glu Gly Val Asp Val Tyr Pro Val Tyr Gln Lys Glu Leu Ile
   805 810 815
   Gly Ser Val Lys
   820
<210> 114
   <211> 1099
   <212> PRT
   <213> Anabaena variabilis ATCC29413
<400> 114
   Met Ser Ile Val Gln Ala Lys Phe Glu Ala Lys Glu Thr Ser Phe His
   1               5                   10                  15
   Val Glu Gly Tyr Glu Lys Ile Glu Tyr Asp Leu Val Tyr Val Asp Gly
               20                  25                  30
   Ile Phe Glu Ile Gln Asn Ser Ala Leu Ala Asp Val Tyr Gln Gly Phe
           35                  40                  45
   Gly Arg Cys Leu Ala Ile Val Asp Ala Asn Val Ser Arg Leu Tyr Gly
        50                  55                  60
   Asn Gln Ile Gln Ala Tyr Phe Gln Tyr Tyr Gly Ile Glu Leu Arg Leu
    65                  70                  75                  80
   Phe Pro Ile Thr Ile Thr Glu Pro Asp Lys Thr Ile Gln Thr Phe Glu
                    85                  90                  95
   Arg Val Ile Asp Val Phe Ala Asp Phe Lys Leu Val Arg Lys Glu Pro
               100                 105                 110
   Val Leu Val Val Gly Gly Gly Leu Ile Thr Asp Val Val Gly Phe Ala
           115                 120                 125
   Cys Ser Thr Tyr Arg Arg Ser Ser Asn Tyr Ile Arg Ile Pro Thr Thr
       130                 135                 140
   Leu Ile Gly Leu Ile Asp Ala Ser Val Ala Ile Lys Val Ala Val Asn
   145                 150                 155                 160
   His Arg Lys Leu Lys Asn Arg Leu Gly Ala Tyr His Ala Ser Arg Lys
                   165                 170                 175
   Val Phe Leu Asp Phe Ser Leu Leu Arg Thr Leu Pro Thr Asp Gln Val
               180                 185                 190
   Arg Asn Gly Met Ala Glu Leu Val Lys Ile Ala Val Val Ala His Gln
           195                 200                 205
   Glu Val Phe Glu Leu Leu Glu Lys Tyr Gly Glu Glu Leu Leu Arg Thr
       210                 215                 220
   His Phe Gly Asn Ile Asp Ala Thr Pro Glu Ile Lys Glu Ile Ala His
   225                 230                 235                 240
   Arg Leu Thr Tyr Lys Ala Ile His Lys Met Leu Glu Leu Glu Val Pro
                   245                 250                 255
   Asn Leu His Glu Leu Asp Leu Asp Arg Val Ile Ala Tyr Gly His Thr
               260                 265                 270
   Trp Ser Pro Thr Leu Glu Leu Ala Pro Arg Leu Pro Met Phe His Gly
           275                 280                 285
   His Ala Val Asn Val Asp Met Ala Phe Ser Ala Thr Ile Ala Ala Arg
       290                 295                 300
   Arg Gly Tyr Ile Thr Ile Ala Glu Arg Asp Arg Ile Leu Gly Leu Met
   305                 310                 315                 320
   Ser Arg Val Gly Leu Ser Leu Asp His Pro Met Leu Asp Ile Asp Ile
                   325                 330                 335
   Leu Trp Arg Gly Thr Glu Ser Ile Thr Leu Thr Arg Asp Gly Leu Leu
               340                 345                 350
   Arg Ala Ala Met Pro Lys Pro Ile Gly Asp Cys Val Phe Val Asn Asp
           355                 360                 365
   Leu Thr Arg Glu Glu Leu Ala Ala Ala Leu Ala Asp His Lys Glu Leu
       370                 375                 380
   Cys Thr Ser Tyr Pro Arg Gly Gly Glu Gly Val Asp Val Tyr Pro Val
   385                 390                 395                 400
   Tyr Gln Lys Glu Leu Ile Gly Ser Val Lys Met Ser Ile Val Gln Ala
                   405                 410                 415
   Lys Phe Glu Ala Lys Glu Thr Ser Phe His Val Glu Gly Tyr Glu Lys
               420                 425                 430
   Ile Glu Tyr Asp Leu Val Tyr Val Asp Gly Ile Phe Glu Ile Gln Asn
           435                 440                 445
   Ser Ala Leu Ala Asp Val Tyr Gln Gly Phe Gly Arg Cys Leu Ala Ile
       450                 455                 460
   Val Asp Ala Asn Val Ser Arg Leu Tyr Gly Asn Gln Ile Gln Ala Tyr
   465                 470                 475                 480
   Phe Gln Tyr Tyr Gly Ile Glu Leu Arg Leu Phe Pro Ile Thr Ile Thr
                   485                 490                 495
   Glu Pro Asp Lys Thr Ile Gln Thr Phe Glu Arg Val Ile Asp Val Phe
               500                 505                 510
   Ala Asp Phe Lys Leu Val Arg Lys Glu Pro Val Leu Val Val Gly Gly
           515                 520                 525
   Gly Leu Ile Thr Asp Val Val Gly Phe Ala Cys Ser Thr Tyr Arg Arg
       530                 535                 540
   Ser Ser Asn Tyr Ile Arg Ile Pro Thr Thr Leu Ile Gly Leu Ile Asp
   545                 550                 555                 560
   Ala Ser Val Ala Ile Lys Val Ala Val Asn His Arg Lys Leu Lys Asn
                   565                 570                 575
   Arg Leu Gly Ala Tyr His Ala Ser Arg Lys Val Phe Leu Asp Phe Ser
               580                 585                 590
   Leu Leu Arg Thr Leu Pro Thr Asp Gln Val Arg Asn Gly Met Ala Glu
           595                 600                 605
   Leu Val Lys Ile Ala Val Val Ala His Gln Glu Val Phe Glu Leu Leu
       610                 615                 620
   Glu Lys Tyr Gly Glu Glu Leu Leu Arg Thr His Phe Gly Asn Ile Asp
   625                 630                 635                 640
   Ala Thr Pro Glu Ile Lys Glu Ile Ala His Arg Leu Thr Tyr Lys Ala
                   645                 650                 655
   Ile His Lys Met Leu Glu Leu Glu Val Pro Asn Leu His Glu Leu Asp
               660                 665                 670
   Leu Asp Arg Val Ile Ala Tyr Gly His Thr Trp Ser Pro Thr Leu Glu
           675                 680                 685
   Leu Ala Pro Arg Leu Pro Met Phe His Gly His Ala Val Asn Val Asp
       690                 695                 700
   Met Ala Phe Ser Ala Thr Ile Ala Ala Arg Arg Gly Tyr Ile Thr Ile
   705                 710                 715                 720
   Ala Glu Arg Asp Arg Ile Leu Gly Leu Met Ser Arg Val Gly Leu Ser
   725 730 735
   Leu Asp His Pro Met Leu Asp Ile Asp Ile Leu Trp Arg Gly Thr Glu
   740 745 750
   Ser Ile Thr Leu Thr Arg Asp Gly Leu Leu Arg Ala Ala Met Pro Lys
   755 760 765
   Pro Ile Gly Asp Cys Val Phe Val Asn Asp Leu Thr Arg Glu Glu Leu
   770 775 780
   Ala Ala Ala Leu Ala Asp His Lys Glu Leu Cys Thr Ser Tyr Pro Arg
   785 790 795 800
   Gly Gly Glu Gly Val Asp Val Tyr Pro Val Tyr Gln Lys Glu Leu Ile
   805 810 815
   Gly Ser Val Lys Met Thr Asn Val Ile Val Gln Pro Thr Ala Arg Pro
   820 825 830
   Val Thr Pro Leu Gly Ile Leu Thr Lys Gln Leu Glu Ala Ile Val Gln
   835 840 845
   Glu Val Lys Gln His Pro Asp Leu Pro Gly Glu Leu Ile Ala Asn Ile
   850 855 860
   His Gln Ala Trp Arg Leu Ala Ala Gly Ile Asp Pro Tyr Leu Glu Glu
   865 870 875 880
   Cys Thr Thr Pro Glu Ser Pro Glu Leu Ala Ala Leu Ala Lys Thr Thr
   885 890 895
   Ala Thr Glu Ala Trp Gly Glu His Phe His Gly Gly Thr Thr Val Arg
   900 905 910
   Pro Leu Glu Gln Glu Met Leu Ser Gly His Ile Glu Gly Gln Thr Leu
   915 920 925
   Lys Met Phe Val His Met Thr Lys Ala Lys Lys Val Leu Glu Ile Gly
   930 935 940
   Met Phe Thr Gly Tyr Ser Ala Leu Ala Met Ala Glu Ala Leu Pro Glu
   945 950 955 960
   Asp Gly Leu Leu Val Ala Cys Glu Val Asp Pro Tyr Ala Ala Glu Ile
   965 970 975
   Gly Gln Lys Ala Phe Gln Gln Ser Pro His Gly Gly Lys Ile Arg Val
   980 985 990
   Glu Leu Asp Ala Ala Leu Ala Thr Leu Asp Lys Leu Ala Glu Ala Gly
   995 1000 1005
   Glu Ser Phe Asp Leu Val Phe Ile Asp Ala Asp Lys Lys Glu Tyr Val
   1010 1015 1020
   Ala Tyr Phe His Lys Leu Leu Gly Ser Ser Leu Leu Ala Pro Asp Gly
   1025 1030 1035 1040
   Phe Ile Cys Val Asp Asn Thr Leu Leu Gln Gly Glu Val Tyr Leu Pro
   1045 1050 1055
   Ala Glu Glu Arg Ser Val Asn Gly Glu Ala Ile Ala Gln Phe Asn His
               1060                1065                1070
   Thr Val Ala Ile Asp Pro Arg Val Glu Gln Val Leu Leu Pro Leu Arg
           1075                1080                1085
   Asp Gly Leu Thr Ile Ile Arg Arg Ile Gln Pro
       1090                1095
<210> 115
   <211> 410
   <212> PRT
   <213> Anabaena variabilis ATCC29413
<400> 115
   Met Ser Ile Val Gln Ala Lys Phe Glu Ala Lys Glu Thr Ser Phe His
   1               5                   10                  15
   Val Glu Gly Tyr Glu Lys Ile Glu Tyr Asp Leu Val Tyr Val Asp Gly
               20                  25                  30
   Ile Phe Glu Ile Gln Asn Ser Ala Leu Ala Asp Val Tyr Gln Gly Phe
           35                  40                  45
   Gly Arg Cys Leu Ala Ile Val Asp Ala Asn Val Ser Arg Leu Tyr Gly
       50                  55                  60
   Asn Gln Ile Gln Ala Tyr Phe Gln Tyr Tyr Gly Ile Glu Leu Arg Leu
   65                  70                  75                  80
   Phe Pro Ile Thr Ile Thr Glu Pro Asp Lys Thr Ile Gln Thr Phe Glu
                   85                  90                  95
   Arg Val Ile Asp Val Phe Ala Asp Phe Lys Leu Val Arg Lys Glu Pro
               100                 105                 110
   Val Leu Val Val Gly Gly Gly Leu Ile Thr Asp Val Val Gly Phe Ala
           115                 120                 125
   Cys Ser Thr Tyr Arg Arg Ser Ser Asn Tyr Ile Arg Ile Pro Thr Thr
       130                 135                 140
   Leu Ile Gly Leu Ile Asp Ala Ser Val Ala Ile Lys Val Ala Val Asn
   145                 150                 155                 160
   His Arg Lys Leu Lys Asn Arg Leu Gly Ala Tyr His Ala Ser Arg Lys
                   165                 170                 175
   Val Phe Leu Asp Phe Ser Leu Leu Arg Thr Leu Pro Thr Asp Gln Val
               180                 185                 190
   Arg Asn Gly Met Ala Glu Leu Val Lys Ile Ala Val Val Ala His Gln
           195                 200                 205
   Glu Val Phe Glu Leu Leu Glu Lys Tyr Gly Glu Glu Leu Leu Arg Thr
       210                 215                 220
   His Phe Gly Asn Ile Asp Ala Thr Pro Glu Ile Lys Glu Ile Ala His
   225                 230                 235                 240
   Arg Leu Thr Tyr Lys Ala Ile His Lys Met Leu Glu Leu Glu Val Pro
                   245                 250                 255
   Asn Leu His Glu Leu Asp Leu Asp Arg Val Ile Ala Tyr Gly His Thr
               260                 265                 270
   Trp Ser Pro Thr Leu Glu Leu Ala Pro Arg Leu Pro Met Phe His Gly
           275                 280                 285
   His Ala Val Asn Val Asp Met Ala Phe Ser Ala Thr Ile Ala Ala Arg
       290                 295                 300
   Arg Gly Tyr Ile Thr Ile Ala Glu Arg Asp Arg Ile Leu Gly Leu Met
   305                 310                 315                 320
   Ser Arg Val Gly Leu Ser Leu Asp His Pro Met Leu Asp Ile Asp Ile
                   325                 330                 335
   Leu Trp Arg Gly Thr Glu Ser Ile Thr Leu Thr Arg Asp Gly Leu Leu
               340                 345                 350
   Arg Ala Ala Met Pro Lys Pro Ile Gly Asp Cys Val Phe Val Asn Asp
           355                 360                 365
   Leu Thr Arg Glu Glu Leu Ala Ala Ala Leu Ala Asp His Lys Glu Leu
       370                 375                 380
   Cys Thr Ser Tyr Pro Arg Gly Gly Glu Gly Val Asp Val Tyr Pro Val
   385                 390                 395                 400
   Tyr Gln Lys Glu Leu Ile Gly Ser Val Lys
                   405                 410
<210> 116
   <211> 410
   <212> PRT
   <213> Anabaena variabilis ATCC29413
<400> 116
   Met Ser Ile Val Gln Ala Lys Phe Glu Ala Lys Glu Thr Ser Phe His
   1               5                   10                  15
   Val Glu Gly Tyr Glu Lys Ile Glu Tyr Asp Leu Val Tyr Val Asp Gly
               20                  25                  30
   Ile Phe Glu Ile Gln Asn Ser Ala Leu Ala Asp Val Tyr Gln Gly Phe
           35                  40                  45
   Gly Arg Cys Leu Ala Ile Val Asp Ala Asn Val Ser Arg Leu Tyr Gly
       50                  55                  60
   Asn Gln Ile Gln Ala Tyr Phe Gln Tyr Tyr Gly Ile Glu Leu Arg Leu
   65                  70                  75                  80
   Phe Pro Ile Thr Ile Thr Glu Pro Asp Lys Thr Ile Gln Thr Phe Glu
                   85                  90                  95
   Arg Val Ile Asp Val Phe Ala Asp Phe Lys Leu Val Arg Lys Glu Pro
               100                 105                 110
   Val Leu Val Val Gly Gly Gly Leu Ile Thr Asp Val Val Gly Phe Ala
           115                 120                 125
   Cys Ser Thr Tyr Arg Arg Ser Ser Asn Tyr Ile Arg Ile Pro Thr Thr
   130 135 140
   Leu Ile Gly Leu Ile Asp Ala Ser Val Ala Ile Lys Val Ala Val Asn
   145 150 155 160
   His Arg Lys Leu Lys Asn Arg Leu Gly Ala Tyr His Ala Ser Arg Lys
   165 170 175
   Val Phe Leu Asp Phe Ser Leu Leu Arg Thr Leu Pro Thr Asp Gln Val
   180 185 190
   Arg Asn Gly Met Ala Glu Leu Val Lys Ile Ala Val Val Ala His Gln
   195 200 205
   Glu Val Phe Glu Leu Leu Glu Lys Tyr Gly Glu Glu Leu Leu Arg Thr
   210 215 220
   His Phe Gly Asn Ile Asp Ala Thr Pro Glu Ile Lys Glu Ile Ala His
   225 230 235 240
   Arg Leu Thr Tyr Lys Ala Ile His Lys Met Leu Glu Leu Glu Val Pro
   245 250 255
   Asn Leu His Glu Leu Asp Leu Asp Arg Val Ile Ala Tyr Gly His Thr
   260 265 270
   Trp Ser Pro Thr Leu Glu Leu Ala Pro Arg Leu Pro Met Phe His Gly
   275 280 285
   His Ala Val Asn Val Asp Met Ala Phe Ser Ala Thr Ile Ala Ala Arg
   290 295 300
   Arg Gly Tyr Ile Thr Ile Ala Glu Arg Asp Arg Ile Leu Gly Leu Met
   305 310 315 320
   Ser Arg Val Gly Leu Ser Leu Asp His Pro Met Leu Asp Ile Asp Ile
   325 330 335
   Leu Trp Arg Gly Thr Glu Ser Ile Thr Leu Thr Arg Asp Gly Leu Leu
   340 345 350
   Arg Ala Ala Met Pro Lys Pro Ile Gly Asp Cys Val Phe Val Asn Asp
   355 360 365
   Leu Thr Arg Glu Glu Leu Ala Ala Ala Leu Ala Asp His Lys Glu Leu
   370 375 380
   Cys Thr Ser Tyr Pro Arg Gly Gly Glu Gly Val Asp Val Tyr Pro Val
   385 390 395 400
   Tyr Gln Lys Glu Leu Ile Gly Ser Val Lys
   405 410
<210> 117
   <211> 279
   <212> PRT
   <213> Anabaena variabilis ATCC29413
<400> 117
   Met Thr Asn Val Ile Val Gln Pro Thr Ala Arg Pro Val Thr Pro Leu
   1               5                   10                  15
   Gly Ile Leu Thr Lys Gln Leu Glu Ala Ile Val Gln Glu Val Lys Gln
               20                  25                  30
   His Pro Asp Leu Pro Gly Glu Leu Ile Ala Asn Ile His Gln Ala Trp
           35                  40                  45
   Arg Leu Ala Ala Gly Ile Asp Pro Tyr Leu Glu Glu Cys Thr Thr Pro
       50                  55                  60
   Glu Ser Pro Glu Leu Ala Ala Leu Ala Lys Thr Thr Ala Thr Glu Ala
   65                  70                  75                  80
   Trp Gly Glu His Phe His Gly Gly Thr Thr Val Arg Pro Leu Glu Gln
                   85                  90                  95
   Glu Met Leu Ser Gly His Ile Glu Gly Gln Thr Leu Lys Met Phe Val
               100                 105                 110
   His Met Thr Lys Ala Lys Lys Val Leu Glu Ile Gly Met Phe Thr Gly
           115                 120                 125
   Tyr Ser Ala Leu Ala Met Ala Glu Ala Leu Pro Glu Asp Gly Leu Leu
       130                 135                 140
   Val Ala Cys Glu Val Asp Pro Tyr Ala Ala Glu Ile Gly Gln Lys Ala
   145                 150                 155                 160
   Phe Gln Gln Ser Pro His Gly Gly Lys Ile Arg Val Glu Leu Asp Ala
                   165                 170                 175
   Ala Leu Ala Thr Leu Asp Lys Leu Ala Glu Ala Gly Glu Ser Phe Asp
               180                 185                 190
   Leu Val Phe Ile Asp Ala Asp Lys Lys Glu Tyr Val Ala Tyr Phe His
           195                 200                 205
   Lys Leu Leu Gly Ser Ser Leu Leu Ala Pro Asp Gly Phe Ile Cys Val
       210                 215                 220
   Asp Asn Thr Leu Leu Gln Gly Glu Val Tyr Leu Pro Ala Glu Glu Arg
   225                 230                 235                 240
   Ser Val Asn Gly Glu Ala Ile Ala Gln Phe Asn His Thr Val Ala Ile
                   245                 250                 255
   Asp Pro Arg Val Glu Gln Val Leu Leu Pro Leu Arg Asp Gly Leu Thr
               260                 265                 270
   Ile Ile Arg Arg Ile Gln Pro
           275
<210> 118
   <211> 888
   <212> PRT
   <213> Anabaena variabilis ATCC29413
<400> 118
   Met Gln Thr Ile Asp Phe Asn Ile Arg Lys Leu Leu Val Glu Trp Asn
   1               5                   10                  15
   Ala Thr His Arg Asp Tyr Asp Leu Ser Gln Ser Leu His Glu Leu Ile
               20                  25                  30
   Val Ala Gln Val Glu Arg Thr Pro Glu Ala Ile Ala Val Thr Phe Asp
   35 40 45
   Lys Gln Gln Leu Thr Tyr Gln Glu Leu Asn His Lys Ala Asn Gln Leu
   50 55 60
   Gly His Tyr Leu Gln Thr Leu Gly Val Gln Pro Glu Thr Leu Val Gly
   65 70 75 80
   Val Cys Leu Glu Arg Ser Leu Glu Met Val Ile Cys Leu Leu Gly Ile
   85 90 95
   Leu Lys Ala Gly Gly Ala Tyr Val Pro Ile Asp Pro Glu Tyr Pro Gln
   100 105 110
   Glu Arg Ile Ala Tyr Met Leu Glu Asp Ser Gln Val Lys Val Leu Leu
   115 120 125
   Thr Gln Glu Lys Leu Leu Asn Gln Ile Pro His His Gln Ala Gln Thr
   130 135 140
   Ile Cys Val Asp Arg Glu Trp Glu Lys Ile Ser Thr Gln Ala Asn Thr
   145 150 155 160
   Asn Pro Lys Ser Asn Ile Lys Thr Asp Asn Leu Ala Tyr Val Ile Tyr
   165 170 175
   Thr Ser Gly Ser Thr Gly Lys Pro Lys Gly Ala Met Asn Thr His Lys
   180 185 190
   Gly Ile Cys Asn Arg Leu Leu Trp Met Gln Glu Ala Tyr Gln Ile Asp
   195 200 205
   Ser Thr Asp Ser Ile Leu Gln Lys Thr Pro Phe Ser Phe Asp Val Ser
   210 215 220
   Val Trp Glu Phe Phe Trp Thr Leu Leu Thr Gly Ala Arg Leu Val Ile
   225 230 235 240
   Ala Lys Pro Gly Gly His Lys Asp Ser Ala Tyr Leu Ile Asp Leu Ile
   245 250 255
   Thr Gln Glu Gln Ile Thr Thr Leu His Phe Val Pro Ser Met Leu Gln
   260 265 270
   Val Phe Leu Gln Asn Arg His Val Ser Lys Cys Ser Ser Leu Lys Arg
   275 280 285
   Val Ile Cys Ser Gly Glu Ala Leu Ser Ile Asp Leu Gln Asn Arg Phe
   290 295 300
   Phe Gln His Leu Gln Cys Glu Leu His Asn Leu Tyr Gly Pro Thr Glu
   305 310 315 320
   Ala Ala Ile Asp Val Thr Phe Trp Gln Cys Arg Lys Asp Ser Asn Leu
   325 330 335
   Lys Ser Val Pro Ile Gly Arg Pro Ile Ala Asn Thr Gln Ile Tyr Ile
   340 345 350
   Leu Asp Ala Asp Leu Gln Pro Val Asn Ile Gly Val Thr Gly Glu Ile
   355 360 365
   Tyr Ile Gly Gly Val Gly Val Ala Arg Gly Tyr Leu Asn Lys Glu Glu
   370 375 380
   Leu Thr Lys Glu Lys Phe Ile Ile Asn Pro Phe Pro Asn Ser Glu Phe
   385 390 395 400
   Lys Arg Leu Tyr Lys Thr Gly Asp Leu Ala Arg Tyr Leu Pro Asp Gly
   405 410 415
   Asn Ile Glu Tyr Leu Gly Arg Thr Asp Tyr Gln Val Lys Ile Arg Gly
   420 425 430
   Tyr Arg Ile Glu Ile Gly Glu Ile Glu Asn Val Leu Ser Ser His Pro
   435 440 445
   Gln Val Arg Glu Ala Val Val Ile Ala Arg Asp Asp Asn Ala Gln Glu
   450 455 460
   Lys Gln Ile Ile Ala Tyr Ile Thr Tyr Asn Ser Ile Lys Pro Gln Leu
   465 470 475 480
   Asp Asn Leu Arg Asp Phe Leu Lys Ala Arg Leu Pro Asp Phe Met Ile
   485 490 495
   Pro Ala Ala Phe Val Met Leu Glu His Leu Pro Leu Thr Pro Ser Gly
   500 505 510
   Lys Val Asp Arg Lys Ala Leu Pro Lys Pro Asp Leu Phe Asn Tyr Ser
   515 520 525
   Glu His Asn Ser Tyr Val Ala Pro Arg Asn Glu Val Glu Glu Lys Leu
   530 535 540
   Val Gln Ile Trp Ser Asn Ile Leu His Leu Pro Lys Val Gly Val Thr
   545 550 555 560
   Glu Asn Phe Phe Ala Ile Gly Gly Asn Ser Leu Lys Ala Leu His Leu
   565 570 575
   Ile Ser Gln Ile Glu Glu Leu Phe Ala Lys Glu Ile Ser Leu Ala Thr
   580 585 590
   Leu Leu Thr Asn Pro Val Ile Ala Asp Leu Ala Lys Val Ile Gln Ala
   595 600 605
   Asn Asn Gln Ile His Asn Ser Pro Leu Val Pro Ile Gln Pro Gln Gly
   610 615 620
   Lys Gln Gln Pro Phe Phe Cys Ile His Pro Ala Gly Gly His Val Leu
   625 630 635 640
   Cys Tyr Phe Lys Leu Ala Gln Tyr Ile Gly Thr Asp Gln Pro Phe Tyr
   645 650 655
   Gly Leu Gln Ala Gln Gly Phe Tyr Gly Asp Glu Ala Pro Leu Thr Arg
   660 665 670
   Val Glu Asp Met Ala Ser Leu Tyr Val Lys Thr Ile Arg Glu Phe Gln
   675 680 685
   Pro Gln Gly Pro Tyr Arg Val Gly Gly Trp Ser Phe Gly Gly Val Val
   690 695 700
   Ala Tyr Glu Val Ala Gln Gln Leu His Arg Gln Gly Gln Glu Val Ser
   705                 710                 715                 720
   Leu Leu Ala Ile Leu Asp Ser Tyr Val Pro Ile Leu Leu Asp Lys Gln
                   725                 730                 735
   Lys Pro Ile Asp Asp Val Tyr Leu Val Gly Val Leu Ser Arg Val Phe
               740                 745                 750
   Gly Gly Met Phe Gly Gln Asp Asn Leu Val Thr Pro Glu Glu Ile Glu
           755                 760                 765
   Asn Leu Thr Val Glu Glu Lys Ile Asn Tyr Ile Ile Asp Lys Ala Arg
       770                 775                 780
   Ser Ala Arg Ile Phe Pro Pro Gly Val Glu Arg Gln Asn Asn Arg Arg
   785                 790                 795                 800
   Ile Leu Asp Val Leu Val Gly Thr Leu Lys Ala Thr Tyr Ser Tyr Ile
                   805                 810                 815
   Arg Gln Pro Tyr Pro Gly Lys Val Thr Val Phe Arg Ala Arg Glu Lys
               820                 825                 830
   His Ile Met Ala Pro Asp Pro Thr Leu Val Trp Val Glu Leu Phe Ser
           835                 840                 845
   Val Met Ala Ala Gln Glu Ile Lys Ile Ile Asp Val Pro Gly Asn His
       850                 855                 860
   Tyr Ser Phe Val Leu Glu Pro His Val Gln Val Leu Ala Gln Arg Leu
   865                 870                 875                 880
   Gln Asp Cys Leu Glu Asn Asn Ser
                   885
<210> 119
   <211> 410
   <212> PRT
   <213> Nostoc punctiforme ATCC 29133
<400> 119
   Met Ser Asn Val Gln Ala Ser Phe Glu Ala Thr Glu Ala Glu Phe Arg
   1               5                   10                  15
   Val Glu Gly Tyr Glu Lys Ile Glu Phe Ser Leu Val Tyr Val Asn Gly
               20                  25                  30
   Ala Phe Asp Ile Ser Asn Arg Glu Ile Ala Asp Ser Tyr Glu Lys Phe
           35                  40                  45
   Gly Arg Cys Leu Thr Val Ile Asp Ala Asn Val Asn Arg Leu Tyr Gly
       50                  55                  60
   Lys Gln Ile Lys Ser Tyr Phe Arg His Tyr Gly Ile Asp Leu Thr Val
   65                  70                  75                  80
   Val Pro Ile Val Ile Thr Glu Pro Thr Lys Thr Leu Ala Thr Phe Glu
                   85                  90                  95
   Lys Ile Val Asp Ala Phe Ser Asp Phe Gly Leu Ile Arg Lys Glu Pro
               100                 105                 110
   Val Leu Val Val Gly Gly Gly Leu Thr Thr Asp Val Ala Gly Phe Ala
           115                 120                 125
   Cys Ala Ala Tyr Arg Arg Lys Ser Asn Tyr Ile Arg Val Pro Thr Thr
       130                 135                 140
   Leu Ile Gly Leu Ile Asp Ala Gly Val Ala Ile Lys Val Ala Val Asn
   145                 150                 155                 160
   His Arg Lys Leu Lys Asn Arg Leu Gly Ala Tyr His Ala Pro Leu Lys
                   165                 170                 175
   Val Ile Leu Asp Phe Ser Phe Leu Gln Thr Leu Pro Thr Ala Gln Val
               180                 185                 190
   Arg Asn Gly Met Ala Glu Leu Val Lys Ile Ala Val Val Ala Asn Ser
           195                 200                 205
   Glu Val Phe Glu Leu Leu Tyr Glu Tyr Gly Glu Glu Leu Leu Ser Thr
       210                 215                 220
   His Phe Gly Tyr Val Asn Gly Thr Lys Glu Leu Lys Ala Ile Ala His
   225                 230                 235                 240
   Lys Leu Asn Tyr Glu Ala Ile Lys Thr Met Leu Glu Leu Glu Thr Pro
                   245                 250                 255
   Asn Leu His Glu Leu Asp Leu Asp Arg Val Ile Ala Tyr Gly His Thr
               260                 265                 270
   Trp Ser Pro Thr Leu Glu Leu Ala Pro Met Ile Pro Leu Phe His Gly
           275                 280                 285
   His Ala Val Asn Ile Asp Met Ala Leu Ser Ala Thr Ile Ala Ala Arg
       290                 295                 300
   Arg Gly Tyr Ile Thr Ser Gly Glu Arg Asp Arg Ile Leu Ser Leu Met
   305                 310                 315                 320
   Ser Arg Ile Gly Leu Ser Ile Asp His Pro Leu Leu Asp Gly Asp Leu
                   325                 330                 335
   Leu Trp Tyr Ala Thr Gln Ser Ile Ser Leu Thr Arg Asp Gly Lys Gln
               340                 345                 350
   Arg Ala Ala Met Pro Lys Pro Ile Gly Glu Cys Phe Phe Val Asn Asp
           355                 360                 365
   Phe Thr Arg Glu Glu Leu Asp Ala Ala Leu Ala Glu His Lys Arg Leu
       370                 375                 380
   Cys Ala Thr Tyr Pro Arg Gly Gly Asp Gly Ile Asp Ala Tyr Ile Glu
   385                 390                 395                 400
   Thr Gln Glu Glu Ser Lys Leu Leu Gly Val
                   405                 410
<210> 120
   <211> 277
   <212> PRT
   <213> Nostoc punctiforme ATCC 29133
<400> 120
   Met Thr Ser Ile Leu Gly Arg Asp Thr Ala Arg Pro Ile Thr Pro His
    1               5                  10                  15
   Ser Ile Leu Val Ala Gln Leu Gln Lys Thr Leu Arg Met Ala Glu Glu
               20                  25                  30
   Ser Asn Ile Pro Ser Glu Ile Leu Thr Ser Leu Arg Gln Gly Leu Gln
           35                  40                  45
   Leu Ala Ala Gly Leu Asp Pro Tyr Leu Asp Asp Cys Thr Thr Pro Glu
       50                  55                  60
   Ser Thr Ala Leu Thr Ala Leu Ala Gln Lys Thr Ser Ile Glu Asp Trp
   65                  70                  75                  80
   Ser Lys Arg Phe Ser Asp Gly Glu Thr Val Arg Gln Leu Glu Gln Glu
                   85                  90                  95
   Met Leu Ser Gly His Leu Glu Gly Gln Thr Leu Lys Met Phe Val His
               100                 105                 110
   Ile Thr Lys Ala Lys Ser Ile Leu Glu Val Gly Met Phe Thr Gly Tyr
           115                 120                 125
   Ser Ala Leu Ala Met Ala Glu Ala Leu Pro Asp Asp Gly Arg Leu Ile
       130                 135                 140
   Ala Cys Glu Val Asp Ser Tyr Val Ala Glu Phe Ala Gln Thr Cys Phe
   145                 150                 155                 160
   Gln Glu Ser Pro His Gly Arg Lys Ile Val Val Glu Val Ala Pro Ala
                   165                 170                 175
   Leu Glu Thr Leu His Lys Leu Val Ala Lys Lys Glu Ser Phe Asp Leu
               180                 185                 190
   Ile Phe Ile Asp Ala Asp Lys Lys Glu Tyr Ile Glu Tyr Phe Gln Ile
           195                 200                 205
   Ile Leu Asp Ser His Leu Leu Ala Pro Asp Gly Leu Ile Cys Val Asp
       210                 215                 220
   Asn Thr Leu Leu Gln Gly Gln Val Tyr Leu Pro Ser Glu Gln Arg Thr
   225                 230                 235                 240
   Ala Asn Gly Glu Ala Ile Ala Gln Phe Asn Arg Ile Val Ala Ala Asp
                   245                 250                 255
   Pro Arg Val Glu Gln Val Leu Leu Pro Ile Arg Asp Gly Ile Thr Leu
               260                 265                 270
   Ile Arg Arg Leu Val
           275
<210> 121
   <211> 461
   <212> PRT
   <213> Nostoc punctiforme ATCC 29133
<400> 121
   Met Ala Gln Ser Ile Ser Leu Ser Leu Pro Gln Ser Thr Thr Pro Ser
   1               5                   10                  15
   Lys Gly Val Arg Leu Lys Ile Ala Ala Leu Leu Lys Thr Ile Gly Thr
               20                  25                  30
   Leu Ile Leu Leu Leu Ile Ala Leu Pro Leu Asn Ala Leu Ile Val Leu
           35                  40                  45
   Ile Ser Leu Met Cys Arg Pro Phe Thr Lys Lys Pro Ala Val Ala Thr
       50                  55                  60
   His Pro Gln Asn Ile Leu Val Ser Gly Gly Lys Met Thr Lys Ala Leu
   65                  70                  75                  80
   Gln Leu Ala Arg Ser Phe His Ala Ala Gly His Arg Val Ile Leu Ile
                   85                  90                  95
   Glu Gly His Lys Tyr Trp Leu Ser Gly His Arg Phe Ser Asn Ser Val
               100                 105                 110
   Ser Arg Phe Tyr Thr Val Pro Ala Pro Gln Asp Asp Pro Glu Gly Tyr
           115                 120                 125
   Thr Gln Ala Leu Leu Glu Ile Val Lys Arg Glu Lys Ile Asp Val Tyr
       130                 135                 140
   Val Pro Val Cys Ser Pro Val Ala Ser Tyr Tyr Asp Ser Leu Ala Lys
   145                 150                 155                 160
   Ser Ala Leu Ser Glu Tyr Cys Glu Val Phe His Phe Asp Ala Asp Ile
                   165                 170                 175
   Thr Lys Met Leu Asp Asp Lys Phe Ala Phe Thr Asp Arg Ala Arg Ser
               180                 185                 190
   Leu Gly Leu Ser Ala Pro Lys Ser Phe Lys Ile Thr Asp Pro Glu Gln
           195                 200                 205
   Val Ile Asn Phe Asp Phe Ser Lys Glu Thr Arg Lys Tyr Ile Leu Lys
       210                 215                 220
   Ser Ile Ser Tyr Asp Ser Val Arg Arg Leu Asn Leu Thr Lys Leu Pro
   225                 230                 235                 240
   Cys Asp Thr Pro Glu Glu Thr Ala Ala Phe Val Lys Ser Leu Pro Ile
                   245                 250                 255
   Ser Pro Glu Lys Pro Trp Ile Met Gln Glu Phe Ile Pro Gly Lys Glu
               260                 265                 270
   Leu Cys Thr His Ser Thr Val Arg Asp Gly Glu Leu Arg Leu His Cys
           275                 280                 285
   Cys Ser Asn Ser Ser Ala Phe Gln Ile Asn Tyr Glu Asn Val Glu Asn
       290                 295                 300
   Pro Gln Ile Gln Glu Trp Val Gln His Phe Val Lys Ser Leu Arg Leu
   305                 310                 315                 320
   Thr Gly Gln Ile Ser Leu Asp Phe Ile Gln Ala Glu Asp Gly Thr Ala
                   325                 330                 335
   Tyr Ala Ile Glu Cys Asn Pro Arg Thr His Ser Ala Ile Thr Met Phe
               340                 345                 350
   Tyr Asn His Pro Gly Val Ala Glu Ala Tyr Leu Gly Lys Thr Pro Leu
           355                 360                 365
   Ala Ala Pro Leu Glu Pro Leu Ala Asp Ser Lys Pro Thr Tyr Trp Ile
       370                 375                 380
   Tyr His Glu Ile Trp Arg Leu Thr Gly Ile Arg Ser Gly Gln Gln Leu
   385                 390                 395                 400
   Gln Thr Trp Phe Gly Arg Leu Val Arg Gly Thr Asp Ala Ile Tyr Arg
                   405                 410                 415
   Leu Asp Asp Pro Ile Pro Phe Leu Thr Leu His His Trp Gln Ile Thr
               420                 425                 430
   Leu Leu Leu Leu Gln Asn Leu Gln Arg Leu Lys Gly Trp Val Lys Ile
           435                 440                 445
   Asp Phe Asn Ile Gly Lys Leu Val Glu Leu Gly Gly Asp
       450                 455                 460
<210> 122
   <211> 348
   <212> PRT
   <213> Nostoc punctiforme ATCC 29133
<400> 122
   Met Pro Val Leu Asn Ile Leu His Leu Val Gly Ser Ala His Asp Lys
   1               5                   10                  15
   Phe Tyr Cys Asp Leu Ser Arg Leu Tyr Ala Gln Asp Cys Leu Ala Ala
               20                  25                  30
   Thr Ala Asp Pro Ser Leu Tyr Asn Phe Gln Ile Ala Tyr Ile Thr Pro
           35                  40                  45
   Asp Arg Gln Trp Arg Phe Pro Asp Ser Leu Ser Arg Glu Asp Ile Ala
       50                  55                  60
   Leu Thr Lys Pro Ile Pro Val Phe Asp Ala Ile Gln Phe Leu Thr Gly
   65                  70                  75                  80
   Gln Asn Ile Asp Met Met Leu Pro Gln Met Phe Cys Ile Pro Gly Met
                   85                  90                  95
   Thr Gln Tyr Arg Ala Leu Phe Asp Leu Leu Lys Ile Pro Tyr Ile Gly
               100                 105                 110
   Asn Thr Pro Asp Ile Met Ala Ile Ala Ala His Lys Ala Arg Ala Lys
           115                 120                 125
   Ala Ile Val Glu Ala Ala Gly Val Lys Val Pro Arg Gly Glu Leu Leu
       130                 135                 140
   Arg Gln Gly Asp Ile Pro Thr Ile Thr Pro Pro Ala Val Val Lys Pro
   145                 150                 155                 160
   Val Ser Ser Asp Asn Ser Leu Gly Val Val Leu Val Lys Asp Val Thr
                       165                 170                 175
   Glu Tyr Asp Ala Ala Leu Lys Lys Ala Phe Glu Tyr Ala Ser Glu Val
               180                 185                 190
   Ile Val Glu Ala Phe Ile Glu Leu Gly Arg Glu Val Arg Cys Gly Ile
           195                 200                 205
   Ile Val Lys Asp Gly Glu Leu Ile Gly Leu Pro Leu Glu Glu Tyr Leu
       210                 215                 220
   Val Asp Pro His Asp Lys Pro Ile Arg Asn Tyr Ala Asp Lys Leu Gln
   225                 230                 235                 240
   Gln Thr Asp Asp Gly Asp Leu His Leu Thr Ala Lys Asp Asn Ile Lys
                   245                 250                 255
   Ala Trp Ile Leu Asp Pro Asn Asp Pro Ile Thr Gln Lys Val Gln Gln
               260                 265                 270
   Val Ala Lys Arg Cys His Gln Ala Leu Gly Cys Arg His Tyr Ser Leu
           275                 280                 285
   Phe Asp Phe Arg Ile Asp Pro Lys Gly Gln Pro Trp Phe Leu Glu Ala
       290                 295                 300
   Gly Leu Tyr Cys Ser Phe Ala Pro Lys Ser Val Ile Ser Ser Met Ala
   305                 310                 315                 320
   Lys Ala Ala Gly Ile Pro Leu Asn Asp Leu Leu Ile Thr Ala Ile Asn
                   325                 330                 335
   Glu Thr Leu Gly Ser Asn Lys Lys Val Leu Gln Asn
               340                 345
<210> 123
   <211> 680
   <212> PRT
   <213> Saccharomyces cerevisiae CEN.PK-1D
<400> 123
   Met Thr Gln Phe Thr Asp Ile Asp Lys Leu Ala Val Ser Thr Ile Arg
   1               5                   10                  15
   Ile Leu Ala Val Asp Thr Val Ser Lys Ala Asn Ser Gly His Pro Gly
               20                  25                  30
   Ala Pro Leu Gly Met Ala Pro Ala Ala His Val Leu Trp Ser Gln Met
           35                  40                  45
   Arg Met Asn Pro Thr Asn Pro Asp Trp Ile Asn Arg Asp Arg Phe Val
       50                  55                  60
   Leu Ser Asn Gly His Ala Val Ala Leu Leu Tyr Ser Met Leu His Leu
   65                  70                  75                  80
   Thr Gly Tyr Asp Leu Ser Ile Glu Asp Leu Lys Gln Phe Arg Gln Leu
                   85                  90                  95
   Gly Ser Arg Thr Pro Gly His Pro Glu Phe Glu Leu Pro Gly Val Glu
               100                 105                 110
   Val Thr Thr Gly Pro Leu Gly Gln Gly Ile Ser Asn Ala Val Gly Met
           115                 120                 125
   Ala Met Ala Gln Ala Asn Leu Ala Ala Thr Tyr Asn Lys Pro Gly Phe
       130                 135                 140
   Thr Leu Ser Asp Asn Tyr Thr Tyr Val Phe Leu Gly Asp Gly Cys Leu
   145                 150                 155                 160
   Gln Glu Gly Ile Ser Ser Glu Ala Ser Ser Leu Ala Gly His Leu Lys
                   165                 170                 175
   Leu Gly Asn Leu Ile Ala Ile Tyr Asp Asp Asn Lys Ile Thr Ile Asp
               180                 185                 190
   Gly Ala Thr Ser Ile Ser Phe Asp Glu Asp Val Ala Lys Arg Tyr Glu
           195                 200                 205
   Ala Tyr Gly Trp Glu Val Leu Tyr Val Glu Asn Gly Asn Glu Asp Leu
       210                 215                 220
   Ala Gly Ile Ala Lys Ala Ile Ala Gln Ala Lys Leu Ser Lys Asp Lys
   225                 230                 235                 240
   Pro Thr Leu Ile Lys Met Thr Thr Thr Ile Gly Tyr Gly Ser Leu His
                   245                 250                 255
   Ala Gly Ser His Ser Val His Gly Ala Pro Leu Lys Ala Asp Asp Val
               260                 265                 270
   Lys Gln Leu Lys Ser Lys Phe Gly Phe Asn Pro Asp Lys Ser Phe Val
           275                 280                 285
   Val Pro Gln Glu Val Tyr Asp His Tyr Gln Lys Thr Ile Leu Lys Pro
       290                 295                 300
   Gly Val Glu Ala Asn Asn Lys Trp Asn Lys Leu Phe Ser Glu Tyr Gln
   305                 310                 315                 320
   Lys Lys Phe Pro Glu Leu Gly Ala Glu Leu Ala Arg Arg Leu Ser Gly
                   325                 330                 335
   Gln Leu Pro Ala Asn Trp Glu Ser Lys Leu Pro Thr Tyr Thr Ala Lys
               340                 345                 350
   Asp Ser Ala Val Ala Thr Arg Lys Leu Ser Glu Thr Val Leu Glu Asp
           355                 360                 365
   Val Tyr Asn Gln Leu Pro Glu Leu Ile Gly Gly Ser Ala Asp Leu Thr
       370                 375                 380
   Pro Ser Asn Leu Thr Arg Trp Lys Glu Ala Leu Asp Phe Gln Pro Pro
   385                 390                 395                 400
   Ser Ser Gly Ser Gly Asn Tyr Ser Gly Arg Tyr Ile Arg Tyr Gly Ile
                   405                 410                 415
   Arg Glu His Ala Met Gly Ala Ile Met Asn Gly Ile Ser Ala Phe Gly
               420                 425                 430
   Ala Asn Tyr Lys Pro Tyr Gly Gly Thr Phe Leu Asn Phe Val Ser Tyr
           435                 440                 445
   Ala Ala Gly Ala Val Arg Leu Ser Ala Leu Ser Gly His Pro Val Ile
       450                 455                 460
   Trp Val Ala Thr His Asp Ser Ile Gly Val Gly Glu Asp Gly Pro Thr
   465                 470                 475                 480
   His Gln Pro Ile Glu Thr Leu Ala His Phe Arg Ser Leu Pro Asn Ile
                   485                 490                 495
   Gln Val Trp Arg Pro Ala Asp Gly Asn Glu Val Ser Ala Ala Tyr Lys
               500                 505                 510
   Asn Ser Leu Glu Ser Lys His Thr Pro Ser Ile Ile Ala Leu Ser Arg
           515                 520                 525
   Gln Asn Leu Pro Gln Leu Glu Gly Ser Ser Ile Glu Ser Ala Ser Lys
       530                 535                 540
   Gly Gly Tyr Val Leu Gln Asp Val Ala Asn Pro Asp Ile Ile Leu Val
   545                 550                 555                 560
   Ala Thr Gly Ser Glu Val Ser Leu Ser Val Glu Ala Ala Lys Thr Leu
                   565                 570                 575
   Ala Ala Lys Asn Ile Lys Ala Arg Val Val Ser Leu Pro Asp Phe Phe
               580                 585                 590
   Thr Phe Asp Lys Gln Pro Leu Glu Tyr Arg Leu Ser Val Leu Pro Asp
           595                 600                 605
   Asn Val Pro Ile Met Ser Val Glu Val Leu Ala Thr Thr Cys Trp Gly
       610                 615                 620
   Lys Tyr Ala His Gln Ser Phe Gly Ile Asp Arg Phe Gly Ala Ser Gly
   625                 630                 635                 640
   Lys Ala Pro Glu Val Phe Lys Phe Phe Gly Phe Thr Pro Glu Gly Val
                   645                 650                 655
   Ala Glu Arg Ala Gln Lys Thr Ile Ala Phe Tyr Lys Gly Asp Lys Leu
               660                 665                 670
   Ile Ser Pro Leu Lys Lys Ala Phe
           675                 680
<210> 124
   <211> 370
   <212> PRT
   <213> Saccharomyces cerevisiae CEN.PK-1D
<400> 124
   Met Ser Glu Ser Pro Met Phe Ala Ala Asn Gly Met Pro Lys Val Asn
    1               5                  10                  15
   Gln Gly Ala Glu Glu Asp Val Arg Ile Leu Gly Tyr Asp Pro Leu Ala
               20                  25                  30
   Ser Pro Ala Leu Leu Gln Val Gln Ile Pro Ala Thr Pro Thr Ser Leu
           35                  40                  45
   Glu Thr Ala Lys Arg Gly Arg Arg Glu Ala Ile Asp Ile Ile Thr Gly
       50                  55                  60
   Lys Asp Asp Arg Val Leu Val Ile Val Gly Pro Cys Ser Ile His Asp
    65                  70                  75                  80
   Leu Glu Ala Ala Gln Glu Tyr Ala Leu Arg Leu Lys Lys Leu Ser Asp
                   85                  90                  95
   Glu Leu Lys Gly Asp Leu Ser Ile Ile Met Arg Ala Tyr Leu Glu Lys
               100                 105                 110
   Pro Arg Thr Thr Val Gly Trp Lys Gly Leu Ile Asn Asp Pro Asp Val
           115                 120                 125
   Asn Asn Thr Phe Asn Ile Asn Lys Gly Leu Gln Ser Ala Arg Gln Leu
       130                 135                 140
   Phe Val Asn Leu Thr Asn Ile Gly Leu Pro Ile Gly Ser Glu Met Leu
   145                 150                 155                 160
   Asp Thr Ile Ser Pro Gln Tyr Leu Ala Asp Leu Val Ser Phe Gly Ala
                   165                 170                 175
   Ile Gly Ala Arg Thr Thr Glu Ser Gln Leu His Arg Glu Leu Ala Ser
               180                 185                 190
   Gly Leu Ser Phe Pro Val Gly Phe Lys Asn Gly Thr Asp Gly Thr Leu
           195                 200                 205
   Asn Val Ala Val Asp Ala Cys Gln Ala Ala Ala His Ser His His Phe
       210                 215                 220
   Met Gly Val Thr Lys His Gly Val Ala Ala Ile Thr Thr Thr Lys Gly
   225                 230                 235                 240
   Asn Glu His Cys Phe Val Ile Leu Arg Gly Gly Lys Lys Gly Thr Asn
                   245                 250                 255
   Tyr Asp Ala Lys Ser Val Ala Glu Ala Lys Ala Gln Leu Pro Ala Gly
               260                 265                 270
   Ser Asn Gly Leu Met Ile Asp Tyr Ser His Gly Asn Ser Asn Lys Asp
           275                 280                 285
   Phe Arg Asn Gln Pro Lys Val Asn Asp Val Val Cys Glu Gln Ile Ala
       290                 295                 300
   Asn Gly Glu Asn Ala Ile Thr Gly Val Met Ile Glu Ser Asn Ile Asn
   305                 310                 315                 320
   Glu Gly Asn Gln Gly Ile Pro Ala Glu Gly Lys Ala Gly Leu Lys Tyr
                   325                 330                 335
   Gly Val Ser Ile Thr Asp Ala Cys Ile Gly Trp Glu Thr Thr Glu Asp
               340                 345                 350
   Val Leu Arg Lys Leu Ala Ala Ala Val Arg Gln Arg Arg Glu Val Asn
           355                 360                 365
   Lys Lys
       370

## Description

### [Technical Field]

The present disclosure relates to a microorganism producing a mycosporine-like amino acid, and a method for producing a mycosporine-like amino acid using the microorganism.

### [Background Art]

The ultraviolet (UV) rays emitted by the sun consists of UVA (with a wavelength of about 320 nm to about 400 nm), UVB (with a wavelength of about 290 nm to about 320 nm), and UVC (with a wavelength of about 100 nm to about 280 nm). It is known that UVA rays penetrate into the dermal layer, mainly induce pigmentation and aging of the skin, and are involved in the occurrence of photosensitive skin disease, whereas UVB rays are high-energy rays that penetrate the epidermis and the basal layer of the dermis and are involved in sunburn, pigmentation, and occurrence of skin cancer.

To prevent these side effects of UV rays, attempts have been made to block UV rays. The types of sunscreen agents include chemical sunscreen agents and physical sunscreen agents. Chemical sunscreen agents prevent the penetration of UV rays mainly by absorption of UV rays, whereas physical sunscreen agents prevent the penetration of UV rays through reflection and scattering.

Components that are known to be contained in the chemical sunscreen agents may include those which mainly absorb UVB rays (*e*.*g*., PABA, PABA esters (amyl dimethyl PABA, octyl dimethyl PABA), cinnamates (cinoxate), salicylates (homomenthyl salicylate), camphor, *etc.*); and those which mainly absorb UVA rays (*e.g.*, benzophenones (oxybenzone, dioxybenzone, and suliso benzene), dibenzoyl methane, anthranilate, *etc.*)*.* Although these chemical sunscreen agents can provide effects of absorbing and blocking UV rays, it is known that some of these chemical sunscreen agents can irritate the skin or eyes, and in particular, PABA, PABA esters, benzophenones, and cinnamates, *etc.* can cause contact dermatitis. Additionally, some others of these chemical sunscreen agents have been reported to cause a photosensitive reaction in the skin, *etc.* Accordingly, in some countries, the use or the amount of use of these chemical sunscreen agents is being limited.

Components that are known to be contained in the physical sunscreen agents may include titanium dioxide, talc (magnesium silicate), magnesium oxide, zinc oxide, kaolin, *etc.* The physical sunscreen agents have advantages in that they do not cause side effects, such as contact dermatitis and that they are not easily removed by water. However, they have disadvantages in that it is difficult for them to maintain an effective content while realizing desired formulations and that a white cast, *etc.* occur when they are applied to the skin.

Mycosporine-like amino acids (MAAs) are materials present in natural organisms and they are known to absorb UVA and UVB effectively. More than 35 species of MAAs are known to be present in nature (Mar. Biol., 1991, 108: 157-166; Planta Med., 2015, 81: 813-820). Recently, MAAs, to which various kinds of sugars are attached, have been reported to exist in microalgae and they have an excellent antioxidant function (Journal of Photochemistry and Photobiology, 2015, 142: 154-168). Additionally, MAAs are known to provide not only an ability blocking UV rays, but also resistance to oxidation, osmosis, heat stress, *etc.* (Comp. Biochem. Physiol.C Toxicol. Pharmacol., 2007, 146: 60-78; J. Photochem. Photobiol. B., 2007, 89: 29-35).

However, the amount of MAAs produced within microalgae is very low to be at the level of several micrograms, and the conditions for separation, extraction, and purification of MAAs after culturing microalgae are complicated. Therefore, it is difficult to mass-produce the MAAs material.

### [Prior Art Documents]

### [Non-patent Documents]

(Non-patent Document 1) Comp. Biochem. Physiol. B 1995, 112: 105-114.
(Non-patent Document 2) FEMS Microbiol Lett. 2007, 269: 1-10.
(Non-patent Document *3*) Ann. Rev. Physiol. 2002, 64: 223-262.
(Non-patent Document 4) Mar. Biol. 1991, 108: 157-166.
(Non-patent Document 5) Journal of Photochemistry and Photobiology B: Biology. 2015, 142: 154-168
(Non-patent Document 6) Biol. Rev. 1999, 74: 311-345.
(Non-patent Document *7)* Mol. Biol. Evol. 2006, 23: 1437-1443.
(Non-patent Document 8) Science, 2010, 329: 1653-1656.
(Non-patent Document 9) Genomics 2010, 95: 120-128.
(Non-patent Document 10) Geomicrobiol. J. 1997. 14: 231-241.
(Non-patent Document 11) Comp. Biochem. Physiol. C Toxicol. Pharmacol. 2007. 146: 60-78.
(Non-patent Document 12) Can. J. Bot. 2003. 81: 131-138.
(Non-patent Document 13) J. Photochem. Photobiol. B. 2007, 89: 29-35.
(Non-patent Document 14) J. Bacteriol. 2011. 193(21): 5923-5928.
(Non-patent Document 15) Planta Med. 2015. 81: 813-820
(Non-patent Document 16) ACS Appl. Mater. Interfaces. 2015. 7: 16558-16564
(Non-patent Document 17) Appl Environ Microbiol. 2016, 82(20): 6167-6173
(Non-patent Document 18) ChemBioChem. 2015, 16: 320-327
(Non-patent Document 19) Methods Mol Biol. 2013, 1073: 43-7
(Non-patent Document 20) Nature Review, 2011, 9: 791-802
(Non-patent Document 21) ACS Synthetic Biology, 2018, 7(2): 664-671.
(Non-patent Document 22) Appl Environ Microbiol. 2013, 80(5): 1763-1769.

### [Disclosure]

### [Technical Problem]

The present inventors have made many efforts to increase the production of MAAs in microorganisms. As a result, they have confirmed that the production of MAAs can be increased in microorganisms producing MAAs through various studies associated with the enhancement of the activity of 2-dehydro-3-deoxyphosphoheptonate aldolase, phosphoenolpyruvate synthetase, and transketolase proteins in microorganisms, thereby completing the present disclosure.

### [Technical Solution]

An object of the present disclosure is to provide a microorganism producing a mycosporine-like amino acid (MMA), in which an activity of at least one protein selected from the group consisting of 2-dehydro-3-deoxyphosphoheptonate aldolase, phosphoenolpyruvate synthetase, and transketolase is enhanced.

Another object of the present disclosure is to provide a method for producing a mycosporine-like amino acid, which includes culturing the microorganism in a medium; and recovering the mycosporine-like amino acid from the cultured microorganism or medium.

Still another object of the present disclosure ,not part of the claimed invention, is to provide a use of the microorganism for producing a mycosporine-like amino acid.

### [Advantageous Effects]

Since the microorganism of the present disclosure has an increased mycosporine-like amino acid (MMA)-producing ability, it can be effectively used in producing mycosporine-like amino acids.

### [Best Mode for Carrying Out the Invention]

The present disclosure is described in detail hereinbelow. Meanwhile, respective descriptions and embodiments disclosed in the present disclosure may also be applied to other descriptions and embodiments. That is, all combinations of various elements disclosed in the present disclosure fall within the scope of the present disclosure. In addition, the scope of the present disclosure is not limited by the specific description below. In addition, one of ordinary skill in the art can recognize or identify a number of equivalents with regard to certain aspects of the present disclosure only by routine experimentation. Further, such equivalents are intended to be included in the present disclosure.

To achieve the above objects, an aspect of the present disclosure provides a microorganism producing a mycosporine-like amino acid (MMA), in which an activity of at least one protein selected from the group consisting of 2-dehydro-3-deoxyphosphoheptonate aldolase, phosphoenolpyruvate synthetase, and transketolase is enhanced.

As used herein, the term "2-dehydro-3-deoxyphosphoheptonate aldolase" refers to an enzyme that catalyzes the reversible reaction of the following reaction scheme, and specifically, may refer to an enzyme that synthesizes 3-deoxy-D-arabino-heptulosonate-7-phosphate (DAHP), but is not limited thereto.

[Reaction Scheme] phosphoenolpyruvate + D-erythrose-4-phosphate + H₂O ↔ 3-deoxy-D-arabinoheptulosonate-7-phosphate + phosphate

In the present disclosure, 2-dehydro-3-deoxyphosphoheptonate aldolase can be used interchangeably with 3-deoxy-D-arabino-heptulosonate-7-phosphate (DAHP) synthase.

As used herein, the term "phosphoenolpyruvate synthetase" refers to an enzyme that catalyzes the reversible reaction of the following reaction scheme, and specifically, may refer to an enzyme that synthesizes phosphoenolpyruvate, but is not limited thereto.

[Reaction Scheme] ATP + pyruvate + H₂O ↔ AMP + phosphoenolpyruvate + phosphate

As used herein, the term "transketolase" refers to an enzyme that catalyzes the reversible reaction of the following reaction scheme.

[Reaction Scheme] sedoheptulose 7-phosphate + D-glyceraldehyde 3-phosphate ↔ D-ribose 5-phosphate + D-xylulose 5-phosphate or fructose 6-phosphate + D-glyceraldehyde 3-phosphate ↔ erythrose 4-phosphate + D-xylulose 5-phosphate

The genetic information of the 2-dehydro-3-deoxyphosphoheptonate aldolase, phosphoenolpyruvate synthetase, and transketolase can be obtained from known database (*e*.*g.*, GenBank database of the National Center for Biotechnology Information (NCBI), *etc*.), but is not limited thereto.

The 2-dehydro-3-deoxyphosphoheptonate aldolase, phosphoenolpyruvate synthetase, and transketolase are not limited to their origins or sequences because there are cases where the proteins showing the activities differ in their amino acid sequences depending on the species of a microorganism or the microorganism itself.

Specifically, the 2-dehydro-3-deoxyphosphoheptonate aldolase may be a protein including the amino acid sequence of SEQ ID NO: 2, 37, or 124; the phosphoenolpyruvate synthetase may be a protein including the amino acid sequence of SEQ ID NO: 19 or 98; and the transketolase may be a protein including the amino acid sequence of SEQ ID NO: 24, 96, or 123, but the amino acid sequences of these proteins are not limited thereto. As used herein, the term "a protein including an amino acid sequence" can be used interchangeably with the expression of "a protein having an amino acid sequence" or "a protein consisting of an amino acid sequence".

Additionally, in the present disclosure, these enzymes may include those proteins which have the amino acid sequences of SEQ ID NOS described above as well as 80% or higher, 85% or higher, 90% or higher, 91% or higher, 92% or higher, 93% or higher, 94% or higher, 95% or higher, 96% or higher, 97% or higher, 98% or higher, 99% or higher homology or identity to the above amino acid sequences, as long as these proteins have a biological activity identical or corresponding to each of these enzymes.

Additionally, it is apparent that any protein which has an amino acid sequence with deletion, modification, substitution, or addition in part of the sequence can also be included within the scope of the present disclosure, as long as the amino acid sequence has a homology or identity to the SEQ ID NOS described above and has a biological activity substantially identical or corresponding to the enzyme proteins of the SEQ ID NOS described above.

As used herein, the term "homology or identity" refers to a degree of matching between given amino acid sequences or nucleotide sequences and may be expressed as a percentage. In the present disclosure, a homologous sequence having an activity identical or similar to a given amino acid sequence or nucleotide sequence is represented as "% homology" or "% identity". For example, homology may be confirmed using standard software, specifically BLAST 2.0, for calculating parameters such as score, identity, and similarity or by comparing sequences by southern hybridization under defined stringent conditions. Defined appropriate hybridization conditions may be within the scope of the art and may be determined by a method well known to those skilled in the art (*e.g.*, J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York). The term "stringent conditions" refers to conditions which enables specific hybridization between polynucleotides. For example, such conditions are specifically disclosed in a literature (*e*.*g*., J. Sambrook *et al.*, *supra*)*.*

The 2-dehydro-3-deoxyphosphoheptonate aldolase, phosphoenolpyruvate synthetase, and transketolase of the present disclosure may include the polynucleotides encoding proteins which have the amino acid sequences of the SEQ ID NOS described above, or 80% or higher, 85% or higher, 90% or higher, 91% or higher, 92% or higher, 93% or higher, 94% or higher, 95% or higher, 96% or higher, 97% or higher, 98% or higher, 99% or higher homology or identity to the amino acid sequences of the SEQ ID NOS described above, as long as these polynucleotides have a biological activity identical or corresponding to each of these enzymes.

Additionally, considering the codons preferred in an organism, where the protein is to be expressed, due to codon degeneracy, various modifications may be performed in the coding region of the nucleotide sequence within the scope not altering the amino acid sequence of the protein to be expressed from the coding region. Therefore, any polynucleotide having a sequence encoding each of the enzyme proteins can be included without limitation.

Additionally, any sequence which encodes a protein having an activity of the 2-dehydro-3-deoxyphosphoheptonate aldolase, phosphoenolpyruvate synthetase, or transketolase enzyme proteins by hybridizing with any probe that can be prepared from known gene sequences (*e*.*g*., complementary sequences to all or part of the above polynucleotide sequence) under stringent conditions, may be included without limitation.

The term "stringent conditions" refers to conditions which enables specific hybridization between polynucleotides. Such conditions are specifically described in a literature (*e.g.*, J Sambrook *et al.*, *supra*)*.* For example, the condition for a hybridization between genes having a high homology or identity, a homology or identity of 40% or higher, specifically 90% or higher, more specifically 95% or higher, even more specifically 97% or higher, and most specifically 99% or higher, without a hybridization between genes having a homology or identity of lower than the homologies or identities described above; or conventional washing condition for southern hybridization, *i.e.*, washing once, specifically, twice or three times at a salt concentration and temperature corresponding to 60°C, 1×SSC, 0.1% SDS, specifically 60°C, 0.1×SSC, 0.1% SDS, and more specifically 68°C, 0.1×SSC, 0.1% SDS, may be listed.

Hybridization requires that two polynucleotides have complementary sequences, although mismatches between bases are possible depending on the stringency of the hybridization. The term "complementary" is used to describe the relationship between nucleotide bases that can hybridize with each other. For example, with respect to DNA, adenosine is complementary to thymine, and cytosine is complementary to guanine. Accordingly, the present disclosure may also include isolated polynucleotide fragments complementary to the entire sequence as well as substantially similar polynucleotide sequences.

Specifically, polynucleotides having a homology or identity can be detected using hybridization conditions that include a step of hybridizing at a Tm value of 55°C and using the conditions described above. Additionally, the Tₘ value may be 60°C, 63°C, or 65°C, but the temperature is not limited thereto and may be appropriately adjusted by those skilled in the art according to the purpose.

The stringency suitable for the hybridization of polynucleotides depends on the length and degree of complementarity of the polynucleotides, and the variables are well known in the art (*see* Sambrook *et al.*, *supra*, 9.50 to 9.51 and 11.7 to 11.8). As used herein, the term "enhancement of activity" means that an activity of an enzyme protein is introduced, or the activity is enhanced compared to its endogenous activity or the activity before its modification in which a microorganism possesses. The "introduction" of an activity means that a microorganism naturally or artificially exhibits the activity of a particular protein which was not originally possessed in the microorganism. Specifically, a microorganism with an enhanced activity of an enzyme protein refers to a microorganism in which the activity of an enzyme protein is enhanced compared to that of a natural wild-type microorganism or non-modified microorganism. The enhancement of an activity may include, for example, both the enhancement of an activity by introducing an exogenous 2-dehydro-3-deoxyphosphoheptonate aldolase, phosphoenolpyruvate synthetase, and/or transketolase into a microorganism; or the enhancement of the activity of the endogenous 2-dehydro-3-deoxyphosphoheptonate aldolase, phosphoenolpyruvate synthetase, and/or transketolase.

Specifically, in the present disclosure, the method for the enhancement of an activity may include:
(1) a method of increasing the copy number of the polynucleotides encoding the enzymes;
(2) a method of modifying the expression control sequence for the increase of the expression of the polynucleotides;
(3) a method of modifying the polynucleotide sequences on the chromosome for the enhancement of the activities of the enzymes; or
(4) a method of modifying for the enhancement by a combination of the above methods (1) to (3), *etc.*, but the methods are not limited thereto.

The method (1) of increasing the copy number of the polynucleotides may be performed in a form where the polynucleotide is operably linked to a vector or by inserting the polynucleotide into the chromosome of a host cell, but the method is not particularly limited thereto. Additionally, as an alternative, the copy number may be increased by introducing an exogenous polynucleotide exhibiting the activity of an enzyme or a codon-optimized modified polynucleotide of the polynucleotide above into a host cell. The exogenous polynucleotide may be used without limitation in its origin or sequence as long as the polynucleotide exhibits an activity identical or similar to the enzyme. The introduction may be performed by those skilled in the art by appropriately selecting a known transformation method, and the activity of the enzyme may be enhanced such that the introduced polynucleotide is expressed in the host cell thereby producing the enzyme.

Next, the method (2) of modifying the expression control sequence for the increase of the expression of the polynucleotides may be performed by inducing a modification in the sequence by deletion, insertion, non-conservative or conservative substitution, or a combination thereof so as to further enhance the activity of the expression control sequence; or by replacing the nucleic acid sequence with a nucleic acid sequence having a stronger activity, but the method is not particularly limited thereto. The expression control sequence may include a promoter, an operator sequence, a sequence encoding a ribosome-binding site, sequences controlling the termination of transcription and translation, *etc.*, but the expression control sequence is not particularly limited thereto.

Specifically, a strong heterologous promoter instead of the original promoter may be linked upstream of the polynucleotide expression unit, and examples of the strong promoter may include a CJ7 promoter, a lysCP1 promoter, an EF-Tu promoter, a groEL promoter, an aceA or aceB promoter, *etc.* More specifically, the polynucleotide expression unit may be operably linked to a *Corynebacterium*-derived promoter such as lysCP1 promoter (WO 2009/096689), a CJ7 promoter (WO 2006/065095), an SPL promoter (KR 10-1783170 B), or an o2 promoter (KR 10-1632642 B) so as to improve the expression rate of the polynucleotide encoding the enzyme, but the method is not limited thereto.

Additionally, the method (3) of modifying the polynucleotide sequence on the chromosome may be performed by inducing a modification in the expression control sequence by deletion, insertion, non-conservative or conservative substitution, or a combination thereof so as to further enhance the activity of the polynucleotide sequence; or by replacing the nucleic acid sequence with an improved polynucleotide sequence having a stronger activity, but the method is not particularly limited thereto.

Lastly, the method (4) of modifying for enhancement by a combination of the methods (1) to (3) may be performed by applying one or more methods together among the methods: a method of increasing the copy number of the polynucleotides encoding the enzymes; a method of modifying the expression control sequence for the increase of the expression; and a method of modifying the polynucleotide sequences on the chromosome, or a method of modifying the exogenous polynucleotides exhibiting the activity of the enzyme or a codon-optimized modified polynucleotide thereof.

The polynucleotides may be described as genes when they are an assembly of polynucleotides capable of functioning. In the present disclosure, polynucleotides and genes can be used interchangeably, and polynucleotide sequences and nucleotide sequences can be used interchangeably.

As used herein, the term "vector" refers to a DNA construct including a nucleotide sequence of a polynucleotide encoding a target protein, in which the target protein is operably linked to a suitable control sequence so that it can be expressed in an appropriate host. The control sequence may include a promoter capable of initiating transcription, any operator sequence for controlling the transcription, a sequence encoding an appropriate mRNA ribosome-binding site, and sequences for controlling the termination of transcription and translation. The vector, after being transformed into a suitable host cell, may be replicated or function irrespective of the host genome, or may be integrated into the host genome itself.

The vector used in the present disclosure is not particularly limited as long as the vector can be replicated in a host cell, and any vector known in the art may be used. Examples of conventional vectors may include a natural or recombinant plasmid, cosmid, virus, and bacteriophage. For example, pWE15, M13, MBL3, MBL4, IXII, ASHII, APII, t10, t11, Charon4A, and Charon21A, *etc.* may be used as a phage vector or cosmid vector; and pBR, pUC, pBluescriptII, pGEM, pTZ, pCL, and pET, *etc.* may be used as a plasmid vector. Specifically, vectors such as pDZ, pACYC177, pACYC184, pCL, pECCG117, pUC19, pBR322, pMW118, pCC1BAC, pSKH, pRS-413, pRS-414, and pRS-415, *etc.* may be used, but the vectors are not limited thereto.

The vectors to be used in the present disclosure are not particularly limited, but any known expression vector may be used. Additionally, a polynucleotide encoding a target protein in the chromosome can be inserted into the chromosome with a vector for chromosomal insertion in a cell. The insertion of a polynucleotide into the chromosome can be performed using any method known in the art (*e.g.*, by homologous recombination), but the method of insertion is not limited thereto. A selection marker for confirming the insertion into the chromosome may be further included. The selection marker is used for selection of cells transformed with a vector (*i.e.*, to confirm whether a target nucleic acid molecule has been inserted) and markers capable of providing selectable phenotypes (*e*.*g*., drug resistance, auxotrophy, resistance to cytotoxic agents, and expression of surface proteins) may be used. Under the circumstances where selective agents are treated, only the cells capable of expressing the selection markers can survive or express other phenotypic traits, and thus the transformed cells can easily be selected.

As used herein, the term "transformation" refers to the introduction of a vector including a polynucleotide encoding a target protein into a host cell so that the protein encoded by the polynucleotide can be expressed in the host cell. The transformed polynucleotide may not be particularly limited as long as it can be expressed in a host cell, regardless of whether the transformed polynucleotide is inserted into the chromosome of the host cell to be located therein or located outside of the chromosome. Additionally, the polynucleotide includes DNA and RNA encoding a target protein. As long as the polynucleotide can be introduced into a host cell and expressed therein, it does not matter in which form the polynucleotide is introduced. For example, the polynucleotide may be introduced into a host cell in the form of an expression cassette, which is a gene construct including all essential elements for self-expression. The expression cassette may include a promoter, which is conventionally operably linked to the polynucleotide, a transcription termination signal, a ribosome-binding site, and a translation termination signal. The expression cassette may be a self-replicable expression vector. Additionally, the polynucleotide may be one which is introduced into a host cell as a polynucleotide itself and linked to a sequence necessary to be expressed in a host cell, but is not limited thereto. The methods for transformation include any method for introducing a nucleic acid into a cell, and may be performed by selecting a suitable standard technique known in the art depending on a host cell. For example, the transformation methods may include electroporation, calcium phosphate (CaPO₄) precipitation, calcium chloride (CaCl₂) precipitation, microinjection, polyethylene glycol (PEG) method, EAE-dextran method, cationic liposome method, lithium acetate-DMSO method, *etc.*, but the methods are not limited thereto.

Additionally, as used herein, the term "operably linked" means a functional linkage between a promoter sequence which initiates and mediates transcription of the polynucleotide encoding the target protein of the present disclosure and the polynucleotide sequence. An operable linkage may be prepared by a genetic recombination technique known in the art, and site-specific DNA cleavage and ligation may be prepared using a restriction enzyme, a ligase, *etc.*, known in the art, without being limited thereto.

In the microorganism of the present disclosure, the activity of 3-dehydroquinate dehydratase may be further inactivated.

As used herein, the term "3-dehydroquinate dehydratase" refers to an enzyme that catalyzes the reversible reaction in the reaction scheme below, and specifically, it can convert 3-dehydroquinate to 3-dehydroshikimate, but is not limited thereto.

[Reaction Scheme] 3-dehydroquinate ↔ 3-dehydroshikimate + H₂O

3-dehydroquinate dehydratase is not limited to its origin or sequence because there are cases where the proteins showing the activity of 3-dehydroquinate dehydratase differ in their amino acid sequences depending on the species of a microorganism or the microorganism. Specifically, the 3-dehydroquinate dehydratase may be a protein including the amino acid sequence of SEQ ID NO: 90, but is not limited thereto. Additionally, the 3-dehydroquinate dehydratase may include an amino acid sequence of SEQ ID NO: 90 or an amino acid sequence having a homology or identity of at least 80%, 90%, 95%, 96%, 97%, 98%, or 99% to SEQ ID NO: 90. Additionally, it is apparent that any amino acid sequence with deletion, modification, substitution, or addition in part of the sequence can also be included within the scope of the present disclosure, as long as the amino acid sequence has the homology or identity described above and has a biological activity identical or corresponding to the above protein.

As used herein, the term "inactivation" refers to a case where the activity of an enzyme protein is weakened compared to the endogenous activity of the enzyme protein or its activity before modification originally possessed by a microorganism; a case where the protein is not expressed at all; or a case where the protein is expressed but has no activity. The inactivation is a concept which includes: a case where the activity of an enzyme itself is weakened compared to its endogenous activity possessed by a microorganism due to a modification of the polynucleotide encoding the enzyme, *etc.* or the activity is removed; a case where the degree of overall intracellular activity of an enzyme is lower compared to that of its wild-type microorganism or the activity is removed, due to the inhibition of expression or translation of the gene encoding the enzyme, *etc.*; a case where all or part of the gene encoding the enzyme is deleted; and a combination thereof, but the inactivation is not limited thereto. That is, a microorganism in which the activity of an enzyme is inactivated refers to a microorganism in which the activity of an enzyme protein is lower compared to that of its natural wild-type microorganism or non-modified microorganism or in which the activity is removed.

The inactivation of the activity of an enzyme may be achieved by the application of various methods known in the art. Examples of the above methods may include: 1) a method of deleting all or part of the gene encoding the enzyme on the chromosome; 2) a method of modifying the expression control sequence to reduce the expression of the gene encoding the protein on the chromosome ; 3) a method of modifying the sequence of the gene encoding the protein on the chromosome so that the activity of the protein is removed or weakened; 4) a method of introducing an antisense oligonucleotide (*e*.*g*., antisense RNA), which binds complementarily to a transcript of the gene encoding the protein on the chromosome; 5) a method of making the attachment of a ribosome impossible by a secondary structure formed by adding a sequence, which is complementary to the Shine-Dalgarno (SD) sequence, on the front end of the SD sequence of the gene encoding the protein on the chromosome; 6) a method of reverse transcription engineering (RTE), in which a promoter that is transcribed reversely is added to the 3' terminal of the open reading frame (ORF) of the polynucleotide sequence encoding the protein, *etc.*; and the inactivation may be achieved by a combination thereof, but the methods are not particularly limited thereto.

The method of deleting all or part of the gene encoding the enzyme on the chromosome may be performed by replacing the polynucleotide encoding the endogenous target protein within the chromosome with a polynucleotide or marker gene having a partially deleted nucleotide sequence using a vector for chromosomal insertion. As an example of the method for deleting all or part of a polynucleotide, a method for deleting a polynucleotide by homologous recombination may be used, but the method is not limited thereto.

The method of modifying the expression control sequence may be performed by inducing a modification of the nucleic acid sequence in the expression control sequence via deletion, insertion, conservative or non-conservative substitution, or a combination thereof so as to further weaken the activity of the expression control sequence; or by replacing the nucleic acid sequence with a nucleic acid sequence having a weaker activity. The expression control sequence may include a promoter, an operator sequence, a sequence encoding a ribosome-binding site, and a sequence for regulating transcription and translation, but is not limited thereto.

The method of modifying a gene sequence on the chromosome may be performed by inducing a modification in the gene sequence via deletion, insertion, conservative or non-conservative substitution, or a combination thereof so as to further weaken the activity of the enzyme; or by replacing the gene sequence with a gene sequence modified to have a weaker activity or a gene sequence modified to have no activity at all, but the method is not limited thereto.

In the above, the term "part", although what it refers to may vary depending on the kinds of polynucleotide, may be specifically 1 nucleotide to 300 nucleotides, more specifically 1 nucleotide to 100 nucleotides, and even more specifically 1 nucleotide to 50 nucleotides, but is not particularly limited thereto.

In the microorganism of the present disclosure, the activity of 3-dehydroquinate synthase protein can be further strengthened compared to that of a non-modified microorganism.

The 3-dehydroquinate synthase refers to an enzyme that catalyzes the reversible reaction of the following reaction scheme, and specifically can synthesize 3-dehydroquinate (3-DHQ), but is not limited thereto.

[Reaction Scheme] 3-deoxy-arabino-heptulosonate-7-phosphate ↔ 3-dehydroquinate + phosphate

As used herein, the term "mycosporine-like amino acid (MAA)" refers to a cyclic compound that absorbs ultraviolet (UV) rays. In the present disclosure, the mycosporine-like amino acid is not limited as long as it can absorb UV rays, and specifically, it may be a compound which has the central ring of cyclohexanone or cyclohexenimine; or may be a compound in which various materials (*e.g.*, amino acids, *etc.*) are bound to the central ring, and more specifically, it may be mycosporine-2-glycine, palythinol, palythenic acid, deoxygadusol, mycosporine-methylamine-threonine, mycosporine-glycine-valine, palythine, asterina-330, shinorine, porphyra-334, euhalothece-362, mycosporine-glycine, mycosporine-ornithine, mycosporine-lysine, mycosporine-glutamic acid-glycine, mycosporine-methylamine-serine, mycosporine-taurine, palythene, palythine-serine, palythine-serine-sulfate, palythinol, usujirene, or a combination thereof.

In the present disclosure, the term mycosporine-like amino acid may be used interchangeably with MAA and MAAs.

As used herein, the term "microorganism producing a mycosporine-like amino acid (MAA)" may refer to a microorganism which includes a gene of an enzyme involved in the biosynthesis of MAA or a cluster of these genes, or a microorganism in which the cluster is introduced or enhanced. Additionally, as used herein, the term "mycosporine-like amino acid (MAA) biosynthesis gene cluster" may refer to a group of genes encoding the enzymes involved in MAA biosynthesis, and specifically, may include an MAA biosynthesis gene, a gene of an enzyme having an activity of attaching an additional amino acid residue to MAA, or a cluster of the above genes. The MAA biosynthesis gene includes both the exogenous genes and/or endogenous genes of a microorganism, as long as the microorganism including such gene can produce MAA. The exogenous genes may be homogeneous or heterogeneous.

The species of the microorganisms from which the MAA biosynthesis gene is derived is not limited, as long as the microorganisms including the same can produce enzymes involved in MAA biosynthesis and consequently can produce MAA. Specifically, the species of the microorganisms from which the MAA biosynthesis gene is derived may be cyanobacteria (*e.g., Anabaena variabilis*, *Nostoc punctiforme*, *Nodularia spumigena*, *Cyanothece sp.* PCC 7424, *Lyngbya sp.* PCC 8106, *Microcystis aeruginosa*, *Microcoleus chthonoplastes*, *Cyanothece sp.* ATCC 51142, *Crocosphaera watsonii*, *Cyanothece sp.* CCY 0110, *Cylindrospermum stagnate sp.* PCC 7417, *Aphanothece halophytica*, or *Trichodesmium erythraeum*); fungi (*e.g.*, *Magnaporthe orzyae*, *Pyrenophora tritici-repentis*, *Aspergillus clavatus*, *Nectria haematococca*, *Aspergillus nidulans*, *Gibberella zeae, Verticillium albo-atrum*, *Botryotinia fuckeliana*, or *Phaeosphaeria nodorum*); or *Nematostella vectensis; Heterocapsa triquetra, Oxyrrhis marina, Karlodinium micrum, Actinosynnema mirum*, *etc.*, but are not limited thereto.

According to an embodiment, the microorganism of the present disclosure, which produces MAA, includes an MAA biosynthesis gene or a cluster thereof. Specifically, in the microorganism, a MAA biosynthesis gene cluster may be introduced or the activities of the proteins encoded by the genes may be enhanced compared to the endogenous activities or the activities before modification, but the microorganism is not limited thereto.

Additionally, although the MAA biosynthesis gene are not limited in their names of the enzymes or the microorganisms from which these genes are derived, as long as the microorganisms can produce MAA, the MAA biosynthesis gene may include gene encoding enzyme protein having an activity identical and/or similar to one or more, specifically, one or more, two or more, three or more, or all of the enzyme proteins selected from the group consisting of 2-demethyl 4-deoxygadusol synthase, O-methyltransferase, and a C-N ligase.

For example, the 2-demethyl 4-deoxygadusol synthase is an enzyme which converts sedoheptulose-7-phosphate to 2-demethyl-4-deoxygadusol. The O-methyltransferase is an enzyme which converts 2-demethyl-4-deoxygadusol to 4-deoxygadusol, and glycylation of the 4-deoxygadusol is catalyzed by the C-N ligase.

Additionally, the microorganism producing MAAs may include a gene of an enzyme, which has an activity of attaching an additional amino acid residue to MAA, or a cluster of the genes. Although the above gene or a cluster of the genes are not limited in their names of the enzymes or the microorganisms from which these genes are derived, as long as the microorganisms producing MAAs can produce MAAs to which two or more amino acid residues are attached. The microorganisms producing MAAs may include gene encoding enzyme protein having an activity identical and/or similar to one or more, specifically, one or more, two or more, three or more, or all of the enzyme proteins, selected from the group consisting of non-ribosomal peptide synthetase (NRPS), a non-ribosomal peptide synthetase-like enzyme (NRPS-like enzyme), and D-alanine D-alanine ligase (D-Ala D-Ala ligase; DDL).

Some of the MAAs include a second amino acid residue in a mycosporine-glycine. The one or more enzymes, which are selected from the group consisting of non-ribosomal peptide synthetase, a non-ribosomal peptide synthetase-like enzyme, and D-Ala D-Ala ligase, can attach a second amino acid residue to a mycosporine-glycine.

According to an embodiment, the microorganism producing MAAs may include, without limitation on enzyme names or the species of the microorganisms from which the MAA biosynthesis genes are derived, enzymes as long as they have an activity capable of attaching a second amino acid to a mycosporine-glycine, as in the non-ribosomal peptide synthetase, non-ribosomal peptide synthetase-like enzyme, and D-Ala D-Ala ligase.

For example, the non-ribosomal peptide synthetase-like enzyme (Ava_3855) in *Anabaena variabilis* or D-Ala D-Ala ligase (NpF5597) in *Nostoc punctiforme* can attach a serine residue to mycosporine-glycine to form shinorine. In another example, mycosporine-2-glycine can be formed by the attachment of a second glycine residue by D-Ala D-Ala ligase homolog (Ap_3855) in *Aphanothece halophytica.* Similarly, in *Actinosynnema mirum*, serine or alanine can be attached by D-Ala D-Ala ligase and thereby shinorine or mycosporine-glycine-alanine can be formed. The microorganism according to an embodiment of the present disclosure can select and include those enzymes which are suitable for the production of desired MAAs among the above-described enzymes or the enzymes having activities identical and/or similar to the above-described enzymes.

The 2-demethyl 4-deoxygadusol synthase, O-methyltransferase, a C-N ligase, non-ribosomal peptide synthetase, non-ribosomal peptide synthetase-like enzyme, and/or D-Ala D-Ala ligase that can be used in the present disclosure are not limited to the species of microorganisms from which these enzymes are derived, as long as these enzymes are known to be able to perform the functions and roles identical and/or similar to those of the above-described enzymes, and the range of homology or identity values among them is also not limited. For example, MylA, MylB, MylD, MylE, and MylC of *C*. *stagnale* PCC 7417 are homologous with the 2-demethyl 4-deoxygadusol synthase, O-methyltransferase, a C-N ligase, and a D-Ala D-Ala ligase derived from *Anabaena variabilis* and *Nostoc punctiforme*, and the degree of similarity between them is in a range of about 61% to about 88% (Appl Environ Microbiol, 2016, 82(20), 6167-6173; J Bacteriol, 2011, 193(21), 5923-5928). That is, the enzymes that can be used in the present disclosure are not significantly limited in the species from which the enzymes are derived, sequence homology, or sequence identity, as long as they are known to exhibit identical and/or similar functions and effects. Additionally, the non-patent documents described in Prior Art Documents are included in their entirety as a reference to the present disclosure as a whole.

Additionally, the MAA biosynthesis gene may be a polynucleotide encoding a protein which includes an amino acid sequence of SEQ ID NO: 115, 116, 117, 118, 119, 120, 121, or 122, but the MAA biosynthesis gene is not limited thereto.

Additionally, the MAA biosynthesis gene may include a nucleotide sequence encoding a protein which includes the amino acid sequence having 50%, 60%, or 70% or higher, specifically 80% or higher, more specifically 90% or higher, even more specifically 95% or higher, and most specifically 99% or higher homology or identity to the amino acid sequence of SEQ ID NO: 115, 116, 117, 118, 119, 120, 121, or 122, and may include without limitation a nucleotide sequence encoding a protein which is out of the range of the above homology or identity, as long as the microorganism can produce MMA. Specifically, the MAA biosynthesis gene may include a nucleotide sequence of SEQ ID NO: 102, 103, 104, 105, 106, 107, 108, or 109, but is not limited thereto.

Additionally, it is apparent that any amino acid sequence with deletion, modification, substitution, or addition in part of the sequence can also be included in the present disclosure, as long as the amino acid sequence has a homology or identity to the above sequences and substantially has a biological activity identical or corresponding to the proteins of the SEQ ID NOS described above.

Additionally, considering the codons preferred in an organism, where the protein is to be expressed, due to codon degeneracy, various modifications may be performed in the coding region of the nucleotide sequence within the scope not altering the amino acid sequence of the protein to be expressed from the coding region. Therefore, with regard to the MAA biosynthesis gene, any nucleotide sequence can be included in the present disclosure without limitation as long as the nucleotide sequence is a nucleotide sequence, which encodes a protein involved in the MAA biosynthesis.

Alternatively, any sequence which encodes a protein involved in the MAA biosynthesis, by hybridizing with any probe that can be prepared from known gene sequences (*e*.*g*., complementary sequences to all or part of the polynucleotide sequence) under stringent conditions, can be included in the present disclosure without limitation.

According to an embodiment, a microorganism producing MAA may include MAA biosynthesis genes having different origins.

In the present disclosure, the enhancement of a protein activity and/or the introduction of genes may be performed in a simultaneous, sequential, and reverse order regardless of the order.

The microorganism producing MAA can produce MAA by including a MAA biosynthesis gene cluster, and additionally, may be a microorganism in which the MAA-producing ability is increased by enhancing the activity of one or more proteins selected from the group consisting of 2-dehydro-3-deoxyphosphoheptonate aldolase, phosphoenolpyruvate synthetase, and transketolase. Additionally, the microorganism of the present disclosure is not limited as long as it is a microorganism in which the MAA-producing ability is increased by enhancing the activity of one or more proteins selected from the group consisting of 2-dehydro-3-deoxyphosphoheptonate aldolase, phosphoenolpyruvate synthetase. Specifically, the microorganism of the present disclosure may be a microorganism of the genus *Corynebacterium,* a microorganism of the genus *Escherichia*, or a yeast.

The microorganism of the genus *Corynebacterium* may be, specifically, *Corynebacterium glutamicum, Corynebacterium ammoniagenes, Brevibacterium lactofermentum, Brevibacterium flavum*, *Corynebacterium thermoaminogenes*, *Corynebacterium efficiens*, *etc.*, and more specifically may be *Corynebacterium glutamicum,* but the microorganism is not limited thereto.

The microorganism of the genus *Escherichia* may be, specifically, *Escherichia albertii*, *Escherichia coli*, *Escherichia fergusonii*, *Escherichia hermannii*, *Escherichia vulneris*, *etc.*, and more specifically may be *Escherichia coli*, but the microorganism is not limited thereto.

The yeast may be, specifically, *Saccharomycotina* and *Taphrinomycotina* of the phylum *Ascomycota*, *Agaricomycotina* of the phylum *Basidiomycota*, a microorganism belonging to the phylum *Pucciniomycotina*, *etc.*, more specifically a microorganism of the genus *Saccharomyces*, a microorganism of the genus *Schizosaccharomyces*, a microorganism of the genus *Phaffia*, a microorganism of the genus *Kluyveromyces*, a microorganism of the genus *Pichia*, and a microorganism of the genus *Candida*, and more specifically *Saccharomyces cerevisiae,* but the microorganism is not limited thereto.

Still another aspect of the present disclosure provides a method for producing a mycosporine-like amino acid, which includes culturing the microorganism of the present disclosure in a medium; and recovering the mycosporine-like amino acid (MAA) from the cultured microorganism or medium.

The "microorganism" and "mycosporine-like amino acid (MAA)" are as described above.

As used herein, the term "culture" means that the microorganism is grown under appropriately controlled environmental conditions. The culture process of the present disclosure can be performed in a suitable culture medium and culture conditions known in the art. Such a culture process may be easily adjusted for use by those skilled in the art according to the microorganism to be selected. The step of culturing the microorganism may be performed in batch culture, continuous culture, fed-batch culture, *etc.* known in the art, but the step of culturing the microorganism is not particularly limited thereto. The medium and other culture conditions used for culturing the microorganism of the present disclosure are not particularly limited, but any medium used in the conventional culture for a microorganism may be used. Specifically, the microorganism of the present disclosure may be cultured under aerobic conditions in a conventional medium containing an appropriate carbon source, nitrogen source, phosphorus source, inorganic compound, amino acid, and/or vitamin, *etc.* while adjusting temperature, pH, *etc.* Specifically, the pH may be adjusted using a basic compound (*e*.*g*., sodium hydroxide, potassium hydroxide, or ammonia) or an acidic compound (*e.g.*, phosphoric acid or sulfuric acid) so as to obtain an optimal pH (*e.g.*, pH 5 to 9, specifically pH 6 to 8, and most specifically pH 6.8), but the method of pH adjustment is not limited thereto. Additionally, oxygen or oxygen-containing gas may be injected into the culture in order to maintain an aerobic state of the culture; or nitrogen, hydrogen, or carbon dioxide gas may be injected into the the culture without gas injection in order to maintain an anaerobic or microaerobic state of the cultured, but the gas is not limited thereto. Additionally, the culture temperature may be maintained at 20°C to 45°C, and specifically 25°C to 40°C, and the culture may be performed for about 10 hours to about 160 hours, without being limited thereto. Additionally, during the culture, an antifoaming agent (*e.g.*, fatty acid polyglycol ester) may be added to prevent foam generation, but is not limited thereto.

Additionally, as a carbon source to be used in the medium for culture, saccharides and carbohydrates (*e*.*g*., glucose, sucrose, lactose, fructose, maltose, molasses, starch, and cellulose), oils and fats (*e*.*g*., soybean oil, sunflower oil, peanut oil, and coconut oil), fatty acids (*e.g.*, palmitic acid, stearic acid, and linoleic acid), alcohols (*e.g.*, glycerol and ethanol), organic acids (*e.g.*, acetic acid), *etc.* may be used alone or in combination, but the carbon source is not limited thereto. As a nitrogen source, a nitrogen-containing organic compound (*e.g.*, peptone, yeast extract, meat gravy, malt extract, corn steep liquor, bean flour, and urea), and an inorganic compound (*e*.*g*., ammonium sulfate, ammonium chloride, ammonium phosphate, ammonium carbonate, and ammonium nitrate), *etc.* may be used alone or in combination, but the nitrogen source is not limited thereto. As a phosphorous source, potassium dihydrogen phosphate, dipotassium hydrogenphosphate, and sodium-containing salts corresponding thereto may be used alone or in combination, but the phosphorous source is not limited thereto. Additionally, the medium may include essential growth-promoting materials, such as a metal salt (*e*.*g*., magnesium sulfate or iron sulfate), amino acids, and vitamins.

The MAAs produced by culturing may be secreted into the medium or remain in the cells.

As used herein, the term "medium" refers to a culture medium for culturing the microorganism of the present disclosure and/or a product obtained after culture. The medium is a concept, which includes both a form where the microorganism is included and a form where the microorganism is removed from the microorganism-containing cultured solution by centrifugation, filtration, *etc.*

In the step of recovering the MAAs produced in the culturing step of the present disclosure above, the desired MAAs can be collected from the culture solution using an appropriate method known in the art according to the culture method. For example, centrifugation, filtration, anion-exchange chromatography, crystallization, HPLC, *etc.* can be used, and the desired MAAs can be recovered from the cultured microorganism or medium using an appropriate method known in the art. The step of recovering the MAAs may further include a separation step and/or a purification step.

Still another aspect of the present disclosure provides a use of the microorganism of the present disclosure for the production of a mycosporine-like amino acid (MMA).

The "microorganism" and "mycosporine-like amino acid" are as described above.

### [Mode for Carrying Out the Invention]

Hereinafter, the present disclosure will be described in more detail with reference to the following Examples. However, these Examples are for illustrative purposes only and the scope of the disclosure is not limited by these Examples.

### <Preparation of E. coli-based recombinant microorganism producing MAAs and production of MAAs using the same >

### Example 1: Preparation of strain in which activity of 2-dehydro-3-deoxyphosphoheptonate aldolase is enhanced

To increase the MAAs-producing ability of a microorganism, *E. coli* strains in which the activity of 2-dehydro-3-deoxyphosphoheptonate aldolase is enhanced were prepared. Specifically, the *aroG* gene (2-dehydro-3-deoxyphosphoheptonate aldolase; SEQ ID NOS: 1 and 2) was further introduced based on an *E. coli* W3110 strain. The templates and primers used for the preparation of plasmids are shown in Table 1 below.

**[Table 1]**

| PCR Product | Template Used | Primers Used (Forward Direction, Reverse Direction) |
|---|---|---|
| fhuA arm 1 | W3110 genomic DNA | SEQ ID NO: 3, SEQ ID NO: 4 |
| fhuA arm 2 | W3110 genomic DNA | SEQ ID NO: 5, SEQ ID NO: 6 |
| Pn_*aroG* | W3110 genomic DNA | SEQ ID NO: 7, SEQ ID NO: 8 |
| Ptrc | pECCG117 Ptrc_GFP | SEQ ID NO: 9, SEQ ID NO: 10 |
| Pcj 1 (KR10-620092B) | pECCG117 Pcj1_GFP | SEQ ID NO: 11, SEQ ID NO: 12 |
| aroG | W3110 genomic DNA | SEQ ID NO: 13, SEQ ID NO: 8 |

After amplifying gene fragments through PCR using the templates and primers above, the amplified fragments were ligated to a pSKH vector in which the fragments of fhuA arm 1 and fhuA arm 2 genes were treated with BamH1-SpeI restriction enzymes, using the In-FusionR HD cloning kit (Clontech), and the prepared vector was named as pSKH-ΔfhuA. Due to the deletion of the *fhuA* gene, the phage infection of *E. coli* is inhibited.

A Pn *aroG* gene fragment was ligated to pSKH-ΔfhuA vector, which was cut with *Spe*1*-Eco*RV restriction enzymes, using the In-Fusion^{R} HD cloning kit (Clontech). After digesting *Ptrc* and *Pcj1* gene fragments, which are known as enhanced promoters, with *Spe*1*-Nde*1 restriction enzymes and digesting an *aroG* gene fragment with *Nde*1*-Eco*RV restriction enzymes, the *Ptrc* and *aroG* gene fragments or the *Pcj1* (Korean Patent No. 10-620092) and *aroG* gene fragments were respectively ligated to the pSKH-ΔfhuA vector which was cut with *Spe*1*-Eco*RV restriction enzymes, using the In-Fusion^{R} HD cloning kit (Clontech). The prepared vectors were named as pSKH-ΔfhuA-Pn-*aroG*, pSKH-ΔfhuA-Ptrc-*aroG*, and pSKH-ΔfhuA-Pcj1*-aroG*, respectively.

The above vectors were confirmed by sequencing with regard to the success of cloning and gene sequence in each vector, and then, were transformed into each wild-type *E*. *coli* W3110 strain by electroporation. Each transformed gene was introduced into the chromosome by primary recombination (cross-over), and the plasmid region was excised from the chromosome through secondary recombination (cross-over). For each of the transformed *E. coli* strains, in which the secondary recombination is completed, the introduction of the *aroG* gene was confirmed by PCR using primers of SEQ ID NOS: 14 (forward direction) and 8 (reverse direction).

### Example 2: Preparation of vector overexpressing microalgae-derived shinorine biosynthesis gene

*A. variabilis*-based gene cluster for shinorine biosynthesis consists of four genes (*Ava_ABCD*), which encode 2-demethyl 4-deoxygadusol synthase, O-methyltransferase, a C-N ligase, and non-ribosomal peptide synthetase. The gene cluster for shinorine biosynthesis was identified using the genomic DNA of *A*. *variabilis* ATCC29413. A vector including a shinorine biosynthesis gene derived from *A*. *variabilis* ATCC29413 was prepared using the pECCG117_Pcj1_GFP_terminator vector. The name of the shinorine biosynthesis gene expression vector and the respective template and primers for the preparation of the vector are shown in Table 2 below.

**[Table 2]**

| PCR Product | Template Used | Primers Used (Forward Direction, Reverse Direction) |
|---|---|---|
| Ava_ABCD | genomic DNA of *A. variabilis* ATCC29413 | SEQ ID NO: 15, SEQ ID NO: 16 |

Gene fragments were obtained using the template and primers above, and each gene fragment was ligated to a pECCG 117_Pcj1_GFP _terminator vector, which was treated with *Eco*RV-*Xba*I restriction enzymes, using the In-Fusion^{R} HD cloning kit (Clontech). The prepared vector was named as pECCG117_Pcj1_Ava _ABCD, and the success of cloning and the gene sequence of the vector were confirmed by sequencing. The nucleotide sequence of the *Ava_ABCD* gene is shown in SEQ ID NO: 17.

### Example 3: Evaluation of shinorine-producing ability of strain in which activity of 2-dehvdro-3-deoxyphosphoheptonate aldolase is enhanced

The pECCG117_Pcj1_Ava_ABCD plasmid prepared in Example 2 was introduced into each of the strains prepared in Example 1, in which an *aroG* gene is enhanced, and a wild-type W3110 strain by electroporation, and each transformed strain was plated on a LB solid medium. The strains were cultured in a 37°C incubator overnight, and then one platinum loop thereof was inoculated into a 25 mL titration medium [medium composition: glucose 40 g/L, KH₂PO₄ 0.3 g/L, K₂HPO₄ 0.6 g/L, (NH₄)₂SO₄ 15 g/L, MgSO₄ • 7H₂O 1 g/L, NaCl 2.5 g/L, sodium citrate 1.2 g/L, yeast extract 2.5 g/L, calcium carbonate 40 g/L: pH 7.0], which was incubated in a 37°C incubator at 200 rpm for 48 hours. The resulting strains were analyzed by HPLC (Waters Corp.) and the results are shown in Table 3 below.

**[Table 3]**

| Name of Strain | OD (600 nm) | Shinorine Conc. (mg/L) |
|---|---|---|
| W3110/pECCG117_Pcj1_Ava_ABCD | 19.4 | 348 |
| W3110ΔfhuA::Pn-*aroG*/ pECCG117 _Pcj1_Ava_ABCD | 19.6 | 418 |
| W3110ΔfhuA::Ptrc-*aroG*/ pECCG117_Pcj1_Ava_ABCD | 20.3 | 589 |
| W3110ΔfhuA::Pcj1*-aroG*/ pECCG117_Pcj1Ava ABCD | 20.5 | 674 |

As shown in Table 3 above, the concentration of shinorine produced in the strain (W3110ΔfhuA::Pn-*aroG*/pECCG117_Pcj1_Ava_ABCD), where the *aroG* gene is enhanced, was increased by about 20% compared to that of the control group. In particular, in the case of the strains where the *aroG* gene is enhanced by enhancing the promoter (*i.e.*, W3110ΔfhuA::Ptrc-*aroG*/pECCG117_Pcj1_Ava_ABCD and W3110ΔfhuA::Pcj1-*aroG*/pECCG117_Pcj1_Ava_ABCD), the shinorine concentration was increased by 69% and 94%, respectively.

### Example 4: Preparation of strain in which activity of phosphoenolpyruvate synthetase is enhanced

To increase the MAAs-producing ability of a microorganism, *E. coli* strains in which the activity of phosphoenolpyruvate synthetase is enhanced were prepared. Specifically, the *pps* gene (phosphoenolpyruvate synthetase; SEQ ID NOS: 18 and 19) was further introduced based on an *E. coli* W3110 strain. The templateand primers used for the preparation of the plasmid are shown in Table 4 below.

**[Table 4]**

| PCR Product | Template Used | Primers Used (Forward Direction, Reverse Direction) |
|---|---|---|
| *Pn-pps* | W3110 genomic DNA | SEQ ID NO: 20, SEQ ID NO: 21 |
| pps | W3110 genomic DNA | SEQ ID NO: 22, SEQ ID NO: 21 |

After amplifying gene fragments using the template and primers above, the Pn*_pps* gene fragment was ligated to pSKH-ΔfhuA vector, which was cut with *Spe*1*-Eco*RV restriction enzymes, using the In-Fusion^{R} HD cloning kit (Clontech). After digesting the *Ptrc* and *Pcj1* gene fragments prepared in Example 1 with *Spe*1*-Nde*1 restriction enzymes and digesting *the pps* gene fragment with *Nde*1*-Eco*RV restriction enzymes, the *Ptrc* and *pps* gene fragments or the *Pcj1* and *pps* gene fragments were respectively ligated to the pSKH-ΔfhuA vector, which was cut with *Spe*1*-Eco*RV restriction enzymes, using the In-Fusion^{R} HD cloning kit (Clontech). The prepared vectors were named as pSKH-ΔfhuA-Pn-*pps*, pSKH-ΔfhuA-Ptrc-*pps*, and pSKH-ΔfhuA-Pcj 1-*pps*, respectively.

The above vectors were confirmed by sequencing with regard to the success of cloning and gene sequence in each vector, and then, transformed into each wild-type *E. coli* W3110 strain by electroporation. Each transformed gene was introduced into the chromosome by primary recombination (cross-over), and the plasmid region was excised from the chromosome through secondary recombination (cross-over). For each of the transformed *E. coli* strains, in which the secondary recombination is completed, the introduction of the *pps* gene was confirmed by PCR using primers of SEQ ID NOS: 14 (forward direction) and 21 (reverse direction).

### Example 5: Evaluation of shinorine-producing ability of strain in which activity of phosphoenolpyruvate synthetase is enhanced

The pECCG117_Pcj1_Ava_ABCD plasmid prepared in Example 2 was introduced into each of the strains prepared in Example 4, in which a *pps* gene is introduced, and a wild-type W3110 strain by electroporation, and each transformed strain was plated on a LB solid medium. The strains were cultured in a 37°C incubator overnight, and one platinum loop thereof was inoculated into the 25 mL titration medium of Example 3, which was then incubated in a 37°C incubator at 200 rpm for 48 hours. The results are shown in Table 5 below.

**[Table 5]**

| Name of Strain | OD (600 nm) | Shinorine Conc. (mg/L) |
|---|---|---|
| W3110/pECCG117_Pcj1_Ava_ABCD | 19.4 | 348 |
| W3110ΔfhuA::Pn*-pps*/ pECCG117_Pcj1Ava_ABCD | 21.3 | 494 |
| W3110ΔfhuA:*:*Ptrc-*pps*/ pECCG117_Pcj1Ava_ABCD | 20.8 | 511 |
| W3110ΔfhuA::Pcj1*-pps*/ pECCG117_Pcj1Ava_ABCD | 21.4 | 556 |

As shown in Table 5 above, the concentration of shinorine produced in the strain where a *pps* gene is enhanced was increased by 41%, and in the case where its activity is enhanced by replacing with a strong promoter, the shinorine concentration was increased up to 60% compared to that of the control group.

### Example 6: Preparation of strain in which activity of transketolase I/II is enhanced

To increase the MAAs-producing ability of a microorganism, *E. coli* strains in which the activity of transketolase is enhanced were prepared. Specifically, based on an *E. coli* W3110 strain, a *tktA* gene (transketolase; SEQ ID NOS: 23 and 24) was introduced thereinto. The template and primers used in the preparation of plasmids are shown in Table 6 below.

**[Table 6]**

| PCR Product | Template Used | Primers Used (Forward Direction, Reverse Direction) |
|---|---|---|
| *Pn-tktA* | W3110 genomic DNA | SEQ ID NO: 25, SEQ ID NO: 26 |
| *tktA* | W3110 genomic DNA | SEQ ID NO: 27, SEQ ID NO: 26 |

After amplifying gene fragments through PCR using the template and primers above, a Pn*_tktA* gene fragment was ligated to pSKH-ΔfhuA vector, which was cut with *Spe*1*-Eco*RV restriction enzymes, using the In-Fusion^{R} HD cloning kit (Clontech). After digesting the *Ptrc* and *Pcj1* gene fragments prepared in Example 1 with *Spe*1, *Nde*1 restriction enzymes and digesting a *tktA* gene fragment with *Nde*1*-Eco*RV restriction enzymes, the *Ptrc* and *tktA* gene fragments or the *Pcj1* and *tktA* gene fragments were respectively ligated to pSKH-ΔfhuA vector, which was cut with *Spe*1*-Eco*RV restriction enzymes, using the In-Fusion^{R} HD cloning kit (Clontech). The prepared vectors were named as pSKH-ΔfhuA-Pn-*tktA*, pSKH-ΔfhuA-Ptrc-*tktA*, and pSKH-ΔfhuA-Pcj 1*-tktA*, respectively.

The above vectors were confirmed by sequencing with regard to the success of cloning and gene sequence in each vector, and then, transformed into each wild-type *E. coli* W3110 strain by electroporation. Each transformed gene was introduced into the chromosome by primary recombination (cross-over), and the plasmid region was excised from the chromosome through secondary recombination (cross-over). For each of the transformed *E. coli* strains, in which the secondary recombination is completed, the introduction of the *tktA* gene was confirmed by PCR using primers of SEQ ID NOS: 14 (forward direction) and 26 (reverse direction).

### Example 7: Evaluation of shinorine-producing ability in strain in which activity of transketolase is enhanced

The pECCG117_Pcj1_Ava_ABCD plasmid prepared in Example 2 was introduced into each of the strains prepared in Example 6, in which a *tktA* gene is introduced, and a wild-type W3110 strain by electroporation, and each transformed strain was plated on a LB solid medium. The strains were cultured in a 37°C incubator overnight, and one platinum loop of the overnight culture of each strain was inoculated into the 25 mL titration medium of Example 3, which was then incubated in a 37°C incubator at 200 rpm for 48 hours. The results are shown in Table 7 below.

**[Table 7]**

| Name of Strain | OD (600 nm) | Shinorine Conc. (mg/L) |
|---|---|---|
| W3110/pECCG117_Pcj1_Ava_ABCD | 19.4 | 348 |
| W3110ΔfhuA:*:*Pn*-tktA*/ pECCG117_Pcj1_Ava_ABCD | 19.5 | 364 |
| W3110ΔfhuA::Ptrc-*tktA*/ pECCG117_Pcj1_Ava_ABCD | 19.3 | 447 |
| W3110ΔfhuA::Pcj1*-tktA*/ pECCG117_Pcj1Ava ABCD | 19.5 | 461 |

As shown in Table 7 above, the concentration of shinorine produced in the strain where the *tktA* gene is enhanced was increased by 4.5%, and in the case where its activity is enhanced by replacing with a strong promoter, the shinorine concentration was increased up to 32% compared to that of the control group.

### Example 8: Preparation of strain in which activities of 2-dehydro-3-deoxyphosphoheptonate aldolase/phosphoenolpyruvate synthetase/transketolase are enhanced

To increase the MAAs-producing ability of a microorganism, *E. coli* strains in which the activity of each of 2-dehydro-3-deoxyphosphoheptonate aldolase/phosphoenolpyruvate synthetase/transketolase is enhanced were prepared. Specifically, based on an *E. coli* W3110 strain, each of an *aroG* gene, a *pps* gene, and a *tktA* gene was further introduced thereinto. The templates and primers used in the preparation of plasmids are shown in Table 8 below.

**[Table 8]**

| PCR Product | Template Used | Primers Used (Forward Direction, Reverse Direction) |
|---|---|---|
| Pcj1*-aroG* | pSKH-ΔfhuA-Pcj1*-aroG* | SEQ ID NO: 11, SEQ ID NO: 28 |
| Pcj1-*pps* | pSKH-ΔfhuA-Pcj1*-pps* | SEQ ID NO: 29, SEQ ID NO: 30 |
| Pcj1*-tktA* | pSKH-ΔfhuA-Pcj1*-tktA* | SEQ ID NO: 31, SEQ ID NO: 26 |

After amplifying gene fragments using the templates and primers above, each gene fragment thereof was ligated to the pSKH-ΔfhuA vector, which was cut with *Spe*1*-Eco*RV restriction enzymes, using the In-Fusion^{R} HD cloning kit (Clontech). The prepared vector was named as pSKH-ΔfhuA-Pcj1*-aroG-*Pcj1*ppsA*-Pcj1*-tktA.*

The above vector was confirmed by sequencing with regard to the success of cloning and gene sequences in the vector, and then, transformed into a wild-type *E. coli* W3110 strain by electroporation. The transformed genes were introduced into the chromosome by primary recombination (cross-over), and the plasmid region was excised from the chromosome through secondary recombination (cross-over). For the transformed *E. coli* strains, in which the secondary recombination is completed, the introduction of the *aroG*, *pps*, and *tktA* genes was confirmed by PCR using primers of SEQ ID NOS: 14 (forward direction) and 26 (reverse direction).

### Example 9: Evaluation of shinorine-producing ability in strain in which activities of 2-dehydro-3-deoxyphosphoheptonate aldolase, phosphoenolpyruvate synthetase, and transketolase are enhanced

The pECCG117_Pcj1_Ava_ABCD plasmid prepared in Example 2 was introduced into each of the strain prepared in Example 8, in which *aroG*, *pps*, and *tktA* genes are introduced, and a wild-type W3110 strain by electroporation, and each transformed strain was plated on a LB solid medium. The strains were cultured in a 37°C incubator overnight, and one platinum loop of the overnight culture of each strain was inoculated into the 25 mL titration medium of Example 3, which was then incubated in a 37°C incubator at 200 rpm for 48 hours. The results are shown in Table 9 below.

**[Table 9]**

| Name of Strain | OD (600 nm) | Shinorine Conc. (mg/L) |
|---|---|---|
| W3110/pECCG117_Pcj1_Ava_ABCD | 19.4 | 348 |
| W3110ΔfhuA::Pcj1*-aroG-*Pcj1-*pps*-Pcj1*-tktA*/ pECCG117_Pcj1_Ava_ABCD | 18.2 | 1,279 |

As shown in Table 9 above, the concentration of shinorine produced in the strain where the three kinds of genes (*i.e.*, *aroG*, *pps*, and *tktA*) are combined and enhanced was increased by 267% compared to that of the control group. This is an unexpected result showing an improvement beyond expectation compared to the sum of the maximum increases obtained by replacing the promoter of each gene with a strong promoter. That is, it was confirmed that when the three genes (*i.e.*, *aroG*, *pps*, and *tktA*) are combined, it is possible to produce shinorine at a higher concentration.

### Example 10: Preparation of strain in which activity of 3-dehydroquinate dehydratase is inactivated

To increase the MAAs-producing ability of a microorganism, *E. coli* strains in which the activity of 3-dehydroquinate dehydratase (*aroD*) is inactivated were prepared.

Specifically, a chloramphenicol resistance gene of a pKD3 plasmid was used as a gene insertion marker, and an *aroD*-deletion cassette, which includes part of an *aroD* gene and the chloramphenicol resistance gene of a pKD3 plasmid, was prepared by PCR using the primers of SEQ ID NO: 32 (forward direction) and 33 (reverse direction). Competent cells were prepared by transforming a wild-type *E. coli* W3110 strain and the strain prepared in Example 8, in which the *aroG*, *pps*, and *tktA* genes are introduced, with a pKD46 plasmid including a lambda red recombinase gene, followed by inducing the expression of the corresponding gene using arabinose. After introducing the *aroD*-deletion cassette into the competent cells by electroporation, the resulting competent cells were plated on a LB solid medium containing 30 mg/L of chloramphenicol. The thus-obtained strain was subjected to PCR using the primers of SEQ ID NOS: 34 (forward direction) and 35 (reverse direction), and the *aroD* gene deletion was confirmed by observing the 1,300 bp amplified fragment.

### Example 11: Evaluation of shinorine-producing ability of strain in which 3-dehydroquinate dehydratase is inactivated

The pECCG117 Pcj1_Ava_ABCD plasmid prepared in Example 2 was introduced into the strain prepared in Example 10, in which an *aroD* gene is deleted, by electroporation, and the resulting strain was plated on a LB solid medium. The strain was cultured in a 37°C incubator overnight, and one platinum loop thereof was inoculated into the 25 mL titration medium of Example 3, which was incubated in a 37°C incubator at 200 rpm for 48 hours. The results are shown in Table 10 below.

**[Table 10]**

| Name of Strain | OD (600 nm) | Shinorine Conc. (mg/L) |
|---|---|---|
| W3110ΔfhuA::Pcj1*-aroG-*Pcj1*ppsA*-Pcj1*-tktA*/ pECCG117_PCJ1_Ava_ABCD | 18.6 | 1,248 |
| W3110Δ*aroD*ΔfhuA::Pcj1*-aroG-*Pcj1-*ppsA*-Pcj1*-tktA*/ pECCG117_PCJ1_Ava_ABCD | 17.3 | 2,077 |

As shown in Table 10 above, the concentration of shinorine produced in the strain, where the *aroD* gene is further deleted, was increased by 66% compared to that of the strain producing shinorine where the *aroG*, *pps*, and *tktA* genes are enhanced. The W3110ΔfhuA::Pcj1*-aroG*-Pcj1-*ppsA*-Pcj1-*tktA*/pECCG117_PCJ1_Ava_ABCD strain, which is a strain in which the *aroG*, *pps*, and *tktA* genes are enhanced, was named as CB06-0020, and deposited under the Budapest Treaty on February 14, 2018, in the Korean Culture Center of Microorganisms (KCCM) and assigned Accession No. KCCM12224P.

### <Preparation of Corynebacterium glutamicum-based recombinant microorganism producing MAAs and production of MAAs using the same>

### Example 12: Preparation of vector in which activity of 2-dehydro-3-deoxyphosphoheptonate aldolase is enhanced and evaluation of shinorine-producing ability of the same

To increase the MAAs-producing ability of a microorganism, *E. coli* strains in which the activity of 2-dehydro-3-deoxyphosphoheptonate aldolase is enhanced were prepared. Specifically, the *aroG* gene (2-dehydro-3-deoxyphosphoheptonate aldolase; SEQ ID NOS: 36 and 37) was further introduced based on a *Corynebacterium glutamicum* ATCC13032 strain. The template and primers used for the preparation of the plasmid are shown in Table 11 below.

**[Table 11]**

| PCR Product | Template Used | Primers Used (Forward Direction, Reverse Direction) |
|---|---|---|
| Pn-cgl *aroG* | c.gl 13032 genomic DNA | SEQ ID NO: 38, SEQ ID NO: 39 |
| Pcj7-cgl *aroG* | c.gl 13032 genomic DNA | SEQ ID NO: 40, SEQ ID NO: 41 |

After obtaining gene fragments using the template and primers above, each gene fragment was ligated to the pECCG 117 and pECCG 117_Pcj7_GFP_terminator (Korean Patent No. 10-620092, p117-cj7-gfp) vectors, which were treated with *Eco*RV/*Xba*I restriction enzymes, using the In-Fusion^{R} HD cloning kit (Clontech). The prepared vectors were named as pECCG117_Pn_cgl *aroG* and pECCG117_Pcj7_cgl *aroG*, respectively. The above vectors were confirmed by sequencing with regard to the success of cloning and gene sequence in each vector.

First, since a microorganism of the genus *Corynebacterium* cannot produce shinorine, a strain in which shinorine biosynthetic pathway is introduced was prepared. Specifically, the *Ava_ABCD* gene was subjected to PCR using the pECCG117_Ptrc_Ava_ABCD as a template along with a primer pair of SEQ ID NOS: 42 (forward direction) and 43 (reverse direction). The pDZTn_Ava_ABCD was prepared by ligating an about 7 kb PCR fragment to the pDZTn vector (WO 2009-125992A), which is treated with an *Nde*I restriction enzyme, using the In-Fusion^{R} HD cloning kit (Clontech). Then, a fragment of the O2 promoter (KR Patent No. 10-1632642) was subjected to PCR using a primer pair of SEQ ID NOS: 44 (forward direction) and 45 (reverse direction), and ligated to the pDZTn_Ava_ABCD, which is treated with an *Nde*I restriction enzyme, using the In-Fusion^{R} HD cloning kit (Clontech), thereby preparing pDZTn_PO2_Ava_ABCD.

The recombinant plasmid was transformed into the wild-type ATCC13032 by electroporation (van der Rest *et al.* 1999), and the plasmid was introduced into the chromosome by primary recombination (cross-over), and the plasmid region was excised from the chromosome through secondary recombination (cross-over).

For each of the transformed *Corynebacterium glutamicum* strains, in which the secondary recombination is completed, the introduction of the *Ava_ABCD* gene was confirmed by PCR using a gene-specific primer pair of SEQ ID NOS: 42 (forward direction) and 43 (reverse direction). The prepared strain was named as *Corynebacterium glutamicum* 13032 ΔN1021PO2_Ava_ABCD.

The pECCG117_Pn_cgl *aroG* and pECCG117_Pcj7_cgl *aroG* vectors were each transformed into the *Corynebacterium glutamicum* 13032 ΔN1021_PO2_Ava_ABCD strain by electroporation.

The strains prepared above and the control group *Corynebacterium glutamicum* ATCC13032 (c.gl 13032) were cultured overnight in a BHIS solid medium containing kanamycin, and one platinum loop thereof were inoculated into a 25 mL titration medium [medium composition: glucose 40 g/L, KH₂PO₄ 1 g/L, (NH₄)₂SO₄ 10 g/L, MgSO₄7H₂O 5g/L, NaCl 5 g/L, yeast extract 5 g/L, calcium carbonate 30 g/L: pH 7.0], which was incubated in a 37°C incubator at 200 rpm for 48 hours. The results are shown in Table 12 below.

**[Table 12]**

| Name of Strain | OD (600 nm) | Shinorine Conc. (mg/L) |
|---|---|---|
| c.gl 13032 | 72.1 | - |
| c.gl 13032ΔN1021_PO2_Ava_ABCD | 71.5 | 180 |
| c.gl 13032ΔN1021_PO2_Ava ABCD/ pECCG117_Pn_cgl *aroG* | 69.6 | 250 |
| c.gl 13032ΔN1021_PO2_Ava ABCD/ pECCG117_Pcj7_cgl *aroG* | 71.9 | 324 |

As shown in Table 12 above, when the *aroG* expression level was increased in the strain containing the shinorine biosynthesis gene, the concentration of shinorine was increased by 39%. In particular, when the promoter was enhanced, it was confirmed that the shinorine concentration could be improved up to 80%.

### Example 13: Preparation of vector in which activities of phosphoenolpyruvate synthetase/transketolase are enhanced and evaluation of shinorine-producing ability of the same

To increase the MAAs-producing ability of a microorganism, *Corynebacterium glutamicum* strains in which the activity of *tkt* or *pps* is enhanced were prepared. Specifically, the *tkt* (transketolase; SEQ ID NO: 95 and 96) or *pps* (phosphoenolpyruvate synthetase; SEQ ID NOS: 97 and 98) was further introduced based on a *Corynebacterium glutamicum* ATCC13032 strain. The template and primers used for the preparation of the plasmid are shown in Table 13 below.

**[Table 13]**

| PCR Product | Template Used | Primers Used (Forward Direction, Reverse Direction) |
|---|---|---|
| Pn-cgl *tkt* | c.gl 13032 genomic DNA | SEQ ID NO: 46, SEQ ID NO: 47 |
| Pcj7-cgl *tkt* | c.gl 13032 genomic DNA | SEQ ID NO: 48, SEQ ID NO: 49 |
| Ptrc-cgl *pps* | c.gl 13032 genomic DNA | SEQ ID NO: 50, SEQ ID NO: 51 |
| Pcj7-cgl *pps* | c.gl 13032 genomic DNA | SEQ ID NO: 52, SEQ ID NO: 51 |

Vectors were prepared by ligating the gene fragments, which were obtained through PCR technology in which the template was matched to combination of the primers, to the pECCG117, pECCG117_Ptrc_GFP_terminator and pECCG 117_Pcj7_GFP_terminator vectors, which were treated with *Eco*RV/*Xba*I restriction enzymes, using the In-Fusion^{R} HD cloning kit (Clontech). The prepared vectors were named as pECCG117-Pn-tkt/pECCG1 17-Pcj7-tkt and pECCG117-Ptrc-pps/pECCG117-Pcj7-pps, respectively. The above vectors were confirmed by sequencing with regard to the success of cloning and gene sequence in each vector and then transformed into a *Corynebacterium glutamicum* 13032 ΔN1021_PO2_Ava_ABCD strain by electroporation. Each strain was cultured in a kanamycin-containing BHIS solid medium overnight and a platinum loop thereof was inoculated into the 25 mL titration medium of Example 12, which was incubated in a 37°C incubator at 200 rpm for 48 hours. The results are shown in Table 14 below.

**[Table 14]**

| Name of Strain | OD (600 nm) | Shinorine Conc. (mg/L) |
|---|---|---|
| c.gl 13032ΔN1021_PO2_Ava_ABCD | 73 | 175 |
| c.gl 13032ΔN1021_PO2_Ava ABCD/ pECCG117 _Pn_cgl *tkt* | 72 | 211 |
| c.gl 13032ΔN1021_PO2_Ava ABCD/ pECCG117-Pcj7-cgl *tkt* | 71.5 | 275 |
| c.gl 13032ΔN1021_PO2_Ava ABCD/ pECCG117-Ptrc-cgl *pps* | 70.9 | 298 |
| c.gl 13032ΔN1021_PO2_Ava ABCD/ pECCG117-Pcj7-cgl *pps* | 70.2 | 302 |

As shown in Table 14 above, it was confirmed that when the *tkt* gene or *pps* gene was enhanced, the shinorine production was improved up to 57% or 72%, respectively.

### Example 14: Preparation of strain in which activities of 2-dehydro-3-deoxyphos phoheptonate aldolase/phosphoenolpyruvate synthetase/transketolase are enhanced and evaluation of the same

In order to increase the MAAs-producing ability of a microorganism, *E. coli* strains in which the activities of *aroG*, *pps*, and *tkt* genes are enhanced were prepared, and to confirm the presence of a higher amount of MAAs production, the 3-dehydroquinate dehydratase (*aroD*) was further inactivated. Specifically, to enhance *aroG*, *pps*, and *tkt* genes, a pDZ-ΔaroD-Pcj7-*aroG-*Pcj7-*pps*-Pcj7-*tktA* plasmid was prepared. The templates and primers used for the preparation of the pDZ-ΔaroD-Pcil-*aroG*-Pcj7*-pps-*Pcj7*-tktA* plasmid are shown in Table 15 below.

**[Table 15]**

| PCR Product | Template Used | Primers Used (Forward Direction, Reverse Direction) |
|---|---|---|
| Pcj7*-aroG* | pECCG117_Pcj7_cgl *aroG* | SEQ ID NO: 53, SEQ ID NO: 54 |
| Pcj7*-tkt* | pECCG117-Pcj7-cgl *tkt* | SEQ ID NO: 55, SEQ ID NO: 56 |
| *Pcj7-pps* | pECCG117-Pcj7-cgl *pps* | SEQ ID NO: 57, SEQ ID NO: 58 |

First, in order to prepare a strain in which the *aroD* gene (SEQ ID NOS: 89 and 90) of *Corynebacterium glutamicum* is deleted, a pDZ-ΔaroD plasmid in which the open reading frame of the *aroD* gene is internally deleted was prepared. The internal gene deletion of the pDZ-ΔaroD plasmid was achieved by performing a cross-PCR using the genomic DNA of the *Corynebacterium glutamicum* ATCC 13032 strain as a template along with SEQ ID NOS: 91 and 92, and SEQ ID NOS: 93 and 94 as a pair of forward and reverse primers, followed by introducing the resulting gene fragments into the pDZ vector.

Then, each gene fragment of the *aroG, pps,* and *tkt* genes was amplified through PCR using the templates and primers shown in Table 15 above, and was then introduced into the pDZ-ΔaroD vector, cleaved with a *Spe*I restriction enzyme, respectively. The above two kinds of vectors were confirmed by sequencing with regard to the success of cloning and gene sequence in each vector, and then, transformed into a *Corynebacterium glutamicum* 13032ΔN1021_PO2_Ava_ABCD strain by electroporation. Each strain was cultured in a kanamycin-containing BHIS solid medium overnight and a platinum loop of overnight culture of each strain was inoculated into the 25 mL titration medium of Example 12, which was incubated in a 37°C incubator at 200 rpm for 48 hours. The results are shown in Table 16 below.

**[Table 16]**

| Name of Strain | OD (600 nm) | Shinorine Conc. (mg/L) |
|---|---|---|
| c.gl 13032ΔN1021_PO2_Ava_ABCD_Δ*aroD* | 73 | 425 |
| c.gl 13032ΔN1021_PO2_Ava_ABCD_Δa*roD*, Pcj7_*aroG*, Pcj7*_tkt,* Pcj7*-pps* | 69 | 531 |

As shown in Table 16 above, the concentration of shinorine produced in the strain where the three kinds of genes (*aroG*, *pps*, and *tktA*) are enhanced was increased by about 25%. It was confirmed that even in the strain, where the shinorine-producing ability was increased through the deletion of the *aroD* gene, shinorine could be produced at a high concentration by the combination of the three kinds of genes. Additionally, it may be interpreted that when the *aroD* gene is further inactivated in the strain where the three kinds of genes are combined, shinorine can be produced at an even higher concentration.

### <Preparation of yeast-based recombinant microorganism producing MAAs and production of MAAs using the same >

### Example 15: Preparation of Saccharomyces cerevisiae (S. cerevisiae) strain producing shinorine

In order to use a *Saccharomyces cerevisiae* (*S. cerevisiae*) strain as a strain producing shinorine, a shinorine biosynthesis gene derived from A. *variabilis* ATCC29413 was introduced into a vector for yeast expression. *Ava*_*A* and *Ava_B* genes were inserted into the pRS-413 vector using the GPD promoter. Specifically, pGPD-Ava_A and pGPD-Ava_B regions were ligated by overlapping PCR. The vectors and PCR products were treated with *Bam*HI and *Sai*I restriction enzymes, and then ligated using T4 ligase to prepare the pRS-413-pGPD-Ava_A-pGPD-Ava_B vector.

Then, *Ava_A* and *Ava_B* genes were inserted into the pRS-414 vector using the GPD promoter. Specifically, pGPD-Ava_C and pGPD-Ava_D regions were ligated by overlapping PCR, and then, the vectors and PCR products were treated with *Bam*HI and *Sai*I and ligated using T4 ligase to prepare the pRS-414-pGPD-Ava_C-pGPD-Ava_D vector. The primers and template DNAs used for the preparation of the vector are shown in Table 17 below.

**[Table 17]**

| PCR Product | Template Used | SEQ ID NO (Forward Direction, Reverse Direction) |
|---|---|---|
| pGPD | *S. cerevisiae* gDNA | SEQ ID NO: 59, SEQ ID NO: 60 |
| Ava_A | *A. variabilis* ATCC29413 gDNA | SEQ ID NO: 61, SEQ ID NO: 62 |
| pGPD-Ava _A | PCR product | SEQ ID NO: 59, SEQ ID NO: 62 |
| pGPD | *S. cerevisiae* gDNA | SEQ ID NO: 63, SEQ ID NO: 64 |
| Ava_B | *A. variabilis* ATCC29413 gDNA | SEQ ID NO: 65, SEQ ID NO: 66 |
| pGPD-Ava _B | PCR product | SEQ ID NO: 63, SEQ ID NO: 66 |
| pGPD-Ava_A-pGPD-Ava_B | PCR product | SEQ ID NO: 59, SEQ ID NO: 66 |
| pGPD | *S. cerevisiae* gDNA | SEQ ID NO: 67, SEQ ID NO: 68 |
| Ava_C | *A. variabilis* ATCC29413 gDNA | SEQ ID NO: 69, SEQ ID NO: 70 |
| pGPD-Ava_C | PCR product | SEQ ID NO: 67, SEQ ID NO: 70 |
| pGPD | *S. cerevisiae* gDNA | SEQ ID NO: 71, SEQ ID NO: 72 |
| Ava_D | *A. variabilis* ATCC29413 gDNA | SEQ ID NO: 73, SEQ ID NO: 74 |
| pGPD-Ava_D | PCR product | SEQ ID NO: 71, SEQ ID NO: 74 |
| pGPD-Ava_C-pGPD-Ava _D | PCR product | SEQ ID NO: 67, SEQ ID NO: 74 |

The pRS-413-pGPD-Ava_A-pGPD-Ava_B vector and the pRS-414-pGPD-Ava_C-pGPD-Ava_D vector were introduced into *Saccharomyces cerevisiae* CEN.PK-1D (*S. cerevisiae* CEN.PK-1D) strain through lithium acetate transformation, and then the presence of shinorine production was confirmed. The strain was plated on a synthetic complete (SC) solid medium, in which Trp and His (*i.e.*, auxotrophic markers) are excluded, and cultured overnight in a 30°C incubator. One platinum loop of the strain cultured overnight in the synthetic complete (SC) solid medium, in which Trp and His are excluded, was inoculated into the 25 mL titration medium of Table 18, and then was cultured in a 30°C incubator at 150 rpm for 24 hours. The results are shown in Table 19 below.

**[Table 18]**

| Composition | Conc. Used (g/L) |
|---|---|
| Yeast Nitrogen Base (YNB) without Amino Acids | 6.7 |
| Amino Acid Mixtures (without Leucine, Tryptophan, Histidine, Uracil) | 2 |
| Glucose | 20 |

**[Table 19]**

| Name of Strain | 24 Hour | | Shinorine Conc. (mg/L) |
|---|---|---|---|
| | OD (600nm) | Residual Saccharide | |
| CEN.PK-1D WT + pRS413-pGPD-Ava_A-pGPD-Ava_B + pRS414-pGPD-Ava_C-pGPD-Ava_D | 11.2 | 0 | 331 |

As a result of the experiment, it was confirmed that the wild-type *Saccharomyces cerevisiae* strain, not producing shinorine, produced 331 mg/L of shinorine due to the introduction of the shinorine biosynthesis gene.

### Example 16: Increase of amount of shinorine production through enhancement of TKL1 (transketolase) of Saccharomyces cerevisiae

In order to increase the MAAs-producing ability, a *Saccharomyces cerevisiae* strain in which the activity of *TKL1* is enhanced was prepared. For this purpose, the expression of the *TKL1* gene was enhanced by cloning the TKL1 gene (SEQ ID NOS: 110 and 123) into pRS-415-pGPD, pRS-415-pADH, and pRS-415-pTEF vectors

The GPD promoter included in the pRS-415-pGPD vector is the promoter of glyceraldehyde-3-phosphate dehydrogenase (*GAPDH*) and isozyme 3 (*TDH3*) genes, and it includes a sequence of -674 bp to -1 bp from the initiation codon of the ORF of the *THD3* gene.

The ADH promoter included in the pRS-415-pADH vector is the promoter of alcohol dehydrogenase (*ADH1*) gene, and it includes a sequence of -1,500 bp to -1 bp from the initiation codon of the ORF of the *ADH1* gene*.*

The TEF promoter included in the pRS-415-pTEF vector is the promoter of translational elongation factor EF-1 alpha (*TEF1*) gene, and it includes a sequence of -500 bp to -1 bp from the initiation codon of the ORF of the *TEF1* gene.

Specifically, the *TEF1*gene was subjected to PCR using the primers of Table 20 below, and the PCR products and pRS-415-pGPD, pRS-415-pADH, and pRS-415-pTEF vectors were treated with *BamHI* and *Sal*I restriction enzymes, and then ligated using a T4 ligase to prepare the pRS-415-pGPD-*TKL1*, pRS-415-pADH-*TKL1*, and pRS-415-pTEF-*TKL1* vectors.

**[Table 20]**

| PCR Product | Template Used | SEQ ID NO (Forward Direction, Reverse Direction) |
|---|---|---|
| *TKL1* | *S. cerevisiae* gDNA | SEQ ID NO: 75, SEQ ID NO: 76 |

Then, the plasmid for shinorine biosynthesis prepared in Example 15 was introduced into *Saccharomyces cerevisiae* CEN.PK-1D strain along with the pRS-415-pGPD-*TKL1*, pRS-415-pADH-*TKL1*, and pRS-415-pTEF-*TKL1*, and each resulting strain was plated on a synthetic complete (SC) solid medium, in which Trp, Ura, and His are excluded, and cultured overnight in a 30°C incubator. One platinum loop of the strain cultured overnight in the synthetic complete (SC) solid medium, in which Trp, Ura, and His are excluded, was inoculated into a 25 mL titration medium, and then was cultured in a 30°C incubator at 150 rpm for 24 hours. The results are shown in Table 21 below.

**[Table 21]**

| Name of Strain | 24 Hour | | Shinorine Conc. (mg/L) |
|---|---|---|---|
| | OD (600 nm) | Residual Saccharide | |
| CEN.PK-1D WT + pRS413-pGPD-Ava A-pGPD-Ava B, pRS414-pGPD-Ava C-pGPD-Ava D | 11.2 | 0 | 327 |
| CEN.PK-1D(pADH-*TKL1*) + pRS413-pGPD-Ava A-pGPD-Ava B, pRS414-pGPD-Ava C-pGPD-Ava D | 13 | 0 | 370 |
| CEN.PK-1D(pTEF-*TKL1*) + pRS413-pGPD-Ava A-pGPD-Ava B, pRS414-pGPD-Ava C-pGPD-Ava D | 12.1 | 0 | 420 |
| CEN.PK-1D(pGPD-*TKL1*) + pRS413-pGPD-Ava A-pGPD-Ava B, pRS414-pGPD-Ava C-pGPD-Ava D | 12.5 | 0 | 610 |

As shown in Table 21 above, it was confirmed that the amount of shinorine production was increased in the strain, in which the *TKL1* gene expression is enhanced using the GPD promoter, compared to that of the WT strain. Additionally, it was further confirmed that as the strength of the promoter increased (*i.e.*, pGPD> pTEF> pADH), the amount of shinorine production was increased.

### Example 17: Increase of amount of shinorine production through enhancement of ARO4 (3-deoxy-D-arabino-heptulosonate-7-phosphate (DAHP) synthase) of Saccharomyces cerevisiae

In order to increase the MAAs-producing ability, *Saccharomyces cerevisiae* strains in which the activity of *ARO4* is enhanced were prepared. For this purpose, a strategy was employed in which the expression of the *ARO4* gene was enhanced by cloning the *ARO4* gene (SEQ ID NOS: 111 and 124) into pRS-415-pGPD, pRS-415-pADH, and pRS-415-pTEF vectors. Specifically, the *ARO4* gene was subjected to PCR using the primers of Table 22 below, and the PCR products of *ARO4* and pRS-415-pGPD, pRS-415-pADH, and pRS-415-pTEF vectors were treated with *Bam*HI and *Sal*I restriction enzymes, and then ligated using T4 ligase to prepare the pRS-415-pGPD-*ARO4*, pRS-415-pADH-*ARO4*, and pRS-415-pTEF-*ARO4* vectors.

**[Table 22]**

| PCR Product | Template Used | SEQ ID NO (Forward Direction, Reverse Direction) |
|---|---|---|
| *ARO4* | *S. cerevisiae* gDNA | SEQ ID NO: 77, SEQ ID NO: 78 |

Then, the plasmid for shinorine biosynthesis prepared in Example 15 was introduced into *Saccharomyces cerevisiae* CEN.PK-1D strain along with the pRS-415-pGPD-*ARO4*, pRS-415-pADH-*ARO4*, and pRS-415-pTEF-*ARO4*, and each resulting strain was plated on a synthetic complete (SC) solid medium, in which Trp, Ura, and His are excluded, and cultured overnight in a 30°C incubator. One platinum loop of the strain cultured overnight in the synthetic complete (SC) solid medium, in which Trp, Ura, and His are excluded, was inoculated into a 25 mL titration medium, and then was cultured in a 30°C incubator at 150 rpm for 24 hours. The results are shown in Table 23 below.

**[Table 23]**

| Name of Strain | 24 Hour | | Shinorine Conc. (mg/L) |
|---|---|---|---|
| | OD (600nm) | Residual Saccharide | |
| CEN.PK-1D WT + pRS413-pGPD-Ava_A-pGPD-Ava_B, pRS414-pGPD-Ava C-pGPD-Ava D | 11.2 | 0 | 310 |
| CEN.PK-1D(pADH-*ARO4*) + pRS413-pGPD-Ava_A-pGPD-Ava_B, pRS414-pGPD-Ava C-pGPD-Ava D | 13 | 0 | 415 |
| CEN.PK-1D(pTEF-*ARO4*) + pRS413-pGPD-Ava_A-pGPD-Ava_B, pRS414-pGPD-Ava C-pGPD-Ava D | 12.1 | 0 | 610 |
| CEN.PK-1D(pGPD-*ARO4*) + pRS413-pGPD-Ava A-pGPD-Ava B, pRS414-pGPD-Ava C-pGPD-Ava D | 12.5 | 0 | 890 |

As shown in Table 23 above, it was confirmed that the amount of shinorine production was increased by 187% in the strain, in which the *ARO4* gene expression is enhanced using the GPD promoter, compared to that of the WT strain.

### Example 18: Increase of amount of shinorine production through enhancement of phosphoenolpyruvate synthetase (pps) of Saccharomyces cerevisiae

In order to increase the MAAs-producing ability, *Saccharomyces cerevisiae* strains in which the activity of *pps* is enhanced were prepared. For this purpose, a strategy was employed in which the expression of the pps gene was enhanced by cloning *the pps* gene into pRS-415-pGPD, pRS-415-pADH, and pRS-415-pTEF vectors and the expression of the *pps* gene was enhanced.

Specifically, *the pps* gene was subjected to PCR using the primers of Table 24 below, and the PCR products of *pps* and pRS-415-pGPD, pRS-415-pADH, and pRS-415-pTEF vectors were treated with *Bam*HI and *Sal*I restriction enzymes, and then ligated using T4 ligase to prepare the pRS-415-pGPD-*pps*, pRS-415-pADH-*pps*, and pRS-415-pTEF-*pps* vectors.

**[Table 24]**

| PCR Product | Template Used | SEQ ID NO (Forward Direction, Reverse Direction) |
|---|---|---|
| *pps* | *E. coli* MG1655 gDNA | SEQ ID NO: 79, SEQ ID NO: 80 |

Then, the plasmid for shinorine biosynthesis prepared in Example 15 was introduced into *Saccharomyces cerevisiae* CEN.PK-1D strain along with the pRS-415-pGPD-*pps*, pRS-415-pADH-*pps*, and pRS-415-pTEF-*pps*, and each resulting strain was plated on a synthetic complete (SC) solid medium, in which Trp, Ura, and His are excluded, and cultured overnight in a 30°C incubator. One platinum loop of the strain cultured overnight in the synthetic complete (SC) solid medium, in which Trp, Ura, and His are excluded, was inoculated into a 25 mL titration medium, and then was cultured in a 30°C incubator at 150 rpm for 24 hours. The results are shown in Table 25 below.

**[Table 25]**

| Name of Strain | 24 Hour | | Shinorine Conc. (mg/L) |
|---|---|---|---|
| | OD (600nm) | Residual Saccharide | |
| CEN.PK-1D WT + pRS413-pGPD-Ava A-pGPD-Ava B, pRS414-pGPD-Ava C-pGPD-Ava D | 11.2 | 0 | 340 |
| CEN.PK-1D(pADH-*pps*) + pRS413-pGPD-Ava A-pGPD-Ava B, pRS414-pGPD-Ava C-pGPD-Ava D | 13 | 0 | 375 |
| CEN.PK-1D(pTEF-*pps*) + pRS413-pGPD-Ava A-pGPD-Ava B, pRS414-pGPD-Ava C-pGPD-Ava D | 12.1 | 0 | 410 |
| CEN.PK-1D(pGPD-*pps*) + pRS413-pGPD-Ava A-pGPD-Ava B, pRS414-pGPD-Ava C-pGPD-Ava D | 12.5 | 0 | 580 |

As shown in Table 25 above, it was confirmed that the amount of shinorine production was increased by 70% in the strain, in which the *pps* gene is overexpressed, compared to that of the WT strain. Additionally, it was further confirmed that as the strength of the promoter increased (*i*.*e*., pGPD> pTEF> pADH), the amount of shinorine production was increased.

### Example 19: Increase of amount of shinorine production through enhancement of TKL1, enhancement of ARO4, and introduction of pps gene in Saccharomyces cerevisiae strain

Based on the results of Examples 16, 17, and 18, *TKL1*, *ARO4*, and *pps* (*E. coli*) genes were selected as effective factors which have an influence on shinorine biosynthesis in *Saccharomyces cerevisiae,* and an attempt was made to increase the shinorine biosynthesis through simultaneous enhancement of these three kinds of genes. For the introduction of these three kinds of genes, the pRS-415-pGPD-*TKL1*-pGPD-*ARO4*, and pRS-416-pGPD-*pps* vectors were prepared. Specifically, after pGPD-*TKL1* and pGPD-*ARO4* regions were connected by overlapping PCR, the vectors and PCR products were treated with *Bam*HI and *Sal*I restriction enzymes, and then ligated using T4 ligase to prepare the pRS-415-pGPD-*TKL1-*pGPD*-ARO4* vector.

Then, *the pps* gene derived from *E. coli* was subjected to PCR. The PCR products of the *pps* gene and the pRS-416-pGPD vector were treated with *Bam*HI and *Sal*I restriction enzymes, and then ligated using T4 ligase to prepare the pRS-416-pGPD-*pps* vector. The primers and template DNAs used for the preparation of the vectors are shown in Table 26 below.

**[Table 26]**

| PCR Product | Template Used | SEQ ID NO (Forward Direction, Reverse Direction) |
|---|---|---|
| pGPD | *S. cerevisiae* gDNA | SEQ ID NO: 81, SEQ ID NO: 82 |
| *TKL1* | *S. cerevisiae* gDNA | SEQ ID NO: 83, SEQ ID NO: 84 |
| pGPD-*TKL1* | *S. cerevisiae* gDNA | SEQ ID NO: 81, SEQ ID NO: 84 |
| pGPD | *S. cerevisiae* gDNA | SEQ ID NO: 85, SEQ ID NO: 86 |
| *ARO4* | *S. cerevisiae* gDNA | SEQ ID NO: 87, SEQ ID NO: 88 |
| pGPD-*ARO4* | *S. cerevisiae* gDNA | SEQ ID NO: 85, SEQ ID NO: 88 |
| pGVD-*TKL1*-pGVD-*ARO4* | *S. cerevisiae* gDNA | SEQ ID NO: 81, SEQ ID NO: 88 |
| *ppsA* | *E.coli* MG1655 gDNA | SEQ ID NO: 79, SEQ ID NO: 80 |

Then, the plasmid for shinorine biosynthesis prepared in Example 15 was introduced into *Saccharomyces cerevisiae* CEN.PK-1D strain along with the pRS-415-pGPD-*TKL1*-pGPD-*ARO4* and pRS-416-pGPD-*pps* vectors, and each resulting strain was plated on a synthetic complete (SC) solid medium, in which Leu, Trp, Ura, and His are excluded, and cultured overnight in a 30°C incubator. One platinum loop of the strain cultured overnight in the synthetic complete (SC) solid medium, in which Leu, Trp, Ura, and His are excluded, was inoculated into a 25 mL titration medium, and then was cultured in a 30°C incubator at 150 rpm for 24 hours. The results are shown in Table 27 below.

**[Table 27]**

| Name of Strain | 24 Hours | | Shinorine Conc. (mg/L) |
|---|---|---|---|
| | OD (600nm) | Residual Saccharide | |
| CEN.PK-1D WT + pRS413-pGPD-Ava A-pGPD-Ava B, pRS414-pGPD-Ava C-pGPD-Ava D | 11.2 | 0 | 333 |
| CEN.PK-1D WT+ pRS413-pGPD-Ava A-pGPD-Ava B, pRS414-pGPD-Ava_C-pGPD-Ava_D, p415 -pGPD-*TKL1*-pGPD-*ARO4*, p416-pGPD-*pps* | 13.3 | 0 | 1,100 |

As shown in Table 27 above, it was confirmed that the amount of shinorine production was significantly increased by 230% in the strain, in which the three kinds of effective genes (*i.e.*, *pps*, *TKL1*, and *ARO4*) are overexpressed, compared to that of the WT strain.

In this specification, detailed descriptions of the contents that can be fully recognized and inferred by one of ordinary skill in the art of the present disclosure have been omitted. Therefore, the present disclosure can be implemented in a manner different from those specifically described and exemplified in this specification, which can be understood by one of ordinary skill in the art.
## Claims

1. A microorganism producing a mycosporine-like amino acid, wherein the activity of at least one protein selected from the group consisting of 2-dehydro-3-deoxyphosphoheptonate aldolase, phosphoenolpyruvate synthetase, and transketolase is enhanced.

2. The microorganism according to claim 1, wherein the microorganism further comprises a mycosporine-like amino acid biosynthesis gene cluster.

3. The microorganism according to claim 2, wherein the mycosporine-like amino acid biosynthesis gene cluster comprises a gene encoding at least one protein selected from the group consisting of 2-demethyl 4-deoxygadusol synthase, O-methyltransferase, and a C-N ligase.

4. The microorganism according to claim 2, wherein the mycosporine-like amino acid biosynthesis gene cluster comprises a gene encoding at least one protein selected from the group consisting of non-ribosomal peptide synthetase, non-ribosomal peptide synthetase-like enzyme (NRPS-like enzyme), and D-alanine D-alanine ligase (D-Ala D-Ala ligase).

5. The microorganism according to claim 1, wherein the microorganism is a microorganism of the genus *Corynebacterium* or a microorganism of the genus *Escherichia,* or yeast.

6. The microorganism according to claim 1, wherein the mycosporine-like amino acid is at least one selected from the group consisting of mycosporine-2-glycine, palythinol, palythenic acid, deoxygadusol, mycosporine-methylamine-threonine, mycosporine-glycine-valine, palythine, asterina-330, shinorine, porphyra-334, euhalothece-362, mycosporine-glycine, mycosporine-ornithine, mycosporine-lysine, mycosporine-glutamic acid-glycine, mycosporine-methylamine-serine, mycosporine-taurine, palythene, palythine-serine, palythine-serine-sulfate, palythinol, and usujirene.

7. A method for producing a mycosporine-like amino acid, comprising:
culturing the microorganism of any one of claims 1 to 6 in a medium; and
recovering the mycosporine-like amino acid from the cultured microorganism or medium.

## Patentansprüche

1. Mikroorganismus, der eine Mycosporin-ähnliche Aminosäure erzeugt, wobei die Aktivität von mindestens einem Protein, ausgewählt aus der Gruppe, bestehend aus 2-Dehydro-3-desoxyphosphoheptonataldolase, Phosphoenolpyruvatsynthetase und Transketolase, verstärkt ist.

2. Mikroorganismus nach Anspruch 1, wobei der Mikroorganismus ferner einen Gencluster für die Biosynthese von Mycosporin-ähnlicher Aminosäure umfasst.

3. Mikroorganismus nach Anspruch 2, wobei der Gencluster für die Biosynthese von Mycosporin-ähnlicher Aminosäure ein Gen umfasst, das für mindestens ein Protein kodiert, ausgewählt aus der Gruppe bestehend aus 2-Demethyl-4-desoxygadusolsynthase, O-Methyltransferase und einer CN-Ligase.

4. Mikroorganismus nach Anspruch 2, wobei der Gencluster für die Biosynthese von Mycosporin-ähnlicher Aminosäure ein Gen umfasst, das für mindestens ein Protein kodiert, ausgewählt aus der Gruppe bestehend aus nicht-ribosomaler Peptidsynthetase, nicht-ribosomaler Peptidsynthetase-ähnlichem Enzym (NRPS-ähnliches Enzym) und D-Alanin-D-Alanin-Ligase (D-Ala-D-Ala-Ligase).

5. Mikroorganismus nach Anspruch 1, wobei der Mikroorganismus ein Mikroorganismus der Gattung *Corynebacterium* oder ein Mikroorganismus der Gattung *Escherichia,* oder Hefe ist.

6. Mikroorganismus nach Anspruch 1, wobei die Mycosporin-ähnliche Aminosäure mindestens eine ist, ausgewählt aus der Gruppe bestehend aus Mycosporin-2-Glycin, Palythinol, Palythensäure, Desoxygadusol, Mycosporin-Methylamin-Threonin, Mycosporin-Glycin-Valin, Palythin, Asterina-330, Shinorin, Porphyra-334, Euhalothece-362, Mycosporin-Glycin, Mycosporin-Ornithin, Mycosporin-Lysin, Mycosporin-Glutaminsäure-Glycin, Mycosporin-Methylamin-Serin, Mycosporin-Taurin, Palythene, Palythine-Serin, Palythin-Serin-Sulfat, Palythinol und Usujiren.

7. Verfahren zum Erzeugen einer Mycosporin-ähnlichen Aminosäure, umfassend:
Kultivieren des Mikroorganismus nach einem der Ansprüche 1 bis 6 in einem Medium; und
Gewinnen der Mycosporin-ähnlichen Aminosäure aus dem kultivierten Mikroorganismus oder Medium.

## Revendications

1. Micro-organisme produisant un acide aminé de type mycosporine, l'activité d'au moins une protéine choisie dans le groupe constitué par la 2-déshydro-3-désoxyphosphoheptonate aldolase, la phosphoénolpyruvate synthétase, et la transkétolase étant augmentée.

2. Micro-organisme selon la revendication 1, le micro-organisme comprenant en outre un groupe de gènes de biosynthèse d'acide aminé de type mycosporine.

3. Micro-organisme selon la revendication 2, le groupe de gènes de biosynthèse d'acide aminé de type mycosporine comprenant un gène codant pour au moins une protéine choisie dans le groupe constitué par la 2-desméthyl 4-désoxygadusol synthase, la O-méthyltransférase, et une C-N ligase.

4. Micro-organisme selon la revendication 2, le groupe de gènes de biosynthèse d'acide aminé de type mycosporine comprenant un gène codant pour au moins une protéine choisie dans le groupe constitué par une peptide synthétase non ribosomique, une enzyme de type peptide synthétase non ribosomique (enzyme de type NRPS), et une D-alanine D-alanine ligase (D-Ala D-Ala ligase).

5. Micro-organisme selon la revendication 1, le micro-organisme étant un micro-organisme du genre *Corynebacterium* ou un micro-organisme du genre *Escherichia* ou une levure.

6. Micro-organisme selon la revendication 1, l'acide aminé de type mycosporine étant au moins l'un choisi dans le groupe constitué par mycosporine-2-glycine, palythinol, acide palythénique, désoxygadusol, mycosporine-méthylamine-thréonine, mycosporine-glycine-valine, palythine, astérina-330, shinorine, porphyra-334, euhalothèce-362, mycosporine-glycine, mycosporine-ornithine, mycosporine-lysine, mycosporine-acide glutamique-glycine, mycosporine-méthylamine-sérine, mycosporine-taurine, palythène, palythine-sérine, palythine-sérine-sulfate, palythinol, et usujirène.

7. Procédé pour la production d'un acide aminé de type mycosporine, comprenant :
la culture du micro-organisme selon l'une quelconque des revendications 1 à 6 dans un milieu ; et
la récupération de l'acide aminé de type mycosporine à partir du micro-organisme ou milieu cultivé.
